# EUROPEAN PATENT SPECIFICATION

(11) **EP 0 360 258 B1**
(45) Date of publication and mention of the grant of the patent: **14.08.1996**
(21) Application number: 89117428.6
(22) Date of filing: 21.09.1989
(51) Int. Cl.: C07D 471/04, C07D 401/04, C07K 5/04, A61K 31/47, A61K 31/435, A61K 38/00

(54) **Amino acid quinoline and naphthyridine derivatives**
Aminosäure-Derivate von Chinolin und Naphthyridin
Dérivés d'acides aminés de quinoléine et de naphtyridine

(30) Priority: 22.09.1988 US 247990; 21.02.1989 US 313260; 24.03.1989 US 328401; 01.09.1989 US 400111
(43) Date of publication of application: 28.03.1990
(73) Proprietor: ABBOTT LABORATORIES, Abbott Park, Illinois 60064-3500 (US)
(72) Inventor: Chu, Daniel Tim-Wo, Vernon Hills Illinois 60061 (US); Hallas, Robert, Kenosha Wisconsin 53142 (US)
(74) Representative: Modiano, Guido, Dr.-Ing.

(56) References cited:
- EP-A- 0 027 752
- EP-A- 0 169 710
- EP-A- 0 304 087
- US-A- 4 479 898
- US-A- 4 730 000

## Description

### Technical Field

This invention relates to novel derivatives of naphthyridine and quinoline compounds, pharmaceutical compositions and their use as antibacterial agents in humans and mammals in need of such treatment. These compounds also have very low solubility at physiological pH which reduces their oral absorption.

### Background of the Invention

Quinoline and naphthyridine antibacterial compounds are well known in the art. For example, Chu, U.S. Patent No. 4,730,000, issued March 8, 1988 describes quinoline compounds with a 7-pyrrolidine substituent group having antibacterial properties, Chu, U.S. Patent No. 4,616,019, issued October 7, 1986 describes naphthyridine compounds with a 7-pyrrolidine substituent group with antibacterial properties, Matsumoto et al., U.S. Patent No. 4,649,144, issued March 10, 1988 describes 1,8-naphthyridine compounds with a 7-pyrrolidine substituent group with antibacterial activity, and Domagala et al., U.S. Patent No. 4,735,949, issued April 5, 1988 describes disubstituted 7-pyrrolidine-naphthyridine compounds with antibacterial activity. These compounds, however, have limited aqueous solubility which impairs their use in injectable formulations for intravenous, intramuscular or subcutaneous application. These compounds also have very low solubility at physiological pH which reduces their oral absorption.

Of further interest as background to the present invention is EP-A-0 027 752 which discloses naphthyridine antibacterial agents containing an amino or alkylamino group in the 3-position of a 7-pyrrolidine ring. Also of interest is EP-A-0 304 087 published February 22, 1989 which discloses quinolone and naphthyridine antibacterial agents containing an alpha-amino acid in the side chain of the 7-substituent.

In order to improve the solubility of various compounds for pharmaceutical use, their structure has been modified. For example, Gilvarg et al., U.S. Patent No. 4,479,898, issued October 30, 1984 describes peptide chains substituted by a nucleophilic residue in the alpha position for use as prodrugs to increase cell membrane permeability and Delmotte et al., U.S. Patent No. 4,753,984, issued June 28, 1988 describes water soluble macromolecular prodrugs in which a polyhydroxylated polyamine is linked to a therapeutically active drug by a peptide chain.

### Summary of the Invention

The present invention relates to compounds having enhanced agueous solubility.

More particularly, this invention relates to new quinolines and naphthyridines where the 7-pyrrolidine ring is 3-A-amino substituted, wherein A is a solubilizing group. The solubilizing group is a dipeptide.

These novel compounds are useful as broad spectrum antibacterial agents, with activity against both gram positive and gram negative bacteria, as well as enterobacteria.

More particularly, this invention relates to compounds of the following formula: 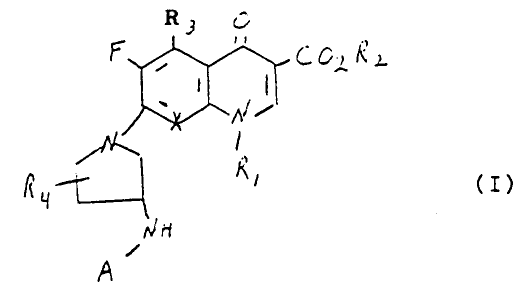 wherein R₁ is alkyl, haloalkyl, hydroxyalkyl, cycloalkyl, vinyl, aryl or aryl substituted with one to three substituents independently selected from the group consisting of hydrogen, halogen, alkyl, haloalkyl, alkanoyloxy, and a group having the formula -Y-R₅, wherein Y is O or S and R₅ is hydrogen or alkyl;
R₂ is hydrogen, alkyl, haloalkyl or a carboxy protecting group;
R₃ is hydrogen or NH₂;
X is N, CH, COH, C-O-alkyl, CF, CCl, C-alkyl or C-NH-alkyl;
R₄ is hydrogen, alkyl or haloalkyl; and
A is a dipeptidyl group; and the pharmaceutically acceptable salts thereof.

The present invention includes antibacterial compositions comprising an antibacterially effective amount of a compound of Formula I and a pharmaceutically acceptable carrier or diluent.

### Detailed Description of the Invention

This invention relates to compounds of the following formula: 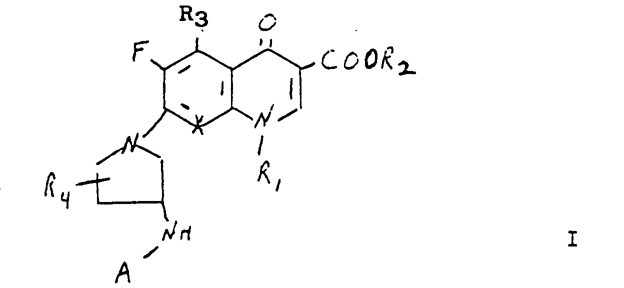 wherein R₁ is a member selected from the group consisting of alkyl, haloalkyl, hydroxyalkyl, cycloalkyl, vinyl, aryl and aryl substituted with one to three substituents independently selected from the group consisting of hydrogen, halogen, alkyl, halosubstituted alkyl, alkanoyloxy, a group having the formula -Y-R₅, wherein Y is 0 or S and R₅ is hydrogen or alkyl;
R₂ is hydrogen, alkyl, haloalkyl or a carboxy protecting group;
R₃ is hydrogen or NH₂;
X is N, CH, COH, C-O-alkyl, CF, CCl, C-alkyl or C-NH-alkyl;
R₄ is hydrogen, alkyl and haloalkyl; and
A is a dipeptidyl group; and the pharmaceutically acceptable salts thereof.

This invention also relates to antibacterial compositions comprising an antibacterially effective amount of a compound of Formula I and a pharmaceutically acceptable carrier or diluent.

A preferred group are compounds of the formula: 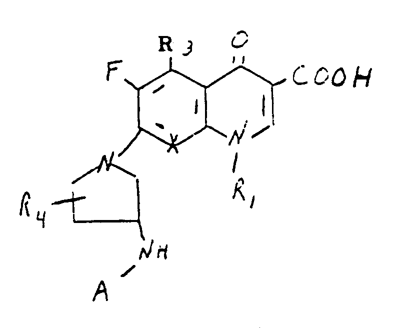 wherein R₁ is ethyl, 2-fluoroethyl, t-butyl, cyclopropyl, 4-fluorophenyl, or 2,4-difluorophenyl;
R₃ is hydrogen or NH₂;
X is N,CH,CF, CCH₃ or CCl;
R₄ is hydrogen, alkyl or haloalkyl;
A is a dipeptidyl group;
and the pharmaceutically acceptable salts thereof.

A more preferred group of compounds of this invention are compounds of the above formula wherein A is Gly-Phe, Ala-Phe, Gly-Nval, Ala-Nval, Gly-Ala, Leu-Nval, Ala-Met, Ala-Leu, Leu-Leu, Leu-Met, Leu-Ala, Met-Leu, Met-Ala, D-Ala-L-Ala, Phe-Ala, Val-Ala, Val-Leu, Gly-Gly, Met-Nval, Nval-Gly, Nval-Ala, Nval-Nval, Phe-Gly or Ala-Ala.

Compounds that are representative of the preferred class of compounds of this invention include the following compounds and their hydrochloride salts:
1. 7-(3-Gly-Phe-amino-1-pyrrolidinyl)-6-fluoro-1-(4-fluorophenyl)-1,4,-dihydro-4-oxo-quinoline-3-carboxylic acid
2. 7-(3-Gly-Phe-amino-1-pyrrolidinyl)-6-fluoro-1-(2,4-difluorophenyl)-1,4,-dihydro-4-oxo-quinoline-3-carboxylic acid
3. 7-(3-Nval-Nval-amino-1-pyrrolidinyl)-6-fluoro-1-(2,4-difluorophenyl)-1,4,-dihydro-4-oxo-quinoline-3-carboxylic acid
4. 7-(3-Nval-Nval-amino-1-pyrrolidinyl)-6-fluoro-1-(4-fluorophenyl)-1,4,-dihydro-4-oxo-quinoline-3-carboxylic acid
5. 7-(3-Phe-Gly-amino-1-pyrrolidinyl)-6-fluoro-1-(4-fluorophenyl)-1,4,-dihydro-4-oxo-quinoline-3-carboxylic acid
6. 7-(3-Ala-Phe-amino-1-pyrrolidinyl)-6-fluoro-1-(2,4-difluorophenyl)-1,4,-dihydro-4-oxo-quinoline-3-carboxylic acid
7. 7-(3-Ala-Phe-amino-1-pyrrolidinyl)-6-fluoro-1-(4-fluorophenyl)-1,4,-dihydro-4-oxo-quinoline-3-carboxylic acid
8. 7-(3-Ala-Ala-amino-1-pyrrolidinyl)-6-fluoro-1-(4-fluorophenyl)-1,4,-dihydro-4-oxo-quinoline-3-carboxylic acid
9. 7-(3-Ala-Ala-amino-1-pyrrolidinyl)-6-fluoro-1-(2,4-difluorophenyl)-1,4,-dihydro-4-oxo-quinoline-3-carboxylic acid
10. 7-(3-Gly-Phe-amino-1-pyrrolidinyl)-6-fluoro-1-(4-fluorophenyl)-1,4,-dihydro-4-oxo-1,8-naphthyridine-3-carboxylic acid
11. 7-(3-Gly-Phe-amino-1-pyrrolidinyl)-6-fluoro-1-(2,4-difluorophenyl)-1,4,-dihydro-4-oxo-1,8-naphthyridine-3-carboxylic acid
12. 7-(3-Nval-Nval-amino-1-pyrrolidinyl)-6-fluoro-1-(4-fluorophenyl)-1,4,-dihydro-4-oxo-1,8-naphthyridine-3-carboxylic acid
13. 7-(3-Nval-Nval-amino-1-pyrrolidinyl)-6-fluoro-1-(2,4-difluorophenyl)-1,4,-dihydro-4-oxo-1,8-naphthyridine-3-carboxylic acid
14. 7-(3-Ala-Phe-amino-1-pyrrolidinyl)-6-fluoro-1-(2,4-difluorophenyl)-1,4,-dihydro-4-oxo-1,8-naphthyridine-3-carboxylic acid
15. 7-(3-Ala-Phe-amino-1-pyrrolidinyl)-6-fluoro-1-(4-fluorophenyl)-1,4,-dihydro-4-oxo-1,8-naphthyridine-3-carboxylic acid
16. 7-(3-Ala-Ala-amino-1-pyrrolidinyl)-6-fluoro-1-(4-fluorophenyl)-1,4,-dihydro-4-oxo-1,8-naphthyridine-3-carboxylic acid
17. 7-(3-Ala-Ala-amino-1-pyrrolidinyl)-6-fluoro-1-(2,4-difluorophenyl)-1,4-dihydro-4-oxo-1,8-naphthyridine-3-carboxylic acid
18. 7-(3-Gly-Phe-amino-1-pyrrolidinyl)-6-fluoro-1-cyclopropyl-1,4-dihydro-4-oxo-quinoline-3-carboxylic acid
19. 7-(3-Nval-Nval-amino-1-pyrrolidinyl)-6-fluoro-1-cyclopropyl-1,4-dihydro-4-oxo-quinoline-3-carboxylic acid
20. 7-(3-Gly-Phe-amino-1-pyrrolidinyl)-6-fluoro-1-cyclopropyl-1,4-dihydro-4-oxo-1,8-naphthyridine-3-carboxylic acid
21. 7-(3-Ala-Nval-amino-1-pyrrolidinyl)-6-fluoro-1-(2,4-difluorophenyl)-1,4-dihydro-4-oxo-1,8-naphthyridine-3-carboxylic acid
22. 7-(3-Leu-Nval-amino-1-pyrrolidinyl)-6-fluoro-1-(2,4-difluorophenyl)-1,4-dihydro-4-oxo-1,8-naphthyridine-3-carboxylic acid
23. 7-(3-Met-Nval-amino-1-pyrrolidinyl)-6-fluoro-1-(2,4-difluorophenyl)-1,4-dihydro-4-oxo-1,8-naphthyridine-3-carboxylic acid
24. 7-(3-Gly-Gly-amino-1-pyrrolidinyl)-6-fluoro-1-(2,4-difluorophenyl)-1,4-dihydro-4-oxo-1,8-naphthyridine-3-carboxylic acid
25. 7-(3-Gly-Nval-amino-1-pyrrolidinyl)-6-fluoro-1-(2,4-difluorophenyl)-1,4-dihydro-4-oxo-1,8-naphthyridine-3-carboxylic acid
26. 7-(3-Nval-Gly-amino-1-pyrrolidinyl)-6-fluoro-1-(2,4-difluorophenyl)-1,4-dihydro-4-oxo-1,8-naphthyridine-3-carboxylic acid
27. 7-(3-Nval-Ala-amino-1-pyrrolidinyl)-6-fluoro-1-(2,4-difluorophenyl)-1,4-dihydro-4-oxo-1,8-naphthyridine-3-carboxylic acid
28. 7-(3-Gly-Gly-amino-1-pyrrolidinyl)-6-fluoro-1-cyclopropyl-1,4-dihydro-4-oxo-1,8-naphthyridine-3-carboxylic acid
29. 7-(3-Nval-Nval-amino-1-pyrrolidinyl)-6-fluoro-1-cyclopropyl-1,4-dihydro-4-oxo-1,8-naphthyridine-3-carboxylic acid
30. 7-(3-Gly-Gly-amino-1-pyrrolidinyl)-6-fluoro-1-cyclopropyl-1,4-dihydro-4-oxo-quinoline-3-carboxylic acid
31. 7-(3-Nval-Nval-amino-1-pyrrolidinyl)-6-fluoro-1-cyclopropyl-1,4-dihydro-4-oxo-quinoline-3-carboxylic acid
32. 7-(3-Gly-Nval-amino-1-pyrrolidinyl)-6-fluoro-1-cyclopropyl-1,4-dihydro-4-oxo-quinoline-3-carboxylic acid
33. 7-(3-Gly-Nval-amino-1-pyrrolidinyl)-6-fluoro-1-cyclopropyl-1,4-dihydro-4-oxo-1,8-naphthyridine-3-carboxylic acid
34. 7-(3-Gly-Ala-amino-1-pyrrolidinyl)-6-fluoro-1-(2,4-difluorophenyl)-1,4-dihydro-4-oxo-1,8-naphthyridine-3-carboxylic acid
35. 7-(3-Ala-Met-amino-1-pyrrolidinyl)-6-fluoro-1-(2,4-difluorophenyl)-1,4-dihydro-4-oxo-1,8-naphthyridine-3-carboxylic acid;
36. 7-(3-Met-Met-amino-1-pyrrolidinyl)-6-fluoro-1-(2,4-difluorophenyl)-1,4-dihydro-4-oxo-1,8-naphthyridine-3-carboxylic acid;
37. 7-(3-Leu-Met-amino-1-pyrrolidinyl)-6-fluoro-1-(2,4-difluorophenyl)-1,4-dihydro-4-oxo-1,8-naphthyridine-3-carboxylic acid;
38. 7-(3-Leu-Leu-amino-1-pyrrolidinyl)-6-fluoro-1-(2,4-difluorophenyl)-1,4-dihydro-4-oxo-1,8-naphthyridine-3-carboxylic acid;
39. 7-(3-Ala-Leu-amino-1-pyrrolidinyl)-6-fluoro-1-(2,4-difluorophenyl)-1,4-dihydro-4-oxo-1,8-naphthyridine-3-carboxylic acid;
40. 7-(3-Met-Leu-amino-1-pyrrolidinyl)-6-fluoro-1-(2,4-difluorophenyl)-1,4-dihydro-4-oxo-1,8-naphthyridine-3-carboxylic acid;
41. 7-(3-Phe-Ala-amino-1-pyrrolidinyl)-6-fluoro-1-(2,4-difluorophenyl)-1,4-dihydro-4-oxo-1,8-naphthyridine-3-carboxylic acid;
42. 7-(3-Leu-Ala-amino-1-pyrrolidinyl)-6-fluoro-1-(2,4-difluorophenyl)-1,4-dihydro-4-oxo-1,8-naphthyridine-3-carboxylic acid;
43. 7-(3-Met-Ala-amino-1-pyrrolidinyl)-6-fluoro-1-(2,4-difluorophenyl)-1,4-dihydro-4-oxo-1,8-naphthyridine-3-carboxylic acid;
44. 7-(3-Val-Ala-amino-1-pyrrolidinyl)-6-fluoro-1-(2,4-difluorophenyl)-1,4-dihydro-4-oxo-1,8-naphthyridine-3-carboxylic acid;
45. 7-(3-Val-Leu-amino-1-pyrrolidinyl)-6-fluoro-1-(2,4-difluorophenyl)-1,4-dihydro-4-oxo-1,8-naphthyridine-3-carboxylic acid; and
46. 7-(3-D-Ala-L-Ala-amino-1-pyrrolidinyl)-6-fluoro-1-(2,4-difluorophenyl)-1,4-dihydro-4-oxo-1,8-naphthyridine-3-carboxylic acid.

As used herein, the term "carboxy-protecting group" refers to a carboxy group which has been esterified with one of the commonly used carboxylic acid protecting ester groups employed to block or protect the carboxylic acid functionality. In addition, compounds containing a carboxy protecting group can be used as prodrugs whereby the carboxy protecting group can be hydrolyzed enzymatically to release the biologically active parent acid. Such protected carboxy groups are noted for their ease of cleavage by hydrolytic methods to the corresponding carboxylic acid. Further, such carboxy-protecting groups can be cleaved to yield the corresponding free carboxy group. Such carboxy-protecting groups are well known to those skilled in the art, having been extensively used in the protection of carboxyl groups in the penicillin and cephalosporin fields, as described in U.S. Patent Nos. 3,840,556 and 3,719,667, the disclosures of which are incorporated herein by reference. Representative protecting groups include C₁ to C₈ alkyl (e.g., methyl, ethyl, tertiary butyl,) benzyl and substituted derivatives thereof such as alkoxy and nitrobenzyl groups, dialkylaminoalkyl (e.g. dimethylaminoethyl), acyloxyalkyl groups such as pivaloyloxymethyl and propionyloxy methyl.

As used herein, the term "pharmaceutically acceptable salts" refers to non-toxic acid addition salts and alkaline earth metal salts of the compounds of Formula I. The salts can be prepared in situ during the final isolation and purification of the compounds of Formula I, or separately by reacting the free base or acid functions with a suitable organic acid or base. Representative acid addition salts include the hydrochloride, hydrobromide, sulphate, bisulphate, acetate, oxalate, valerate, oleate, palmitate, stearate, laurate, borate, benzoate, lactate, phosphate, tosylate, mesylate, citrate, maleate, fumarate, succinate, tartrate, ascorbate glucoheptonate, lactobionate, lauryl sulphate salts and the like. Representative alkali or alkaline earth metal salts include the sodium, calcium, potassium and magnesium salts, and the like.

As used herein, the term "halogen" or "halo" refers to chloro, bromo, fluoro and iodo groups.

As used herein, the term "alkyl" includes both straight or branched chain radicals of one to six carbon atoms. Representative of such radicals are methyl, ethyl, propyl, iso-propyl, t-butyl, sec-butyl, isobutyl, amyl, butyl, neopentyl, hexyl and the like.

As used herein, the term "cycloalkyl" refers to those rings having three to six carbon atoms such as cyclopropyl, cyclobutyl, cyclopentyl and cyclohexyl.

As used herein, the term "haloalkyl", refers to halogen substituted straight and branched carbon chain radicals of one to six carbon atom and one to three halogen atoms. Representative of such groups are fluoromethyl, 2-fluoroethyl, 2-chloroethyl, 2,2-dichloroethyl, 2-chloropropyl, 2-chloroisopropyl, 3-iodobutyl, and the like.

As used herein, the term "hydroxyalkyl" refers to -OH appended to an alkyl radical.

As used herein, the term "alkanoyloxy" refers to R₆COO- wherein R₆ is alkyl.

As used herein, the term "aryl" refers to aromatic radical having five to six atoms in the ring system and may contain one to three hetero atoms selected from S, O and N, the remaining atoms being carbon atoms. Representative aromatic radicals include phenyl, pyridyl, pyrazinyl, thiazoyl, furyl and thienyl.

As used herein, the term "solubilizing group" refers to a peptide chain containing two amino acid residues which are joined covalently through peptide bonds. The amino acid residues used in the compounds of the invention encompass the 20 naturally occurring, standard amino acids, (as designated by their three letter symbols); 4-hydroxyproline, hydroxylysine, demosine, isodemosine, 3-methylhistidine, norvaline (Nval), beta-alanine, gama-amino-butyric acid, homocysteine, homoserine, citrulline, ornithine and methionine sulfone.

For the sake of convenience and understanding, the amino acids three letter and single letter designations are as follows:

| | 3- or 4-letter | 1-letter |
|---|---|---|
| Alanine | Ala | A |
| Arginine | Arg | R |
| Asparagine | Asn | N |
| Aspartic Acid | Asp | D |
| Glutamic Acid | Glu | E |
| Glutamine | Gln | Q |
| Glycine | Gly | G |
| Histidine | His | H |
| Homoserine | Hse | - |
| Isoleucine | Ile | I |
| Leucine | Leu | L |
| Lysine | Lys | K |
| Methionine | Met | M |
| Norvaline | Nval | - |
| Phenylalanine | Phe | F |
| Proline | Pro | P |
| Serine | Ser | S |
| Threonine | Thr | T |
| Tryptophan | Try | O |
| Tyrosine | Tyr | Y |
| Valine | Val | V |

The preferred amino acid residues are those with a non-polar group such as Ala, Val, Nval, Leu, Met, Gly, Pro, Phe; or a basic polar group, such as Lys.

For purposes of clarity, it should be noted that the 3-amino function of the 7-pyrrolidinyl group forms a bond with the carboxyl group of an alpha amino acid residue and, in turn,
the amino acid residues are bonded to each other via conventional peptide bonding; ie., the alpha amino group of the first linked amino acid residue is bonded to the carboxyl group of the second alpha amino acid residue.

As used herein, the term "amino protecting group" refers to a group which protects the N-terminal of the amino acid residue. The alpha amino group of each amino acid employed in the peptide synthesis must be protected during the coupling reaction to prevent side reactions involving the reactive alpha-amino function. It should also be recognized that certain amino acids contain reactive side-chain functional groups (e.g. sulfhydryl, epsilon-amino, carboxyl, and hydroxyl), and that such functional groups must also be protected both during the initial and subsequent coupling steps. Suitable protecting groups are known in the art See for example Protective Groups in Organic Chemistry, M. McOmie, Editor, Plenum Press, N.Y., 1973.

In selecting a particular protecting group, certain conditions must be observed. An alpha-amino protecting group (1) must be stable, (2) must render the alpha-amino function inert under the conditions employed in the coupling reaction, and (3) must be readily removable after the coupling reaction under conditions that will not remove side chain protecting groups and will not alter the structure of the peptide fragment. A side chain protecting group (1) must render the side chain functional group inert under the conditions employed in the coupling reaction, (2) must be stable under the conditions employed in removing the alpha-amino protecting group, and (3) must be readily removable upon completion of the desired amino acid sequence under reaction conditions that will not alter the structure of the peptide chain.

It will be apparent to those skilled in the art that the protecting groups known to be useful for peptide synthesis will vary in reactivity and affect the selection of the agents employed for their removal. For example, certain protecting groups, such as triphenylmethyl and 2-(p-biphenylyl)isopropyloxycarbonyl are very labile and can be cleaved under mild acid conditions. Other protecting groups such as t-butyloxycarbonyl, t-amyloxycarbonyl, adamantyloxycarbonyl, and p-methoxybenzyloxycarbonyl, are less labile and require moderately strong acids, such as trifluoroacetic, hydrochloric, or boron trifluoride in acetic acid, for their removal. Still other protecting groups, such as benzyloxycarbonyl, halobenzyloxycarbonyl, p-nitrobenzyloxycarbonyl, cycloalkyloxycarbonyl, and isopropyloxycarbonyl, are even less labile and require strong acids, such as hydrogen fluoride, hydrogen bromide, or boron trifluoroacetate in trifluoroacetic acid for their removal.

Illustrative examples of amino acid protecting groups are set forth below.
A. for an alpha-amino group, protecting groups may include (a) acyl type groups, such as formyl, trifluoracetyl, phthalyl, p-toluenesulfonyl (tosyl), benzenesulfonyl, nitrophenylsulfenyl, and the like; (b) aromatic urethane type groups, such as benzyloxycarbonyl and substituted benzyloxycarbonyl, such as, for example, p-chlorobenzyloxycarbonyl, p-bromobenzyloxycarbonyl, p-nitrobenzyloxycarbonyl and p-methoxybenzyloxycarbonyl, o-chlorobenzyloxycarbonyl, 2,4-dichlorobenzyloxycarbonyl, 2,6-dichlorobenzyloxycarbonyl, and the like; (c) aliphatic urethane type groups such as t-butyloxycarbonyl, t-amyloxycarbonyl, isopropyloxycarbonyl, 2-(p-biphenylyl)-isopropyloxycarbonyl, allyloxycarbonyl, and the like; (d) cycloalkyl urethane groups such as cyclopentyloxycarbonyl, cyclohexyloxycarbonyl, cycloheptyloxycarbonyl, adamantyloxycarbonyl, and the like; (e) thiourethane type groups such as phenylthiocarbonyl; (f) alkyl type groups such as triphenylmethyl, and (g) trialkylsilane groups, such as trimethylsilane. A preferred a-amino protecting group is t-butyloxycarbonyl (BOC).
B. For the epsilon-amino protecting group present in lysine, protection may be by any of the groups mentioned hereinabove for protection of an alpha-amino group. Typical groups include, for example, benzyloxycarbonyl, p-chlorobenzyloxycarbonyl, p-bromobenzyloxycarbonyl, o-chlorobenzyloxycarbonyl, 2,6-dichlorobenzyloxycarbonyl, 2,4-dichlorobenzyloxycarbonyl, o-bromobenzyloxycarbonyl, p-nitrobenzyloxycarbonyl t-butyloxycarbonyl, isopropyloxycarbonyl, t-amyloxycarbonyl, cyclopentyloxycarbonyl, cyclohexyloxycarbonyl, cycloheptyloxycarbonyl, adamantyloxycarbonyl, p-toluenesulfonyl, and the like. The preferred epsilon-amino protecting group is o-chlorobenzyloxycarbonyl (ClBzl).
C. For the hydroxy group of serine, threonine, or tyrosine, protection may be, for example, by C₁-C₄ alkyl, such as methyl, ethyl, and t-butyl; benzyl; substituted benzyl, such as p-methoxybenzyl, p-nitrobenzyl, p-chlorobenzyl, and o-chlorobenzyl; C₁-C₃ alkanoyl, such as formyl, acetyl, and propionyl; triphenylmethyl; or benzoyl. The preferred hydroxyl protecting group is benzyl (Bzl).
D. For the carboxyl group of aspartic acid of glutamic acid, protection may be, for example, by esterification using groups such as benzyl, t-butyl, cyclohexyl, cyclopentyl, and the like. The current groups of choice are cyclohexyl and cyclopentyl.

As used herein the term "C or carboxy terminal activating group" refers to a group which renders the carboxyl group of an amino acid residue more susceptible to reaction with a free N-terminal amino group (of a peptide fragment).

For example, the amino acid can be converted to a mixed anhydride by reaction of a protected amino acid with ethyl chloroformate, phenyl chloroformate, sec-butyl chloroformate, isobutyl chloroformate, pivaloyl chloride, or like acid chlorides. Alternatively, the amino acid can be converted to an active ester such as a 2,4,5-trichlorophenyl ester, a pentachlorophenyl ester, a p-nitrophenyl ester, an ester formed from N-hydroxysuccinimide, N-hydroxyphthalimide, or an ester formed from 1-hydroxybenzotriazole.

As used herein, the terms "aralkyloxycarbonyl", "alkyloxycarbonyl and "cyclooxycarbonyl" refer to the amino protecting groups as noted above, wherein the aryl, alkyl and cycloalkyl radicals are as defined herein.

Certain substituents on the pyrrolidine ring may exist in the cis or trans stereochemical forms. The pure isomers or mixtures thereof are also contemplated by the invention.

Certain compounds of the invention may exist in optically active forms. The pure R isomers, pure S isomers as well as mixtures thereof; including the racemic mixtures are contemplated by the invention. Additional asymmetric carbon atoms may be present in a substituent such as an alkyl group. All such isomers as well as mixtures thereof are intended to be included in the invention.

The stereoisometric configuration of the amino acids of the invention may be either the D- or L-form, or a mixture of configurations. Preferably, however, the amino acids are all of the L-form. The sequence of amino acids in a peptide chain read from left to right, from the amino (N)-terminal to the carboxy (C)-terminal as are conventionally used in the art.

The compounds according to the invention exhibit a broad antibacterial spectrum against Gram-positive and Gram-negative germs.

The compounds of the invention are also useful as chemotherapeutic active compounds for the treatment of mammals.

In addition, the compounds may be used in scrub solutions for surface inhibition of bacterial growth, e.g., on counter surfaces, and the like, and as substances for preserving inorganic and organic materials, in particular all kinds of organic materials, for example, polymers, lubricants, paints, fibers, leather, paper and wood, foodstuffs and water.

The compounds according to the invention are particularly active against bacteria and bacteria-like micro-organisms. They are therefore particularly suitable in human and veterinary medicine for the prophylaxis and chemotherapy of local and systemic infections carried by pathogems such as Staphylococci (Staph. aureus and Staph. epidermidis) and Streptococci (Strep. agalactiae, Strept. facealis, Strep. pneumoniae, Strep. bovis and Strept. pyogenes); Neisseria, (Neisseria gonorrhoeme) Enterobacteriaceae, for example Escherichia, Haemophilus influenza, Citrobacter (Citrob freundil and Citrob. divermis), Salmonella and Shigella and Klebeiellae (Klebs, pneumoniae and Klebs. oxyvocs), Micrococcus, Entrobacter (Ent. aerogenes and Ent. agglumerenx), Hafnia, Serratia (Serr. marcescens), Proteus (Pr. mirabillis, Pr. rettgerri and Pr. vulgaris), Providencia, Yersinia and the genus Acinetobacter. The antibacterial spectrum also includes the genus Pseudomonas (Ps. aeruginosa, Ps. cepacia and Ps. maltophilia) the genus Chlamydia (such as Chlamydia trachomatis) as well as anaerobic bacteria, such as, for example, bacteroides fragilis, representatives of the genus Peptococcus, Peptostreptococcus and the genus Clostridium; and furthermore Mycoplasma (M. pneumoniae, M. hominis and M. urealyticum) and Mycobactria, for example Mycobacterium tuberculosis.

Examples of diseases which can be caused by the pathogens (or mixed infections as noted above) and can be treated by compounds of this inventions are: infectious diseases in humans, such as, otitis; pharyngitis, pneumonia, peritonitis, pyelonephritis, cystitis, endocarditis, systemic infections, bronchitis (acute and chronic), septic infections, diseases of the upper respiratory tract, diffuse panbronchiolitis, pulmonary emphysema, dysentery, enteritis, abscesses of the liver, urethritis, prostatitis, epididymitis, gastrointestinal infections, bone and joint infections, cystic fibrosis, skin infections, postoperative wound infections, abscesses, phlegmons, wound infections, infected burns, burn wounds, infections in the oral region, infections following dental operations, osteomyelitis, septic arthritis, chlocystitis, peritonitis with appendicitis, chlolangitis, intraabdominal abscesses, pancreatitis, sinusitis, mastoiditis, mastitis, tonsillitis, typhoid fever, meningitis and infections of the nervous system, salpingitis, endometritis, genital infections, pelveoperitonitis and eye infections.

Representative examples of animals and infections in such animals which may be treated are: pigs: coli-diarrhea, enterotoxaemia, sepsis, dysentery, salmonellosia, metritis-mastitis-agalactiae syndrome and mastitis; ruminants (cattle, sheep, goats): diarrhea, sepsis, bronchopneumonia, salmonellosis, pasteurellosis, mycoplasmosis and genital infections; horses: bronchopneumonia, joint ill, puerperal and postpuerperal infections and salmonellosis; dogs and cats: bronchopneumonia, diarrhea, dermatitis, otitis, urinary tract infections and prostatitis; poultry (chicken, turkeys, quail, pigeons, ornamental birds and others): mycoplasmosis, E. coli infections, chronic infections of the respiratory tract, salmonellosis, pasteurellosis and psittacosis.

Bacterial infections in the breeding and rearing of stock and ornamental fish can likewise be treated, the antibacterial spectrum being increased beyond the above-mentioned pathogens to further pathogens, such as, for example, pasteurella, Brucella, Campylobacter, Listeris, Erysipelothrix, Corynebactria, Borellia, Treponema, Nocardia, Rickettsia and Yersinia.

In addition to exhibiting highly effective antibacterial activity, the compounds of the invention exhibit increased and improved solubility characteristics as compared with known naphthyridine and quinoline-3-carboxylic acid compounds. Also, it is believed that the compounds of the invention may act as prodrugs of known naphthyridine and quinoline-3-carboxylic acid compounds such as tosufloxacin.

The present invention includes one or more of the compounds of Formula I formulated into compositions together with one or more non-toxic pharmaceutically acceptable carriers adjuvants or vehicles which are collectively referred to herein as carriers, for parenteral injection, for oral administration in solid or liquid form, for rectal administration, and the like.

Non-toxic, inert pharmaceutically suitable carriers include solid, semi-solid or liquid diluents, fillers and formulation auxiliaries of all types.

The compositions can be administered to humans and animals either orally, rectally, parenterally (intravenously, intramuscularly or subcutaneously), intracisternally, intravaginally, intraperitoneally or locally (powders, ointments or drops), and for the therapy of infections in hollow cavities and body cavities.

Compositions according to the invention for parenteral injection may comprise pharmaceutically acceptable sterile aqueous or nonaqueous solutions, dispersions, suspensions or emulsions and sterile powders for reconstitution into sterile injectible solutions or dispersions. Examples of suitable aqueous and nonaqueous carriers, diluents, solvents or vehicles include water, ethanol, polyols (propylene glycol, polyethylene glycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions and by the use of surfactants.

These compositions may also contain adjuvants such as preserving, wetting, emulsifying, and dispensing agents. Prevention of the action of microoganisms can be ensured by various antibacterial and antifungal agents, for example, parabens, chlorobutanol, phenol, sorbic acid, and the like. It may also be desirable to include isotonic agents, for example sugars, sodium chloride and the like. Prolonged absorption of the injectable pharmaceutical form can be brought about by the use of agents delaying absorption, for example, aluminum monosterate and gelatin.

If desired, and for more effective distribution, the compounds can be incorporated into slow release or targeted delivery systems such as polymer matrices, liposomes, and microspheres. They may be sterilized, for example, by filtration through a bacteria-retaining filter, or by incorporating sterilizing agents in the form of sterile solid compositions which can be dissolved in sterile water, or some other sterile injectable medium immediately before use.

Solid dosage forms for oral administration include capsules, tablets, pills, powders and granules. In such solid dosage forms, the active compound is admixed with at least one inert customary excipient (or carrier) such as sodium citrate or dicalcium phosphate or (a) fillers and extenders, as for example, starches, lactose, sucrose, glucose, mannitol and silicic acid, (b) binders, as for example, carboxymethylcellulose, alginates, gelatin and polyvinylpyrrolidone, sucrose and acacia, (c) humectants, for example glycerol, (d) disintegrating agents, as for example, agar-agar, calcium carbonate, potato or tapioca starch, alginic acid, certain complex silicates, and sodium carbonate (e) solution retarders, as for example paraffin, and (f) absorption accelerators, as for example, quaternary ammonium compounds, (g) wetting agents, as for example, cetyl alcohol and glycerol monostearate, (h) adsorbents, as for example, kaolin and bentonite, and (i) lubricants, as for example, talc, calcium stearate, magnesium stearate, solid polyethylene glycols, sodium lauryl sulfate or mixtures thereof. In the case of capsules, tablets and pills, the dosage forms may also comprise buffering agents.

Solid compositions of a similar type may also be employed as fillers in soft and hard-filled gelatin capsules using such excipients as lactose or milk sugar as well as high molecular weight polyethylene glycols, and the like.

The solid dosage forms of tablets, dragees, capsules, pills and granules can be prepared with coatings and shells, such as enteric coatings and others well known in this art. They may optionally contain opacifying agents, and can also be of such composition that they release the active compound or compounds only or preferentially in a certain part of the intestinal tract, optionally in a delayed manner, examples of embedding compositions which can be used are polymeric substances and waxes.

The active compounds can also be in micro-encapsulated form, if appropriate, with one or more of the abovementioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art, such as water or other solvents solubilizing agents and emulsifiers, as for example, ethyl alcohol, isopropyl alcohol, ethyl carbonate, ethyl acetate, benzyl alcohol, benzyl benzoate, propylene glycol, 1,3-butylene glycol, dimethylformamide, oils, in particular cottonseed oil, groundnut oil, corn germ oil, olive oil, castor oil and sesame oil, glycerol, glycerolformal, tetrahydrofurfuryl alcohol, polyethylene glycols and fatty acid esters of sorbitan or mixtures of these substances, and the like.

Besides such inert diluents, the composition can also include adjuvants, such as wetting agents, emulsifying and suspending agents, sweetening, flavoring and perfuming agents.

Suspensions, in addition to the active compounds, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures or these substances, and the like.

Compositions for rectal administrations are preferably suppositories which can be prepared by mixing the compounds of this invention with suitable non-irritating excipients or carriers such as cocoa butter, polyethylene glycol or a suppository wax, which are solid at ordinary temperatures but liquid at body temperature and therefore, melt in the rectum or vaginal cavity and release the active component.

Dosage forms for topical administrtion of a compound of this invention include ointments, pastes, creams, gels, powders, sprays and inhalants. The active component is admixed under sterile conditions with a pharmaceutically-acceptable carrier and any needed preservatives or buffers as may be required. Opthalmological formulations, ear drops, eye ointments, powders and solutions are also contemplated as being within the scope of this invention.

The ointments, pastes, creams and gels may contain, in addition to an active compound of this invention, excipients such as animal and vegetable fats, waxes, paraffins, starch, tragacanth, cellulose derivatives, polyethylene glycols, silicones, bentonites, silicic acid, talc and zinc oxide, or mixtures thereof.

Powders and sprays can contain, in addition to compounds of this invention, excipients such as lactose, talc, silicic acid, aluminum hydroxide, calcium silicates and polyamide powder, or mixtures of these substances. Sprays can additionally contain customary propellants, as for example, chlorofluorohydrocarbons.

Actual dosage levels of active ingredient in the compositions of the invention may be varied so as to obtain an amount of active ingredient effective to achieve antibacterial activity in accordance with the desired method of administration. The selected dosage level therefore depends upon the nature of the active compound administered, the route of administration, the desired duration of treatment and other factors. Generally, daily dosage levels of the compounds of Formula I are about 0.1 to about 750, preferably about 0.25 to about 500 and most preferably about 0.5 to about 300 mg. of active ingredient per kg. of body weight and are effective when administered to a mammalian patient suffering from an infection caused by a susceptible organism. If desired, the daily dose may be divided into multiple doses for administration, e.g., two to four times per day.

An individual unit dose preferably contains the active compound of compounds according to this invention in amounts of about .5 to about 80, in particular .5 to 30 mg/kg of body weight. It may, however, be necessary to deviate from the dosages mentioned.

### Synthesis of the Compounds

The naphthyridine antibacterial compounds of this invention having a 7-((3-amino)-1-pyrrolidinyl) substituent group can be synthesized by conventional means known in this art as for example, those described in U.S. Pat. No. 4,616,019, issued October 7, 1986 or U.S. Pat. No. 4,649,144, issued March 10, 1987, whose disclosures are incorporated herein by reference.

The quinoline antibacterial compounds of this invention having a 7-(3-amino-1-pyrrolidinyl) substituent group are also prepared by conventional means known in this art as for example, those described in U.S. Pat. No. 4,730,000, issued March 8, 1988 and U.S. Pat. No. 4,735,949, issued April 5, 1988, whose disclosures are incorporated herein by reference.

A general reaction scheme for preparation of the compounds of this invention by standard amino acid coupling techniques as well known in the art and exemplified by Example 1-3 is as follows: 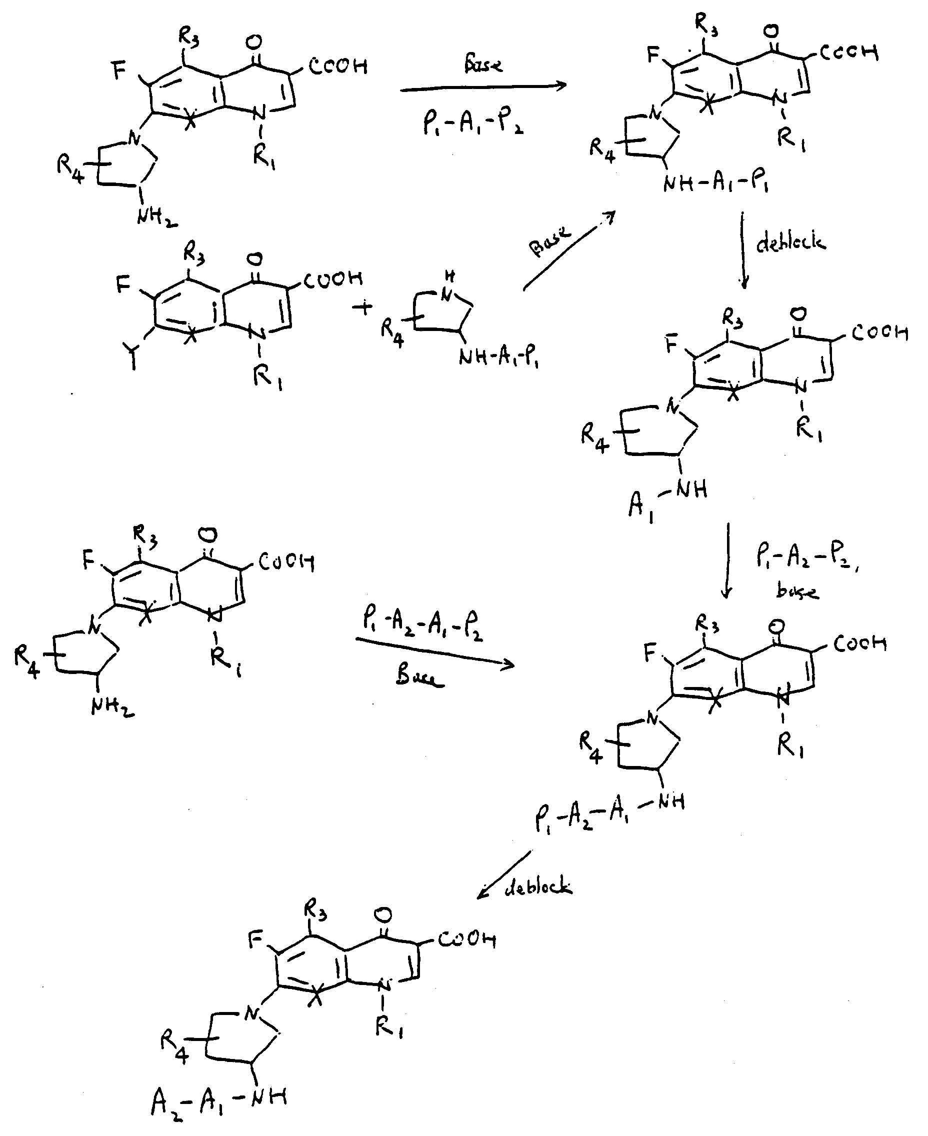 wherein X, R₁, and R₃ and R₄ are as defined previously; A₁ and A₂ are amino acid residues as described previously, and P₁ is an amino protecting and P₂ a carboxy activating group, as also described previously and Y is a leaving group such as chloro or bromo or fluoro or thiomethyl group.

The dipeptide solubilizing groups of this invention can be prepared by any of a variety of recognized peptide synthesis techniques including classical (solution) methods, solid phase methods, and the more recently available recombinant DNA methods.

One method of preparation of the dipeptide solubilizing groups of this invention is by the solid phase technique in which the amino acid sequence is constructed sequentially from an initial insoluble resin-supported C-terminal amino acid. Techniques for the solid phase method are described by J. Stewart et al., Solid Phase Peptide Synthesis, Freeman and Co., San Francisco, 1969.

In general, in the solid phase method, the amino acid corresponding to the C-terminal amino acid residue of the desired peptide is anchored to an insoluble resin support, and the peptide chain then is formed beginning at the resin-supported C-terminal amino acid. A second amino acid is introduced to obtain the desired amino acid sequence. The peptide chain remains attached to the resin throughout synthesis, and, upon completion of the chain, the peptide is cleaved from the resin.

The peptide chain is attached to the polystyrene resin by means of an ester linkage formed between the carboxyl group of the C-terminal moiety and one of the methylene groups present on the resin matrix as sites for such attachment. The polystyrene resin is a styrene polymer which is cross-linked by the addition of about 0.5 to about 3% divinylbenzene and which is chloromethylated or hydroxymethylated to provide sites for ester formation. An example of a hydroxymethylated resin is described by Bodanszky et al., Chem. Ind. (London), 38, 1597-98 (1966). A chloromethylated polystyrene resin is commercially available from Lab System, Inc., San Mateo, California. The resin is also described by Stewart et al., Solid Phase Peptide Synthesis, Freeman and Co., San Francisco, California, pp. 1-6.

The amino acids are coupled using techniques well known in the art for the formation of peptide bonds. One method involves converting the amino acid to a derivative that will render the carboxyl terminus group more susceptible to reaction with the free N-terminal amino group of the peptide fragment.

Another coupling method involves use of a suitable coupling agent, such as N,N′-dicyclohexylcarbodiimide (DCC) or N,N′-diisopropylcarbodiimide (DIC). Other appropriate coupling agents will be apparent to those skilled in the art. See Schroder and Lubke, The Peptides, Academic Press, 1965, Chapter III.

Upon completion of the desired peptide sequence, the protected peptide must be cleaved from the resin support, and all protecting groups must be removed. The cleavage reaction and removal of the protecting groups may be accomplished simultaneously or stepwise. When the resin support is a chloromethylated polystyrene resin, the bond anchoring the peptide to the resin is an ester linkage formed between the free carboxyl group of the C-terminal moiety and one of the many chloromethyl groups present on the resin matrix. It will be recognized that the anchoring bond can be cleaved by reagents which are known to be capable of breaking an ester linkage and of penetrating the resin matrix. One especially convenient method is by treatment with liquid hydrogen fluoride. This reagent not only will cleave the peptide from the resin but will also remove all protecting and activating groups. Hence, use of this reagent will directly afford the desired peptide.

When it is desired to cleave the peptide without removing protecting groups, the protected peptide-resin can undergo methanolysis to give the protected peptide in which the C-terminal carboxyl group is methylated. The methyl ester can then be hydrolyzed under mild, alkaline conditions to give the free C-terminal carboxylic acid. The protecting groups on the peptide chain then can be removed by treatment with a strong acid, such as liquid hydrogen fluoride. A particularly useful technique for methanolysis is that of G. Moore et al., Peptides Proc. 5th Amer. Pept. Symp., M. Goodman and J. Meinhofer, Eds., John Wiley, N.Y., 1977, pp. 581-521, in which the protected peptide-resin is treated with methanol and potassium cyanide in the presence of crown ether.

Another method for cleaving the protected peptide from the resin is by ammonolysis or by treatment with hydrazine. The resulting C-terminal amide or hydrazide can be hydrolyzed to the free C-terminal carboxyl, and the protecting groups can be removed conventionally.

It will also be recognized that the protecting groups present on the N-terminal a-amino group may be removed preferentially either before or after the protected peptide is cleaved from the resin support.

The protecting groups are otherwise removed by appropriate treatment as known in the art. Removal of the dinitrophenyl group from His-containing peptides is usually carried out with N-methylmercaptoacetamide in DMF. The N-formyl group is usually removed from Try by treatment with NaOH at pH 11.5 in the presence of hydrazine. Other protecting groups are removed by treatment with anisole and anhydrous HF.

The following examples relate to (1) compounds of the invention according to formula (I) in which the solubilizing group A is a dipeptidyl group, (2) compounds of formula (I) falling outside the invention in which the group A is an amino acid group, such compounds being able to serve as intermediates for the preparation of the dipeptidyl-substituted compounds of the invention, (3) compounds of formula (I) falling outside the invention in which the group A is a tri- or tetra-peptidyl group. The following examples illustrate methods for preparing the above compounds and present solubility and pharmacological data. Data on some of the above compounds which are not dipeptide derivatives were part of the study leading up to the invention and are presented for comparison purposes.

### Example 1

7-[(3-Gly-L-Phe-amino)-1-pyrrolidinyl]- 1-(2, 4-difluorophenyl)-6-fluoro-1,4-dihydro-4-oxo-1,8-naphthyridine-3-carboxylic acid hydrochloride.
a) To a cold solution of 7-(3-amino-1-pyrrolidinyl)-1-(2,4-difluorophenyl)-1,4 dihydro-4-oxo-1,8-naphthyridine-3-carboxylic acid tosylate (2.08 gm) in dry dimethylformamide (DMF) (80 ml) is added N-carbobenzyloxy-glycyl-L-phenylalanine N-hydroxysuccinimide ester (1.80 gm) followed by N-methylmorpholine (803 mg). The mixture is stirred in the cold for an additional hour, then at room temperature for about 17-18 hours. The resulting solution is poured into 500 ml of 0.5 NHCl and the mixture is extracted with 2 x 250 ml of CHCl₃. The combined extracts are washed with a 5% NaHC0₃ solution, and the organic phase is dried over MgS0₄ and evaporated to leave a solution of DMF and product. The residual DMF is removed by co-distillation with toluene, ethylene dichloride, CHCl₃ and CH₂Cl₂ to leave 2.66 gm of a white colored foam. Trituration with ether yielded 2.56 gm of the desired product. IR and NMR analysis confirmed the product.
   NMR data (CDCl₃) delta: 3.75(d) 2-H, 4.44(m) 1-H, 4.66 (m) 1-H, 4.92(m) 2-H, 5.40(m) 1-H, 6.55(m) 1-H, 7.00-7.30(m) 11-H, 7.35 (m) 4-H, 7.92(d) 1-H, 8.47 (d) 1-H;
   IR data: (CDCl₃); CO, cm⁻¹: 1630, 1665, 1720; [alpha]_{D} = -19° (CHCl₃); MS (M+1)⁺=743 m/z.
b) 7-(3-N-carbobenzyloxy-Gly-L-Phe-amino)-1-pyrrolidinyl]-1-(2,4-difluorophenyl)-6-fluoro-1,4-dihydro-4-oxo-1,8-naphthridine-3-carboxylic acid (2.55 gm), 10% Pd/C (dry) (1.25 gm) and 150 ml acetic acid are reacted on a Parr Shaker apparatus under 60 psi of hydrogen atmosphere for about a 24 hour period. After the reaction is completed, the hydrogen is vented, the catalyst is removed by filteration and the reaction mixture is washed thoroughly. The filtrate is evaporated to leave a residue. This residue is treated with a 0.5N HCl in ethanol solution and evaporated. The residual HCl is removed by co-distillation and ethanol to leave 1.77 grams of a light yellow colored powder. NMR and IR analysis confirmed product identification. MS (M+1)⁺=609 m/z. [alpha]_{D} = +35° (CH₃0H).
   NMR data (DMSO):delta: 2.84(m) 2-H, 3.50(m) 2-H, 4.20(m) 1-H, 4.51(m) 1-H, 7.03(m) 1-H, 7.03(m) 1-H, 7.10-7.23 (m) 5-H, 7.35(m) 1-H, 7.60(m) 1-H, 7.80(m) 1-H, 8.05(m) 2-H, 8.40(m) 1-H, 11.43(s) 1-H.
   IR data (KBR) CO, cm⁻¹: 1630; 1670, 1710.

### Example 2

### 7-[3-Norvaline-amino-1-pyrrolidinyl]-1-(2,4 difluorophenyl)-6-fluoro-1,4-dihydro-4-oxo-1,8-naphthyridine-3-carboxylic acid hydrochloride.

a) To a cold solution of 7-(3-amino-1-pyrrolidinyl)-1-(2,4-difluorophenyl)-6-fluoro-1,4-dihydro-4-oxo-1,8-naphthyridine-3-carboxylic acid tosylate (2.50 gm) in dry DMF (25 ml) is added N-carbobenzyloxy-L-NorValine-N-Hydroxyphthalimide ester (1.89 gm) followed by N-methylmorpholine (1.06 gm). The mixture is stirred in the cold for an additional hour, then at room temperature for about 17-18 hours. The resulting solution is poured into 500 ml. of 0.5N HCL and the mixture is extracted with 2 x 250 ml. portion of CHCl₃. The combined extracts are washed with 5% NaHCO3 solution and the organic phase is dried over MgSO4 and evaporated to leave a solution of DMF and product. The residual DMF is removed by co-distillation with toluene, with ethylene dichloride, with CHCl₃ and with CH₂Cl₂ to leave 2.69 grams of a white colored foam. Trituration with ether caused the foam to crystallize and yield 2.34g (85% of the desired product); mp = 200°-202°; [alpha]_{D} = -2.6° (CHCl₃); MS (M+1)⁺=638 m/z.
   NMR data (CDCl₃):delta: 0.90(m) 3-H, 1.35(m) 2-H, 1.61(m) 2-H, 1.80(m) 1-H, 2.00(m) 2-H, 4.20(m) 1-H, 4.50(m) 1-H, 5.05(s) 2-H, 5.40(m) 1-H, 7.05(m) 1-H, 7.30(m) 5-H, 7.85(m) 1-H, 8.40(m) 1-H.
   IR data (CDCl₃) CO, cm⁻¹: 1630, 1670, 1718.
   Alternately, this compound can be made by reacting 2.5 gm 7-chloro-1-(2,4-difluorophenyl)-6-fluoro-1,4-dihydro-4-oxo-1,8-naphthyridine-3-carboxylic acid with 3 gm of (3-N-carbobenzyloxy Norvalylamino)-pyrrolidine in 20 ml pyridine at 65°C for 24 hours. The solvent was removed and the residue is treated with trifluoroacetic acid and then evaporated to remove the solvent. It is then treated with ethanol and ether and filtered yielding the 7-(3-N-carbobenzyloxy-L-Norvaline-amino-1-pyrrolidinyl-1-(2,4-difluorophenyl)-6-fluoro-1,4-dihydro-4-oxo-1, 8-naphthyridine-3-carboxylic acid.
b) A mixture of 7-(3-N-carbobenzyloxy-L-Norvaline-amino-1-pyrrolidinyl)-1-(2-4-difluorophenyl)-6-fluoro-1,4-dihydro-4-oxo-1,8-naphthyridine-3-carboxylic acid (2.32 gm), 10% Pd/C (1.16 gm) and 150 ml acetic acid is reacted on a Parr Shaker apparatus under 60 psi of hydrogen atmosphere for about 24 hours. After the reaction is completed, the hydrogen is vented, the catalyst is removed by filtration and the reaction mixture is washed thoroughly. The filtrate is evaporated to leave a residue. This residue is treated with 0.5N HCl in ethanol and evaporated. The residual HCl is removed by co-distillation with ethanol to leave 1.94 gm of a yellow colored powder. mp = 190°(dec.); [alpha]_{D} + 3.7° (CH₃0H).
   MS (M+1)⁺=504 m/z.
   NMR data (DMSO) delta: 0.85(m) 3-H, 1.25(m) 3-H, 1.63(m) 2-H, 2.05(m) 1-H, 4.32(m) 1-H, 7.35(m)1H, 7.60 (m) 1-H, 7.80(m) 1-H, 8.25(m) 2-H, 8.82(s) 1-H, 8.90(m) 1-H.
   IR data (KBR) CO, cm⁻¹: 1630, 1680, 1772.
   Alternately, the above compound can be made as follows: To a solution of 2.0 gm of ethyl 7-chloro-1-(2,4-difluorophenyl)-6-fluoro-1,4-dihydro-4-oxo-1,8-naphthyridine-3-carboxylate in 20 ml of pyrridine at 65°C is added in 3 gm of 3-(N-t-butoxy carbonyl-Norvalylamino)-pyrrolidine. After 20 hours, the solvent is removed. The product is purified by column chromatography on silica to give ethyl 7-(3-N-t-butoxycarbonyl Norvalylamino pyrrolidin-1-yl-1-(2,4-difluorophenyl-6-fluoro-1,4-dihydro-4-oxo-1,8-naphthyridine-3-carboxylate. This compound is dissolved in 20 ml trifluoroacetic acid and 20 ml of 6N HCl is added and the mixture is refluxed for 20 hours. The solvent is removed to give 7-(3-Norvaline-amino-1-pyrrolidinyl)-1-(2,4-difluorophenyl)-6-fluoro-1,4-dihydro-4-oxo-1,8-naphthyridine-3-carboxylic acid hydrochloride.

### Example 3

### 7-(3-L-Norvaline-L-Norvaline amino-1-pyrrolidinyl)-1-(2,4-difluorophenyl)-6-fluoro-1,4-dihydro-4-oxo-1,8-naphthyridine-3-carboxylic acid hydrochloride.

(a) To a cold solution of (3-L-Norvaline-amino-1-pyrrolidinyl)-1-(2,4-difluorophenyl)-6-fluoro-1,4-dihydro-4-oxo-1,8-naphthyridine-3-carboxylic acid hydrochloride (681 mg) in dry DMF (20 ml) is added N-carbobenzyloxy-1-Norvaline-N-hydroxyphthalimide ester (500 mg) followed by methylmorpholine (255 mg). The mixture is stirred in the cold for an additional hour, then at room temperature for about 17-18 hours. The resulting solution is poured into 250 ml. of 0.5N HCl and the mixture is extracted with 2 x 200 ml portions of CHCl₃. The combined extracts are washed with 5% NaHC0₃ solution and the organic phase is dried over MgS0₄, and evaporated to leave a solution of DMF and product. The residual DMF is removed by co-distillation with toluene, with ethylene dichloride, CHCl₃ and CH₂Cl₂ to leave 768 mg of a light yellow colored foam. The residue is purified by a column chromatography (EM Sciences Silica Gel, 70-230 Mesh) to obtain 469 mg of product. [alpha]_{D} = -2.6° (CHCl₃); MS (M + 1)=737 m/z.
   NMR data (DMSO) - delta: 0.8(m) 6-H, 1.25(m) 6-H, 1.50(m) 4-H, 3.95(m), 2-H, 4.16(m) 2-H, 4.25(m) 1-H, 4.97(m) 2-H, 7.33(m) 5-H, 7.40(m) 1-H, 7.55(m) 1-H, 7.75(m) 1-H, 7.85(m) 1-H, 8.05(m) 1-H, 8.15(m) 1-H, 8.77(m) 1-H.
   IR data (CHCl₃) CO cm⁻¹: 1635, 1660, 1720.
(b) A mixture of 7-(3-N-carbobenzyloxy-L-Norvaline-L-Norvaline-amino-1-pyrrolidinyl)-1-(2,4-difluorophenyl)-6-fluoro-1,4-dihydro-4-oxo-1,8-naphthyridine-3-carboxylic acid (449 mg), 10% Pd/C (dry, 225 mg) and 50 ml of acetic acid is reacted on a Parr Shaker apparatus under 60 psi of hydrogen atmosphere for about 24 hours. After the reaction is completed, the hydrogen is vented, the catalyst is removed by filtration and the reaction mixture is washed thoroughly. The filtrate is evaporated to leave a residue. This residue is treated with 0.5N HCl in ethanol and evaporated. The residual HCl was removed by co-distillation with ethanol to leave 267 mg of a light yellow colored powder. mp=218°-220°C, [alpha]D = +15.4 (CH₃OH), MS (M+1)⁺=603 m/z.
   NMR data (DMSO) delta: 0.82(m) 6-H, 1.25(m) 4-H, 1.63(m) 4-H, 3.77(m) 1-H, 4.24(m) 2-H, 7.33(m) 1-H, 7.57(m) 1-H, 7.80(m) 1-H, 8.10(d) 1-H, 8.20(m) 2-H, 8.40(m) 1-H, 8.61(m) 1-H, 8.82(s) 1-H.
   IR data: KBR CO, cm-1 - 1630, 1670, 1718.
   Following the procedures of Examples 1-3 above, but substituting the appropriate solubilizing group (A) and protecting and activating groups for those used in Example 1-3, the following compounds, together with their respective IR, NMR data and with melting points where applicable, are set forth below.

### Example 4

Using the process as described in Example 1a or 2a and substituting Phe for the solubilizing group A, yields the protected phenylalanine (Phe) compound, (I) (R₁ = 2,4-difluorophenyl, X=N, R₃ = H, R₂ = R₄ = H, A = carbobenzyloxy Phe), mp=168-170°C. [alpha]_{D}=2.70 (CHCl₃).

NMR data: (CDCl₃)delta: 3.05(m) 1-H, 3.10(m) 1-H, 4.40(m) 2-H, 5.03(s) 2-H, 5.45(m) 1-H, 7.05-7.35(m) 11-H, 7.90(m) 1-H, 8.40(m) 1-H

IR data: (CDCl₃) CO, cm⁻¹: 1630, 1670, 1718.

### Example 5

Using the process as described in Example 2b with the compound of Example 4 yields the deblocked Phe (HCl) salt, (I)(R₁=2,4-difluorophenyl, X=N, R₂ = R₃ = R₄ = H, A = Phe), mp=188-190°C. M.S. (M+1) = 552 m/z. [alpha]_{D}=9.2 (CH₃OH).

NMR data: (DMSO)delta: 2.90(m) 1-H, 3.06(m) 1-H, 3.90(m) 1-H, 4.24(m) 1-H, 7.10(m) 2-H, 7.20-7.40(m) 5-H, 7.60(m) 1-H, 7.82(m) 1-H, 8.10(m) 1-H, 8.40(m) 2-H, 8.83(m) 1-H

IR data: (KBR) CO, cm⁻¹: 1630, 1682, 1720.

### Example 6

Using the process as described in Example 2a and substituting Gly for the solubilizing group A, yields the protected Gly compound, (I)(R₁ = 2,4-difluorophenyl, X=N, R₂ = R₃ = R₄ =H, A = carbobenzyloxygly), mp=179-182°C. MS (M+1)⁺=596m/z.

NMR data: (CDCl₃)delta: 2.05(m) 1-H, 3.92(m) 2-H, 4.59(m) 1-H, 5.06(s) 2-H, 5.50(m) 1-H, 7.04(m) 2-H, 7.28(m) 5-H, 7.75(m) 1-H, 8.32(m) 1-H.

IR data: (CDCl₃) CO, cm⁻¹: 1630, 1678, 1720.

### Example 7

Using the process as described in Example 2b with the compound of Example 6 yields the deblocked Gly (HCl) salt, (I)(R₁ = 2,4-difluorophenyl, X=N, R₂ = R₃ = R₄ = H, A = Gly), mp=195° - 198°C.
MS (M+1)⁺=462 m/z.

NMR data: (DMSO) delta: 3.50(d) 2-H, 4.32(m) 1-H, 7.35(m) 1-H, 7.60(m) 1-H, 7.82(m) 1-H, 8.10(m) 2-H, 8.77(d) 1-H, 8.83(s) 1-H.

IR data: (KBR) CO, cm⁻¹: 1633, 1670, 1733.

### Example 8

Using the process as described in either Example 1a or 3a and substituting Phe-Gly for the solubilizing group A, yields the protected Phe-Gly compound (I)(R₁ = 2,4-difluorophenyl, X=N, R₂ = R₄ = R₃ = H, A = carbobenzyloxy Phe-Gly). MS (M+1)⁺=828 m/z.
[alpha]_{D} = -4.7 (CHCl₃).

NMR data: (CDCl₃) delta: 2.05(m) 2-H, 2.97(m) 1-H, 3.13(m) 1-H, 3.40(d) 1-H, 4.23(m) 1-H, 4.50(m) 1-H, 4.89(m) 2-H, 5.22(m) 1-H, 7.05-7.35(m) 16-H, 7.88(d) 1-H, 8.45(m) 1-H.

IR data: (CDCl₃) CO, cm⁻¹: 1635, 1670, 1710.

### Example 9

Using the process as described in Example 3b with the compound of Example 8 yields the deblocked Phe-Gly(HCl) salt (I)(R₁ = 2,4-difluorophenyl, X=N, R₂ = R₃ = R₄ = H, A = Phe-Gly).

NMR data: (DMSO) delta: 1.85(m) 1-H, 2.05(m) 1-H, 2.95(m) 1-H, 3.10(m) 1-H, 3.75(m) 2-H, 4.05(m) 1-H, 4.30(m) 1-H, 7.27(m) 4-H, 7.60(m) 1-H, 7.70(m) 1-H, 8.10(m) 1-H, 8.35(m) 1-H, 8.82(s) 1-H.

IR data: KBr CO, cm⁻¹: 1630, 1680, 1720.

### Example 10

Using the process as described in either Example 1a or 3a and substituting Ala-Phe for the solubilizing group A, yields the protected Ala-Phe compound (I)(R₁ = 2,4-difluorophenyl, R₂ = R₃ = R₄ = H, X = N, A = carbobenzyloxy Ala-Phe). MS (M+1)⁺ = 757 m/z.

NMR data: (CDCl₃) delta: 1.25(m) 3-H, 2.05(m) 1-H, 3.13(m) 3-H, 4.00(m) 1-H, 4.46(m) 1-H, 4.62(m) 1-H, 4.86(m) 1-H, 4.86(m) 1-H, 5.05(m) 1-H, 6.41(m) 1-H, 7.00-7.40(m) 14-H, 7.96(m) 1-H, 8.52(m) 1-H.

### Example 11

Using the process as described in Example 3b with the compound of Example 10 yields the deblocked Ala-Phe (HCl) salt, (I)(R₁ = 2,4-difluorophenyl, X = N, R₂ = R₃ = R₄ = H, A = Ala-Phe), mp 195-198°C; [alpha] = +45.2 (CH₃OH). MS (M+1)⁺ = 623 m/z.

IR (KBr) CO, cm⁻¹: 1630, 1670, 1720.

NMR data: (CD₃OD) delta: 1.47(m) 3-H, 1.90(m) 1-H, 2.96(m) 2-H, 3.90(m) 1-H, 4.23(m) 1-H, 4.53(m) 1-H, 6.98(m) 1-H, 7.07(m) 1-H, 7.15(m) 1-H, 7.25(m) 5-H, 7.65(m) 1-H, 8.05(m) 1-H, 8.78(m) 1-H.

### Example 12

Using the process as described in Example 1a or 2a and substituting Tyr for the solubilizing group A yields the protected Tyr compound, (I)(R₁ = 2,4-difluorophenyl, X = N, R₂ = R₃ = R₄ = H, A = carbobenzyloxy Tyr). MS (M+1)⁺=702 m/z.

NMR data: (DMSO) delta: 1.62(m) 1-H, 1.96(m) 1-H, 2.64(m) 1-H, 4.08(m) 1-H, 4.23(m) 1-H, 6.53(m) 1-H, 6.62(m) 1-H, 6.42-7.02(m) 2-H, 7.25-7.38(m) 5-H, 7.45(m) 1-H, 7.60(m) 1-H, 7.80(m) 1-H, 8.10(m) 2-H.

IR data: (CHCl₃) CO, cm⁻¹: 1630, 1670, 1718.

### Example 13

Using the process as described in Example 1b or 2b with the compound of Example 12 yields the deblocked Tyr (HCl) salt, (I)(R₁ = 2,4 difluorophenyl, X=N, R₂ = R₃ = R₄ = H, A = Tyr), MS (M+1)⁺=568 m/z.

NMR data: (DMSO)delta: 1.56(m) 1-H, 1.97(m) 1-H, 2.87(m) 1-H, 3.80(m) 1-H, 4.24(m) 1-H, 6.50(m) 1-H, 6.69(m) 1-H, 6.86(m) 2-H, 6.97(m) 1-H, 7.17(m) 1-H, 7.33(m) 1-H, 7.60(m) 1-H, 7.80(m) 1-H, 8.08(d) 1-H, 8.27(m) 2-H, 8.62(m) 1-H, 8.82(m) 1-H, 9.22(m) 1-H, 9.40(m) 1-H, 9.80(m) 1-H

IR data: (KBr) CO, cm⁻¹ 1635, 1683, 1720.

### Example 14

Using the process as described in either Example 1 or 3a and substituting Ala-Ala for the solubilizing group A yields the protected Ala-Ala compound (I)(R₁ = 2,4-difluorophenyl, X=N, R₂ = R₂ = R₄ = H, A = carbobenzyloxy Ala-Ala). MS (M+1)⁺=681 m/z. [alpha]_{D} =-12.1°C (CHCl₃) or 4.0 (DMSO), mp 160-164°C.

NMR data: delta: 1.37(m) 6-H, 2.00(m) 2-H, 3.40(m) 2-H, 4.07(m) 1-H, 4.45(m) 2-H, 4.96(m) 2-H, 5.20(m) 1-H, 6.46(m) 1-H, 7.06(m) 2-H, 7.33(m) 5-H, 7.43(m) 1-H, 8.50(d) 1-H.

IR data: (CDCl₃) CO cm⁻¹: 1630, 1670, 1719.

### Example 15

Using the process as described in Example 3b with the compound of Example 14 yields the deblocked Ala-Ala (HCl) salt, (I)(R₁ = 2,4-difluorophenyl, X=N, R₂ = R₃ = R₄ = H, A = Ala-Ala), mp =202-205°C., [alpha]_{D} = 10.4° (CH₃OH); MS (M+1)⁺=547 m/z.

NMR data (DMSO)delta: 1.20(t) 3-H, 1.30(d) 3-H, 1.80 (m) 2-H, 2.03(m) 2-H, 3.83(m) 2-H, 4.24(m) 2-H, 7.33(m) 1-H, 7.58(m) 1-H, 7.80(m) 1-H, 8.15(m) 2-H, 8.36(m) 1-H, 8.61(m) 1-H, 8.82(s) 1-H.

### Example 16

Using the process as described in Example 1a or 2a and substituting Ala for the solubilizing group A yields the protected alanine (Ala) compound, (I) (R₁ = 2,4-difluorophenyl, X=N, R₃ = H, R₂ = R₄ = H, A = carbobenzyloxy Ala); mp=173-174°C, [alpha]_{D}=1.80 (CHCl₃). MS (M+1) = 610 m/z.

NMR data: (CDCl₃)delta: 1.38(m), 3-H, 2.00(m) 2-H, 4.25(m) 1-H, 4.54(m) 1-H; 5.06(s) 2-H, 5.42(m) 1-H, 7.04(m) 1-H, 7.13(m) 1-H, 7.30(m) 5-H, 7.90(m) 1-H.

IR data: (CDCl₃) CO, cm⁻¹: 1630, 1670, 1720.

### Example 17

Using the process as described in Example 2b with the compound of Example 16 yields the deblocked Ala (HCl) salt, (I)(R₁=2,4-difluorophenyl, X=N, R₂ = R₃ = R₄ = H, A = Ala), mp=202-205°C. M.S. (M+1) 476 m/z. [alpha]_{D}=5.6 (CH₃OH).

NMR data: (DMSO)delta: 1.32(t) 3-H, 1.85(m) 1-H, 2.05(m) 1-H, 3.75(m) 1-H, 4.32(m) 1-H, 7.35(m) 1-H, 7.60(m) 1-H, 7.80(m) 1-H, 8.09(D) 1-H, 8.20(m) 2-H, 8.80(m) 1-H, 8.83(m) 1-H

IR data: (KBR) CO, cm⁻¹: 1635, 1680, 1720.

### Example 18

Using the process as described in Example 2a and substituting Val for the solubilizing Group A yields the protected Val compound, (I)(R₁ = 2,4-difluorophenyl, X=N, R₂ = R₃ = R₄ =H, A = carbobenzyloxyVal), mp=170°C (dec), MS (M+1)⁺=638m/z [alpha]_{D} = -2.1° (CHCl₃).

NMR data: (CDCl₃)delta: .90(m) 3-H, .95(m) 2-H, 2.00(m) 2-H, 2.20(m) 2H, 3.40(m) 2H, 4.10(m) 2-H, 4.55(m) 1-H, 5.05(m) 2H, 5.45(m) 1-H, 7.00(m) 1-H, 7.30(s) 5-H, 8.40(m) 1-H, 8.40(m) 1-H.

IR data: (CDCl₃) CO, cm⁻¹ -1630, 1670, 1720.

### Example 19

Using the process as described in Example 2b with the compound of Example 18 yields the deblocked Val (HCl) salt, (I)(R₁ = 2,4-difluorophenyl, X=N, R₂ = R₃ = R₄ = H, A = Val); mp=195° - 198°C,
MS (M+1)⁺=504 m/z. [alpha]_{D} = 11.2° (CH₃OH).

NMR data: DMSO(delta): 0.85(m) 3H, 1.85(m) 1-H, 2.05(m) 2-H, 4.35(m) 1H, 7.35(m) 1-H, 7.70(m) 1H, 7.80(m) 1H, 8.10(D) 1H, 8.80(m) 1H, 8.82(S) 1H.

IR data: (KBR) CO, cm⁻¹ - 1630, 1680, 1720.

### Example 20

Using the process as described in Example 2a and substituting Leu for the solubilizing group A yields the protected Leucine compound (I)(R₁ = 2,4-difluorophenyl, X=N, R₂ = R₄ = R₃ = H, A = carbobenzyloxy Leu). MS (M+1)⁺=652 m/z, m.p.=188-190°C, [alpha]_{D} = -7.9° (CHCl₃).

NMR data: (CDCl₃) delta: 0.90(S) 3H, 1.50(m) 1H, 1.65(S) 2H, 2.00(m) 2H, 3.45(m) 2H, 3.85(m) 1-H, 4.20(m) 1H, 4.50(m) 1H, 5.05(S) 2H, 5.30(m) 1H, 7.05(m) 1H, 7.15(m) 1H, 7.30(S) 5H, 7.85(m) 1H, 8.40(m) 1H.

IR data: (CDCl₃) CO, cm⁻¹ 1630, 1670, 1720.

### Example 21

Using the process as described in Example 2b with the compound of Example 20 yields the deblocked Leu(HCl) salt (I)(R₁ = 2,4-difluorophenyl, X=N, R₂ = R₃ = R₄ = H, A = Leu). mp=198°C (decomposition), MS (M+1)⁺ 518 m/z [alpha]_{D} = 3.0° (CH₃OH).

NMR data: (DMSO) delta: 0.85(m) 3H, 1.50(m) 2H, 1.85(m) 1H, 2.05(m) 1H, 3.65(m) 1H, 4.30(m) 1H, 7.35(m) 1H, 7.60(m) 1H, 7.80(m) 1H, 8.10(D) 1H, 8.25(m) 2H, 8.82(S) 1H, 8.90(m) 1H.

IR data: KBr CO, cm⁻¹ 1630, 1680, 1730.

### Example 22

Using the process as described in Example 2a and substituting Met for the solubilizing group A yields the protected Methionine compound (I)(R₁ = 2,4-difluorophenyl, R₂ = R₃ = R₄ = H, X = N, A = carbobenzyloxy Met).

### Example 23

Using the process as described in Example 2b with the compound of Example 22 yields the deblocked Met (HCl) salt, (I)(R₁ = 2,4-difluorophenyl, X = N, R₂ = R₃ = R₄ = H, A = Met), MS (M+1)⁺ = 536; [alpha]_{D} 4.1° (CH₃OH).

NMR data DMSO data: 1.95(S) 3H, 2.05(D) 2H, 2.45(m) 2H, 3.75(m) 1H, 4.30(m) 1-H, 7.35(m) 1H, 7.60(m) 1H, 7.80(m) 1H, 8.10(D) 1H, 8.25(m) 2H, 8.32(S) 1H, 8.35(m) 1H.

IR data: KBR CO, cm⁻¹ 1630, 1680, 1730.

### Example 24

### 1-(2,4-Difluorophenyl)-6-Fluoro-7-(3-Gly-L-Phe -Amino-1-Pyrrolidinyl)-1,4-Dihydro-4-Oxo-Quinoline-3-Carboxylic Acid Hydrochloride

(a) To a cold suspension of 701 mg 1-(2,4-difluorophenyl)-6-fluoro-7-(3-amino-1-pyrrolidinyl)-1,4-dihydro-4-oxoquinoline-3-carboxylic acid hydrochloride in 30 ml of dry DMF is added 795 mg of N-carbobenzyloxyglycyl-phenylalanine N-hydroxysuccinimide ester followed by 354 mg of N-methylmorpholine. The mixture is stirred in the cold for an additional hour, then at room temperature overnight. The resulting solution is poured into 200 ml of 0.5 NHCl and the mixture is extracted with 2 x 150 ml portions of CHCl₃. The combined extract is washed with 5% NaHCO₃ solution and the organic phase is dried over MgSO₄, evaporated to leave a solution of DMF and product. The residual DMF is removed by co-distillation with toluene with ethylene dichloride, with CHCl₃ and with CH₂Cl₂ to leave 1.21 grams of a yellow colored foam. Trituration with IPA caused the foam to crystallize. Yield 936 mg (79%), mp=125°-128°C, [delta]_{D} = -16.5° (CHCl₃)
   NMR data, CDCl₃ delta: 1.65(s) 2H, 2.00(m) 2H, 3.10(m) 2H, 3.50(m) 1H, 3.75(m) 2H, 4.50(m) 1H, 4.75(m) 1H, 4.98(s) 2H, 5.40(m) 1H, 5.65(m) 1H, 6.60(m) 1H, 7.10(m) 1H, 7.25(m) 10H, 7.50(m) 1H, 7.70(m) 1H, 8.11(s) 1H, 8.20(m) 1H, 8.25(D) 1H.
   IR data: (CDCl₃) CO, cm⁻¹ 1630, 1680, 1720.
(b) 891 mg of 1-(2,4-difluorophenyl)-6-fluoro-7-(3-N-carbobenzyloxygly-phe-amino-1-pyrrolidinyl)-1,4-dihydro-4-oxoquinoline-3-carboxylic acid from Example 24(a) above, 0.45 grams of 10% Pd/C (Dry) and 50 ml of Acetic Acid is reacted on a Parr Shaker Apparatus under 60 psi of hydrogen atmosphere for 24 hours. After the reaction is completed, the hydrogen is vented, the catalyst is removed by filtration and washed thoroughly. The filtrate is evaporated to leave a residue. This residue is treated with 0.5NHCl in ethanol and evaporated. The residual HCl is removed by co-distillation with ethanol to leave a yellow colored solid yield 708 mg (91%) the title compound. mp=205°C (dec), [delta]_{D} + 35.9 (CH₃OH)
   NMR data, DMSO delta: 1.23(S) 2H, 1.60(m) 2H, 1.75(m) 1H, 2.00(m) 2H, 2.85(m) 2H, 4.20(m) 2H, 4.50(m) 1H, 5.75(m) 1H, 7.20(m) 6H, 7.50(m) 1H, 7.80(m) 1H, 8.05(m) 2H, 8.40(m) 1H, 8.75(m) 1H, 8.77(S) 1H.
   IR Data (KBR) CO cm⁻¹ 1635, 1670, 1720

### Example 25

Using the process as described in Example 24a, using or replacing 1-(2,4-difluorophenyl)-6-fluoro-7-(3-amino-1-pyrrolidinyl)-1,4-dihydro-4-oxoquinoline- 3-carboxylic acid with an appropriate quinolone and naphthyridine derivative such as 1-(4-fluorophenyl)-6-fluoro-7-(3-amino-1-pyrrolidinyl)-1,4-dihydro-4- oxoquinoline-3-carboxylic acid,1-(4-fluorophenyl)-6-fluoro-7-(3-amino-1-pyrrolidi nyl-1,4-dihydro-4-oxo- 1,8-naphthyridine-3-carboxylic acid, 1-cyclopropyl-6-fluoro-7-(3-amino-1-pyrrolidinyl)-1,4-dihydro-4-oxo-quinoline-3-carboxylic acid, 1-cyclopropyl-6-fluoro-7-(3-amino-1-pyrrolidinyl)-1,4-dihydro-4-oxo-1,8-naphthyridine-3-carboxylic acid, 1-cyclopropyl-6,8-difluoro-7-(3-amino-1-pyrrolidinyl)-1,4-dihydro-4-oxoquinoline-3-carboxylic acid, 1-cyclopropyl-7-(3-amino- 1-pyrrolidinyl)-6-fluoro-8-chloro-1,4-dihydro-4-oxo-quinoline-3-carboxylic acid, 1-cyclopropyl-7-(3-amino-1-pyrrolidinyl)-5-amino-6,8-difluoro-1,4-dihydro-4-oxoquinoline-3-carboxylic acid, 1-t-butyl-7-(3-amino-1-pyrrolidinyl)-6-fluoro-1,4-dihydro-4-oxoquinoline-3-carboxylic acid, 1-t-butyl-7-(3-amino-1-pyrrolidinyl)-6,8-difluoro-1,4-dihydro-4-oxoquinoline-3-carboxylic acid, 1-t-butyl-7-(3-amino-1-pyrrolidinyl)-6-fluoro-1,4-dihydro-4-oxo-1,8-naphthyridine-3-carboxylic acid, 1-(2-fluoroethyl)-7-(3-amino-1-pyrrolidinyl)-6-fluoro-1,4-dihydro-4- oxoquinoline-3-carboxylic acid, 1-(2-fluoroethyl)-7-(3-amino-1-pyrrolidinyl)-6,8-difluoro-1,4-dihydro-4- oxoquinoline-3-carboxylic acid, 1-(2-fluoroethyl)-7-(3-amino-1-pyrrolidinyl)-6-fluoro-1,4-dihydro-4-oxo-1,8-naphthyridine-3-carboxylic acid, 1-ethyl-7-(3-amino-1-pyrrolidinyl)-6-fluoro-1,4-dihydro-4-oxoquinoline-3-carboxylic acid or 1-ethyl-7-(3-amino-1-pyrrolidinyl)-6-fluoro-1,4-dihydro-4-oxo-1,8-naphthyridine-3-carboxylic acid, 1-cyclopropyl-6-fluoro-7-(3-amino-1-pyrrolidinyl)-8-methyl-1,4-dihydro-4-oxo-quinoline-3-carboxylic acid and substituting various amino acids or peptides for one solubilizing group A, the following compounds as shown in the Table I are made.

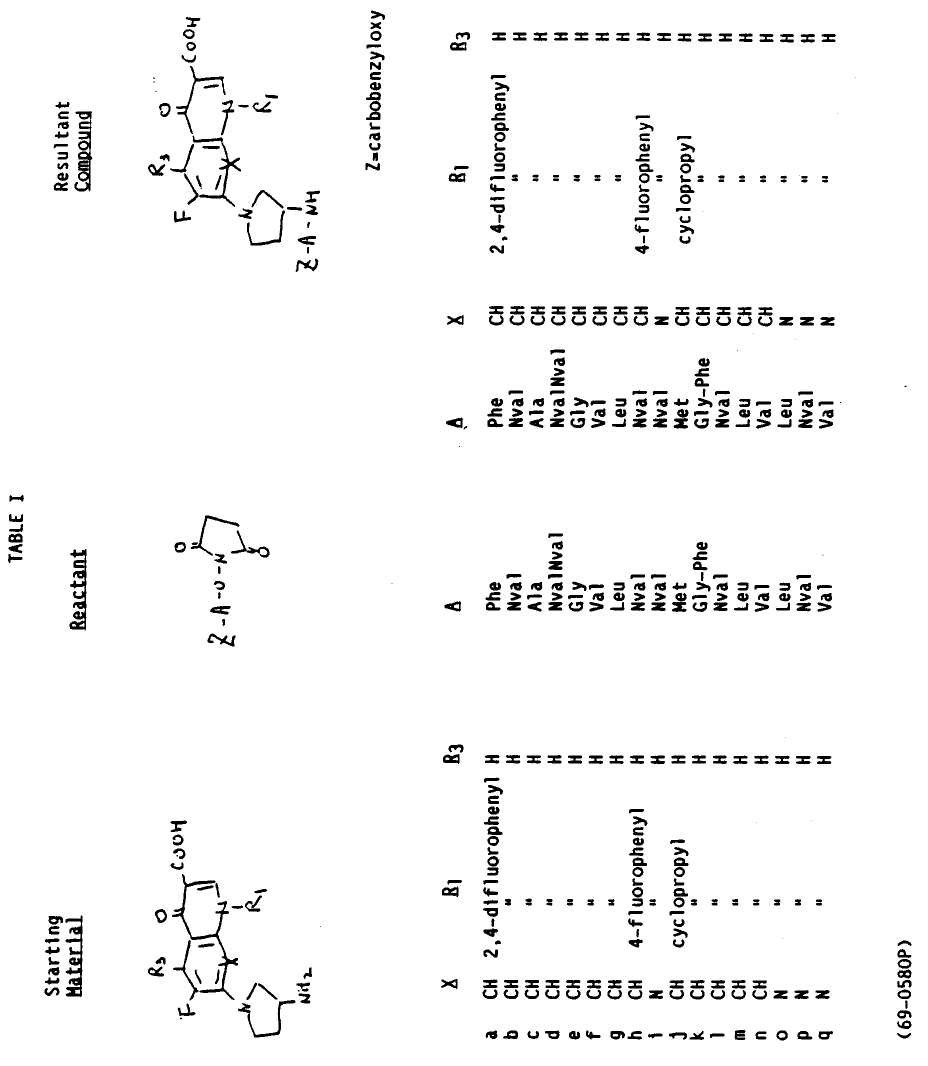

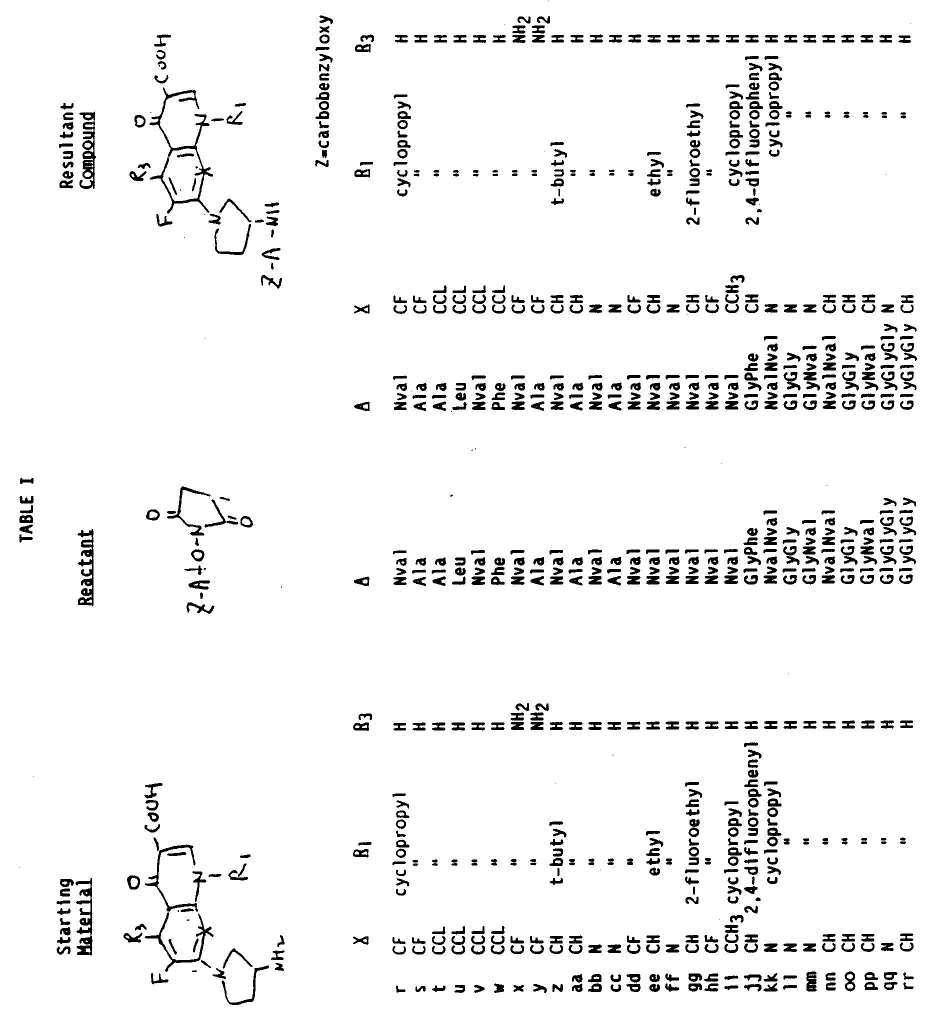

### Table IA

The physical data for the products in the designated Examples is as follows:
Example 25 b
   mp. = 195° - 197°C
   MS (m+1)⁺ = 637 m/z
   [alpha]_{D} = + 19.5 (CHCl₃)
   IR (CDCl₃) CO, cm⁻¹: 1630, 1670, 1720
   NMR(CDCl₃) delta: 0.90 (m) 3H, 1.35 (m) 2H, 1.82 (m) 1H, 2.10 (m) 2H, 2.20 (m) 1H, 3.07 (m) 1H, 3.24 (m) 1H, 3.75 (m) 1H, 3.90 (m) 2H, 4.25 (m) 2H, 4.56 (m) 2H, 5.05 (s) 2H, 5.47 (m) 1H, 5.72 (m) 1H, 7.25 (m) 7H, 7.73 (m) 1H, 8.03 (m) 1H, 8.32 (m) 1H.
Example 25 h
   mp. = 182° - 184°C
   MS (m+1)⁺ = 619 m/z
   [alpha]_{D} = + 22.8 (CHCl₃)
   IR (CDCl₃) CO, cm⁻¹: 1630, 1670, 1720
   NMR (CDCl₃) delta: 0.90 (m) 3H, 1.35 (m) 2H, 1.63 (m) 2H, 1.81 (m) 1H, 2.05 (m) 2H, 2.18 (m) 1H, 3.20 (m) 1H, 3.35 (m) 1H, 3.75 (m) 2H, 4.30 (m) 1H, 4.50 (m) 1H, 4.60 (m) 1H, 5.05 (s) 2H, 5.47 (d) 1H, 5.55 (d) 1H, 5.78 (m) 1H, 7.25 (m) 7H, 7.45 (m) 1H, 7.70 (d) 1H, 7.90 (m) 1H, 8.00 (m) 1H, 8.10 (2-s) 1H.
Example 25 i
   mp = 186° - 188°C
   MS (m+1)⁺ = 620 m/z
   [alpha]_{D} = +10.6 (CHCl₃)
   IR (CDCl₃) CO, cm⁻¹: 1630, 1680, 1725
   NMR (CDCl₃) delta: 0.82 (m) 3H, 1.25 (m) 2H, 1.45 (m) 2H, 1.75(m) 1H, 2.00(m) 1H, 3.87 (m) 2H, 4.23(m) 1H, 4.98 (s) 2H, 7.33 (m) 7H, 7.65 (m) 2H, 8.07 (d) 1H, 8.20 (m) 1H, 8.65 (s) 1H.
Example 25 l
   mp. = 195° - 197°C
   MS (m+1)⁺ = 565 m/z
   [alpha]_{D} = + 16.7 (CHCl₃)
   IR (CDCl₃) CO, cm⁻¹: 1630, 1665, 1720
   NMR (CDCl₃) delta: 0.90 (m) 3H, 1.30 (m) 4H, 1.87 (m) 1H, 2.15 (m) 1H, 2.33 (m) 1H, 3.46 (m) 1H, 3.60 (m) 2H, 3.92 (m) 1H, 4.35 (m) 1H, 5.05 (s) 2H, 6.72 (m) 1H, 7.25 (m) 5H, 7.63 (d) 1H, 7.98 (d) 1H, 8.25 (m) 1H.
Example 25 o
   mp. = 102° - 104°C
   MS (m+1)⁺ = 580 m/z
   [alpha]_{D} = + 1.3 (CHCl₃)
   IR (CDCl₃) CO, cm⁻¹: 1630, 1670, 1710
   NMR(CDCl₃) delta: 0.90 (m) 6H, 1.20(m) 2H, 1.58 (m) 2H, 2.15 (m) 2H, 3.60 (m) 1H, 4.07 (m) 2H, 4.35 (m) 1H, 4.70 (m) 1H, 5.05 (s) 2H, 5.42 (m) 1H, 7.25 (m) 5H, 7.75 (d) 1H, 8.05 (m) 1H, 15.80 (s) 1H.
Example 25 p
   mp. = 103° - 105°C
   MS (m+1)⁺ = 566 m/z
   [alpha]_{D} = + 7.8 (CHCl₃)
   IR (CDCl₃) CO, cm⁻¹: 1630, 1670, 1720
   NMR(CDCl₃) delta: 0.90 (m) 3H, 1.20(m) 2H, 1.40 (m) 2H, 1.70 (m) 2H, 1.90 (m) 1H, 2.20 (m) 2H, 3.60 (m) 1H, 4.10 (m) 2H, 4.30 (m) 1H, 4.70 (m) 2H, 5.05 (s) 2H, 5.50 (d) 1H, 7.25 (m) 6H, 7.75 (d) 1H, 8.05 (s) 1H.
Example 25 r
   mp. = 188° - 190°C
   MS (m+1)⁺ = 583 m/z
   [alpha]_{D} = + 11.8 (CHCl₃)
   IR (KBr) CO, cm⁻¹: 1630, 1680, 1730
   NMR(DMSO) delta: 0.84 (m) 3H, 1.16 (m) 3H, 1.30 (m) 2H, 1.52 (m) 2H, 1.86 (m) 1H, 2.10 (m) 1H, 3.50 (m) 1H, 3.75 (m) 1H, 3.96 (m) 2H, 4.08 (m) 1H, 4.30 (m) 1H, 5.00 (2-s) 2H, 7.35 (m) 5H, 7.72 (m) 1H, 8.25 (m) 1H, 8.61 (s) 1H.
Example 25 s
   mp. = 206° - 208°C
   MS (m+1)⁺ = 555 m/z
   [alpha]_{D} = + 19.5 (CHCl₃)
   IR (CDCl₃) CO, cm⁻¹: 1620, 1665, 1720
   NMR(CDCl₃) delta: 1.20 (m) 5H, 1.45 (m) 3H, 2.08 (m) 2H, 2.22 (m) 1H, 3.85 (m) 6H, 4.35 (m) 1H, 4.54 (m) 1H, 5.10 (2s) 2H, 5.65 (m) 1H, 7.25 (m) 5H, 7.60 (d) 1H, 7.75 (m) 1H, 8.20 (2s) 1H.
Example 25 ff
   mp. = 112° - 114°C
   MS (m+1)⁺ = 554 m/z
   [alpha]_{D} = + 8.5 (CHCl₃)
   IR (KBr) CO, cm⁻¹: 1630, 1675, 1720
   NMR(DMSO) delta: 0.80 (m) 3H, 1.25 (m) 2H, 1.36 (m) 2H, 1.50 (m) 2H, 1.90 (m) 1H, 2.15 (m) 1H, 3.70 (m) 1H, 3.92 (m) 4H, 4.45 (m) 4H, 5.00 (2-s) 2H, 7.35 (m) 5H, 8.00 (d) 1H, 8.30 (m) 1H, 8.93 (s) 1H.
Example 25 jj
   mp = 125° - 128°C
   MS (m+1)⁺ = 742 m/z
   [alpha]_{D} = -16.5 (CHCl₃)
   IR (CDCl₃) CO, cm⁻¹: 1630, 1665, 1680, 1717
   NMR (CDCl₃) delta: 1.65 (s) 3H, 1.95 (m) 2H, 3.12 (m) 3H, 3.50 (m) 1H, 3.75 (m) 3H, 4.46 (m) 1H, 4.75 (m) 1H, 5.02 (2-s) 2H, 5.40 (m) 1H, 5.77 (m) 1H, 6.57 (m) 1H, 7.23 (m) 12H, 7.72 (m) 2H, 8/13 (s) 1H, 8.24 (m) 1H.

### Example 26

Using the process as described in Example 24(b), the compounds of Example 25 (a-jj) yield the following corresponding quinolone and naphthyridine derivatives (a-jj) and their hydrochloride salts as shown in Table II.

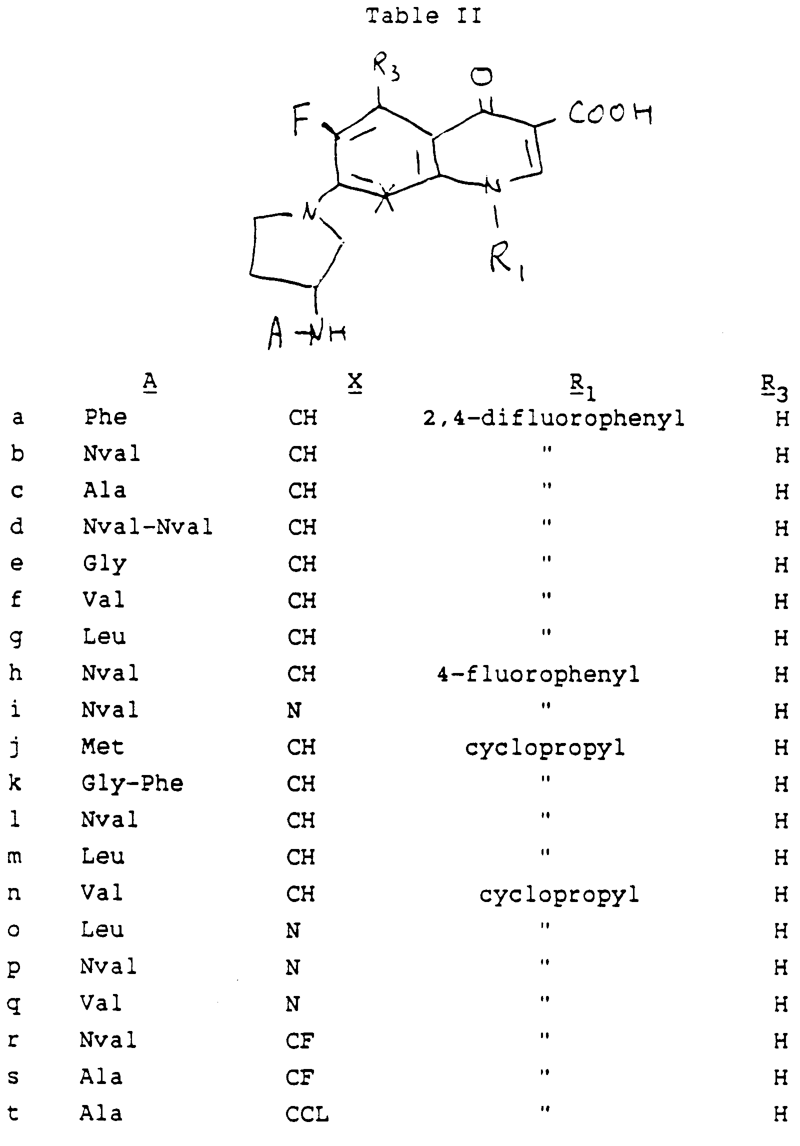

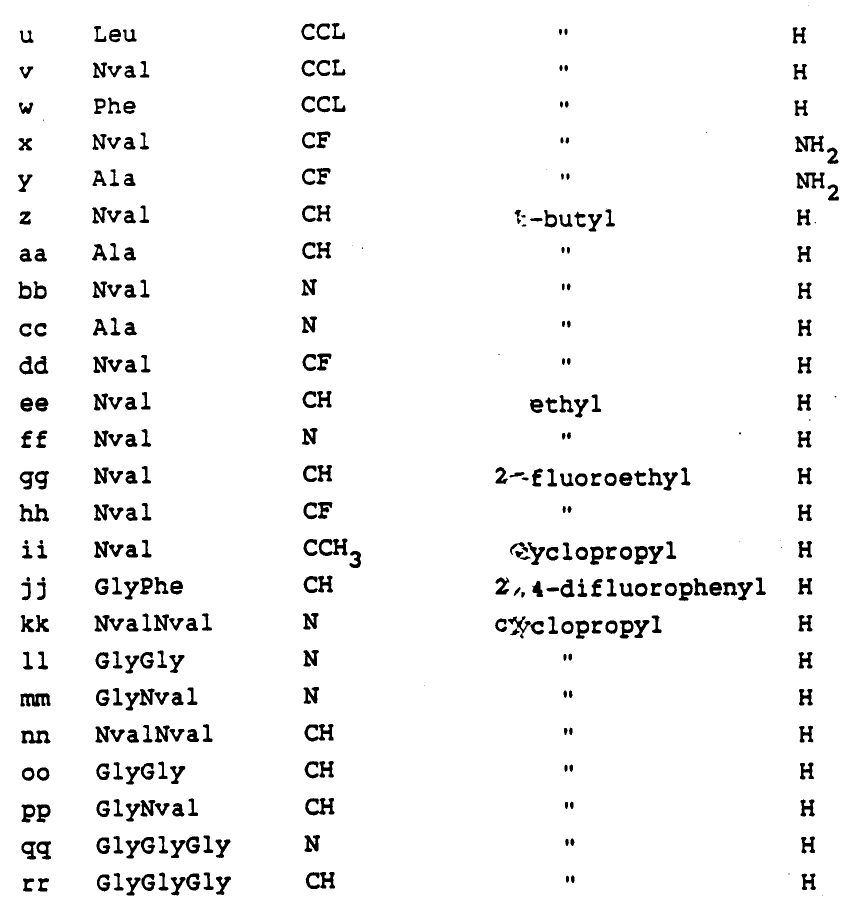

### Table IIA

The physical data for the hydrochloride salt of the products in the designated Examples is as follows:
Example 26 b
   mp. = 205°C (dec.)
   MS (m+1)⁺ = 503 m/z
   [alpha]_{D} = + 8.3 (CH₃OH)
   IR (KBr) CO, cm⁻¹: 1630, 1683, 1730
   NMR(DMSO) delta: 0.85 (m) 3H, 1.26 (m) 3H, 1.63 (m) 2H, 1.90 (m) 1H, 2.10 (m) 1H, 3.67 (m) 2H, 4.34 (m) 1H, 5.82 (d) 1H, 7.45 (m) 1H, 7.75 (m) 1H, 7.92 (m) 2H, 8.21 (m) 2H, 8.75 (s) 1H, 8.85 (m) 1H.
Example 26 h
   mp. = 196° - 198°C
   MS (m+1)⁺ = 485 m/z
   [alpha]_{D} = + 11.9 (MeOH)
   IR (KBr) CO cm⁻¹: 1630, 1680, 1720
   NMR (DMSO) delta: 0.85 (m) 3H, 1.23 (m) 2H, 1.65 (m) 2H, 1.90 (m) 2H, 2.10 (m) 2H, 3.65 (m) 2H, 4.33 (m) 1H, 5.88 (d) 1H, 7.55 (m) 2H, 7.75 (m) 2H, 7.90 (d) 1H, 8.20 (m) 2H, 8.55 (s) 1H, 8.86 (d) 1H.
Example 26 i
   mp. = 208° - 210°C
   MS (m+1)⁺ = 486 m/z
   [alpha]_{D} = +6.0 (meOH)
   IR(KBr) CO, cm⁻¹: 1630, 1680, 1730
   NMR (DMSO) delta: 0.85 (m) 3H, 1.25 (m) 3H, 1.64(m) 2H, 1.85(m) 1H, 2.08(m) 1H, 3.65 (m) 1H, 4.32 (m) 1H, 7.43 (m) 2H, 7.67 (m) 2H, 8.08 (d) 1H, 8.22 (m) 2H, 8.65 (s) 1H, 8.85 (m) 1H
Example 26 l
   mp. = 202° - 204°C
   MS (m+1)⁺ = 431 m/z
   [alpha]_{D} = + 23.1 (DMSO)
   IR (KBr) CO, cm⁻¹: 1630, 1680, 1720
   NMR (DMSO) delta: 0.85 (m) 3H, 1.15 (m) 3H, 1.30 (m) 4H, 1.70 (m) 2H, 2.00 (m) 2H, 2.25 (m) 1H, 3.55 (m) 1H, 3.75 (m) 4H, 3.90 (m) 1H, 4.45 (m) 1H, 7.06 (m) 1H, 7.81 (d) 1H, 8.30 (m) 2H, 8.58 (s) 1H, 9.03 (m) 1H.
Example 26 o
   mp. = 204° - 206°C
   MS (m+1)⁺ = 446 m/z
   [alpha]_{D} = + 29.6 (DMSO)
   IR (KBr) CO, cm⁻¹: 1630, 1680, 1710
   NMR(DMSO) delta: 0.81 (m) 6H, 1.10(m) 2H, 1.18 (m) 2H, 1.55 (m) 2H, 2.08 (m) 1H, 2.23 (m) 1H, 3.71 (m) 2H, 3.95 (m) 2H, 4.42 (m) 1H, 8.02 (d) 1H, 8.25 (m) 2H, 8.57 (s) 1H, 8.95 (m) 1H.
Example 26 p
   mp. = 204° - 206°C
   MS (m+1)⁺ = 432 m/z
   [alpha]_{D} = + 18 (DMSO)
   IR (KBr) CO, cm⁻¹: 1630, 1680, 1710
   NMR(DMSO) delta: 0.85 (m) 3H, 1.10 (m) 2H, 1.18 (m) 2H, 1.30 (m) 2H, 1.70 (m) 2H, 2.00 (m) 1H, 2.20 (m) 1H, 3.70 (m) 2H, 3.91 (m) 2H, 4.42 (m) 1H, 8.01 (d) 1H, 8.21 (m) 2H, 8.59 (s) 1H, 8.96 (m) 1H.
Example 26 r
   mp. = 175° - 177°C
   MS (m+1)⁺ = 449 m/z
   [alpha]_{D} = + 8.9 (MeOH)
   IR (KBr) CO, cm⁻¹: 1625, 1680, 1730
   NMR(DMSO) delta: 0.82, 0.90 (m) 3H, 1.17 (m) 3H, 1.30 (m) 2H, 1.70 (m) 2H, 1.95 (m) 1H, 2.15 (m) 1H, 3.58 (m) 1H, 3.75 (m) 2H, 3.95 (m) 2H, 4.10 (m) 1H, 4.37 (m) 1H, 7.73 (d) 1H, 8.30 (m) 2H, 8.62 (s) 1H, 9.00 (m) 1H.
Example 26 s
   mp. = 185° - 187°C
   MS (m+1)⁺ = 421 m/z
   [alpha]_{D} = + 14.3 (MeOH)
   IR (KBr) CO, cm⁻¹: 1625, 1680, 1730
   NMR(DMSO) delta: 1.17 (m) 3H, 1.36 (m) 3H, 1.95 (m) 2H, 2.15 (m) 2H, 3.55 (m) 2H, 3.80 (m) 2H, 3.95 (m) 2H, 4.10 (m) 1H, 4.35 (m) 2H, 7.73 (d) 1H, 8.22 (m) 2H, 8.63 (s) 1H, 8.90 (m) 1H.
Example 26 ff
   mp. = 190° - 192°C
   MS (m+1)⁺ = 420 m/z
   [alpha]_{D} = + 5.9 (MeOH)
   IR (KBr) CO, cm⁻¹: 1630, 1680, 1720
   NMR(CDCl₃) delta: 1.91 (m) 5H, 2.13 (m) 2H, 2.32 (m) 1H, 3.47 (m) 6H, 3.65 (m) 3H, 4.30 (m) 1H, 4.48 (m) 2H, 5.10 (m) 3H, 7.10 (m) 3H, 7.32 (m) 5H, 7.94 (m) 1H, 8.51 (m) 1H.
Example 26 jj
   mp = 205°C (dec.)
   MS (m+1)⁺ = 608 m/z
   [alpha]_{D} = +35.9 (CH₃OH)
   IR (KBr) CO, cm⁻¹: 1630, 1670, 1720
   NMR (DMSO) delta: 1.60 (m) 1H, 1.75 (m) 1H, 2.84 (m) 2H, 4.23 (m) 1H, 4.52 (m) 1H, 5.75 (m) 1H, 7.17 (m) 4H, 7.48 (m) 1H, 7.77 (m) 1H, 7.90 (m) 1H, 8.03 (m) 2H, 8.75 (m) 1H.

### Example 27

Using the process as described in Example 2a and substituting Pro for the solubilizing group A yields the protected Proline compound (I) (R₁ = 2,4-difluorophenyl, X=N, R₂ = R₃ = R₄ = H, A = carbobenzyloxy Pro). mp. = 217°-218°C.
[alpha]_{D} = -27.8°(CHCl₃)
IR (CDCl₃) CO,cm⁻¹: 1630, 1680, 1720
NMR (CDCl₃) delta: 1.91 (m) 5H, 2.13 (m) 2H, 2.32 (m) 1H, 3.47 (m) 6H, 3.65 (m) 3H, 4.30 (m) 1H, 4.48 (m) 2H, 5.10 (m) 3H, 7.10 (m) 3H, 7.32 (m) 5H, 7.94 (m) 1H, 8.51 (m) 1H.

### Example 28

Using the process as described in Example 2b with the compound of Example 27 yields the deblocked Pro(HCl) salt (I) (R₁ = 2,4-difluorophenyl, X=N, R₂ = R₃ = R₄ = H, A = Pro). mp. = 203°C (dec.).
MS (m+1)⁺ = 502 m/z
[alpha]_{D} = - 18.3 (CH₃OH)
IR (KBr) CO, cm⁻¹: 1630, 1677, 1730
NMR(DMSO) delta: 1.85 (m) 4H, 2.07 (m) 1H, 2.25 (m) 1H, 3.20 (m) 4H, 4.10 (m) 1H, 4.32 (m) 1H, 7.35 (m) 1H, 7.61 (m) 1H, 7.80 (m) 1H, 8.10 (d) 1H, 8.52 (m) 1H, 8.82 (s) 1H, 8.89 (m) 1H, 9.82 (m) 1H.

### Example 29

Using the process as described in 2a and substituting Gly-Gly-Nval for the solubilizing group A yields the protected Gly-Gly-Nval compound (I) (R₁ = 2,4-difluorophenyl, X=N, R₂ = R₃ = R₄ = H, A = Carbobenzyloxy Gly-Gly-Nval).
MS (m+1)⁺ = 752 m/z, mp. = 118° - 120°C.
[Alpha]_{D} = + 8.0 (DMSO)
IR (KBr) CO, cm⁻¹: 1630, 1670, 1725
NMR(CDCl₃) delta: 0.90 (m) 3H, 1.35 (m) 3H, 1.95 (m) 3H, 3.70 (m) 2H, 3.80 (m) 2H, 3.95 (m) 2H, 4.43 (m) 2H, 5.05 (m) 2H, 5.50 (m) 1H, 6.86 (m) 2H, 7.05 (m) 2H, 7.35 (m) 5H, 7.95 (d) 1H, 8.55 (s) 1H.

### Example 30

Using the process as described in 2b with the compound of Example 29 yields the deblocked Gly-Gly-Nval (HCl) salt (I) (R₁ = 2,4-difluorophenyl, x=n, R₂ = R₃ = R₄ = H, A = Gly-Gly-Nval). mp. = 212°C (dec.).
MS (m+1)⁺ = 618 m/z [Alpha]_{D} = + 7.1 (DMSO)
IR (KBr) CO, cm⁻¹: 1635, 1670, 1730
NMR(DMSO) delta: 0.83 (m) 3H, 1.21 (m) 2H, 1.50 (m) 2H, 1.80 (m) 1H, 2.03 (m) 1H, 3.68 (s) 2H, 3.83 (d) 2H, 4.21 (m) 2H, 7.34 (m) 1H, 7.59 (m) 1H, 7.80 (m) 1H, 8.10 (m) 2H, 8.15 (m) 1H, 8.32 (m) 1H, 8.60 (m) 1H, 8.82 (s) 1H.

### Example 31

### 7-[3-L-Methioninesulfone-Amino-1-Pyrrolidinyl]-1-(2,4-Difluorophenyl)-6-Fluoro-1,4-Dihydro-4-Oxo 1,8-Naphthyridine-3-Carboxylic Acid Hydrochloride.

a) To a cold solution of 7-(3-amino-1-pyrrolidinyl)-1-(2,4-difluorophenyl)-6-fluoro 1,4-dihydro-4-oxo-1,8-naphthyridine-3-carboxylic acid tosylate (4.50g) in dry DMF (90 ml) is added N-t-Boc-L-methionine-p-nitrophenyl ester (3.47g) followed by N-methylmorpholine (1.74g). The mixture is stirred in the cold for an additional hour, then at room temperature for about 17-18 hours. The resulting solution is poured into 500 ml of 0.5N HCl. and the mixture is extracted with 2 x 250 ml portion of CHCl₃. The combined extracts are washed with 5% NaHCO₃ solution and the organic phase is dried over MgSO₄ and evaporated to leave a solution of DMF and product. The residual DMF is removed by co-distillation with toluene, with ethylene dichloride, with CHCl₃ and CH₂Cl₂ to leave 5.06 grams of a light yellow colored foam. Trituration with ether caused the foam to crystallize and yield 4.43 grams of 7-(3-N-t-BOC-L-methionine-amino-1-pyrrolidinyl)-1-(2,4-difluorophenyl)-6-fluoro-1,4-dihydro-4-oxo-1,8-naphthyridine-3-carboxylic acid (I) (R₁ = 2,4 - difluorophenyl, X=N, R₂ = R₃ = R₄ = H, A = t-Boc-Met).
   mp. = 214°C (dec), [alpha]_{D} = - 4.9 (CHCl₃)
   IR (CDCl₃) CO, cm⁻¹: 1630, 1673, 1717
   NMR(CDCl₃) delta: 1.40 (s) 9H, 1.90 (m) 2H, 2.10 (2-s) 3H, 2.53 (m) 2H, 4.23 (m) 1H, 4.53 (m) 1H, 5.22 (m) 1H, 7.05 (m) 3H, 7.94 (m) 1H, 8.48 (m) 1H.
b) To a cold solution of 7-(3-N-t-butoxycarbonyl-L-methionine-amino-1-pyrrolidinyl)-1-(2,4-difluorophenyl)-6-fluoro-1,4-dihydro-4-oxo-1,8-naphthyridine-3-Carboxylic Acid (2.16 g) in CH₂Cl₂ (50 ml) is added m-chloroperbenzoic acid (1.75g). This solution is stirred in the cold for an additional hour, then at room temperature of 17-18 hours. To the solution is added 1 ml of cyclohexene and stirred at room temperature for an hour. The reaction is diluted with CHCl₃ and washed with 3 x 250 ml portions of 5% NaHCO₃ solution. The organic phase is dried over MgSO₄ and evaporated to leave 2.04g of a tan colored foam. Trituration with ether caused the foam to crystallize and yield 1.86 grams of 7-(3-N-t-Boc-L-methionine-sulfone-amino-1-pyrrolidinyl]-1-(2,4-difluorophenyl)-6-fluoro-1,4-dihydro-4-oxo-1,8-naphthyridine-3-carboxylic acid (I) (R₁ = 2,4-difluorophenyl, X = N, R₂ = R₃ = R₄ = H, A = t-BOC-NH-CH(CH₂CH₂-SO₂CH₃) CO)
   mp. = 160°C (dec), MS (m+1)⁺ = 668 m/z, [Alpha]_{D} = +13.8 (CHCl₃)
   IR (CDCl₃) CO, cm⁻¹: 1630, 1680, 1710.
   NMR(CDCl₃) delta: 1.40 (s) 9H, 2.10 (m) 3H, 2.40 (m) 2H, 2.90 (2-s) 3H, 3.10 (m) 4H, 3.35 (m) 2H, 4.00 (m) 2H, 4.35 (m) 2H, 4.55 (m) 2H, 5.50 (m) 2H, 7.05 (m) 2H, 7.90 (m) 2H, 8.38 (m) 1H, 8.43 (s) 1H.
c) A solution of 7-(3-N-t-butoxy-carbonyl L-methionine-sulfone-amino-1-pyrrolidinyl]-1-(2,4-difluoro phenyl)-6-fluoro-1,4-dihydro-4-oxo-1,8-naphthyridine-3-carboxylic acid (1.81g) in 1M HCl in HOAc (20 ml) is stirred at room temperature for two hours. This solution is diluted with EtOH and evaporated to leave a yellow colored syrup. The residual HCl and HOAC is removed by co-distillation with Etoh to leave 1.60 grams of a yellow colored solid, 7-(3-L-methionine sulfone-amino-1-pyrrolidinyl)-1-(2,4-difluorophenyl)-6-fluoro-1,4-dihydro-4-oxo-1,8-naphthyridine-3-carboxylic acid hydrochloride (I) (R₁ = 2,4-difluorophenyl, X = N, R₂ = R₃ = R₄ = H, A = NH₂ - CH(CH₂CH₂SO₂CH₃)CO).
   mp. = 185°C (dec), MS (m+1)⁺ = 568 m/z, [Alpha]_{D} = +5.3 (MeOH), IR (KBr) CO, cm⁻¹: 1630, 1675, 1720.
   NMR(DMSO) delta: 1.90 (m) 1H, 2.15 (m) 3H, 3.00 (2-s) 3H, 3.15 (m) 2H, 3.37 (m) 2H, 4.33 (m) 1H, 7.35 (m) 1H, 7.60 (m) 1H, 7.80 (m) 1H, 8.10 (d) 1H, 8.40 (m) 2H, 8.83 (s) 1H, 9.00 (m) 1H.

### Example 32

Using the process as described in Example 31a and replacing 7-(3-amino-1-pyrrolidinyl)-1-(2,4-difluorophenyl)-6-fluoro -1,4-dihydro-4-oxo-1,8-naphthyridine-3-carboxylic acid with 7-(3-amino-1-pyrrolidinyl)-1-cyclopropyl-6-fluoro-1,4-dihydro-4-oxoquinoline-3-carboxylic acid one obtains 7-(3-N-t-Boc-L-methionine-amino-1-pyrrolidinyl)-1-cyclopropyl-6-fluoro-1,4-dihydro-4-oxoquinoline-3-carboxylic acid (I) (R₁ = cyclopropyl, X = CH, R₂ = R₃ = R₄ = H, A = t-Boc-Met). mp. = 198°C (dec), MS (m+1)⁺ = 563 m/z, [Alpha]_{D} = +8.0 (CHCl₃),
IR (CDCl₃) CO, cm⁻¹: 1625, 1665, 1710.
NMR(CDCl₃) delta: 0.96 (m) 1H, 1.35 (m) 1H, 1.40 (2-s) 9H, 1.95 (m) 1H, 2.10 (2-s) 3H, 2.20 (m) 1H, 2.54 (m) 4H, 3.48 (m) 1H, 3.65 (m) 1H, 3.94 (m) 2H, 4.35 (m) 1H, 4.65 (m) 1H, 5.44 (m) 1H, 6.75 (2-d) 1H, 7.66 (2-d) 1H, 8.08 (2-s) 1H, 8.20 (m) 1H.

### Example 33

Using the process as described in Example 31c, the product of Example 32 yields 7-(3-L-methionine-amino-1-pyrrolidinyl)-1-cyclopropyl-6-fluoro-1,4-dihydro-4-oxoquinoline-3-carboxylic acid hydrochloride (I) (R₁ = cyclopropyl, X = CH, R₂ = R₃ = R₄ = H, A = Met). mp. = 185° -186°C, MS (m+1)⁺ = 463 m/z, [Alpha]_{D} = +2.3 (DMSO)
IR (KBr) CO, cm⁻¹: 1625, 1680, 1710.
NMR(DMSO) delta: 1.15 (m) 2H, 1.30 (m) 2H, 1.95, 2.08 (2-s) 3H, 2.00 (m) 3H, 2.25 (m) 1H, 3.55 (m) 1H, 3.75 (m) 4H, 3.85 (m) 3H, 4.45 (m) 1H, 7.06 (d) 1H, 7.82 (d) 1H, 8.35 (m) 2H, 8.58 (s) 1H, 9.05 (m) 1H.

### Example 34

Using the process as described in Example 31b, the product of Example 32 yields 7-(3-L-methionine sulfone-amino-1-pyrrolidinyl)-1-cyclopropyl-6-fluoro-1,4-dihydro-4-oxo-quinoline-3-carboxylic acid (I) (R₁ = cyclopropyl, X = CH, R₂ = R₃ = R₄ = H, A = t-Boc-NH-CH(CH₂CH₂SO₂CH₃)CO). mp. = 156-8°C, MS (m+1)⁺ = 595 m/z, [alpha]_{D} = +20 (CHCl₃) delta: 1.41 (s) 9H, 2.22 (m) 3H, 2.48 (m) 2H, 2.90 (2-s) 3H, 3.11 (m) 2H, 3.48 (m) 1H, 3.65 (m) 2H, 3.95 (m) 2H, 4.40 (m) 1H, 4.60 (m) 1H, 5.58 (m) 1H, 6.77 (d) 1H, 7.66 (m) 1H, 8.05 (2-s) 1H, 8.40 (m) 1H.

### Example 35

Using the process as described in Example 31c, the product of Example 34 yields 7-(3-L-methionine sulfone-amino-1-pyrrolidinyl)-1-cyclopropyl-6-fluoro-1,4-dihydro-4-oxoquinoline-3-carboxylic acid hydrochloride (I) (R₁ = cyclopropyl, X = CH, R₂ = R₃ = R₄ = H, A = NH₂ CH (CH₂CH₂SO₂CH₃)CO).

### Example 36

Using the process as described in Example 31a and replacing 7-(3-amino-1-pyrrolidinyl)-1-(2,4-difluorophenyl)-6-fluoro-1,4-dihydro-4-oxo-1,8-naphthyridine-3-carboxylic acid with 7-(3-amino-1-pyrrolidinyl)-1-cyclopropyl-6,8-difluoro-1,4-dihydro-4-oxoquinoline-3-carboxylic acid one obtains 7-(3-N-t-Boc-L-methionine-amino-1-pyrrolidinyl)-1-cyclopropyl-6,8-difluoro-1,4-dihydro-4-oxoquinoline-3-carboxylic acid (I) (R₁ = cyclopropyl, X = CF, R₂ = R₃ = R₄ = H, A = t-Boc-Met). mp. = 104 - 105°C, MS (m+1)⁺ = 581 m/z, [Alpha]_{D} = +12 (CHCl₃) IR (KBr) CO, cm⁻¹; 1620, 1670, 1710
NMR (CDCl₃) delta: 1.25 (m) 7H, 1.42 (s) 9H, 1.98 (m) 2H, 2.12 (2-s) 3H, 2.55 (m) 2H, 3.75 (m) 2H, 4.00 (m) 4H, 4.33 (m) 2H, 4.55 (m) 2H, 5.40 (m) 1H, 7.65 (m) 2H, 8.32 (2-s) 1H.

### Example 37

Using the process as described in Example 31c, the product of Example 36 yields 7-(3-L-methionine-amino-1-pyrrolidinyl)-1-cyclopropyl-6,8-difluoro-4-oxoquinoline-3-carboxylic acid hydrochloride (I) (R₁ = cyclopropyl, X = CF, R₂ = R₃ = R₄ = H, A = Met).

### Example 38

Using the process as described in Example 31b, the product of Example 36 yields 7-(3-L-methionine sulfone-amino-1-pyrrolidinyl)-1-cyclopropyl-6,8-difluoro-1,4-dihydro-4-oxo-quinoline-3-carboxylic acid (I) (R₁ = cyclopropyl, X = CF, R₂ = R₃ = R₄ = H, A = t - Boc - NH - CH(CH₂CH₂-SO₂CH₃)CO).

### Example 39

Using the process as described in Example 31c, the product of Example 38 yields 7-(3-L-methionine sulfone-amino-1-pyrrolidinyl)-1-cyclopropyl-6,8-difluoro-1,4-dihydro-4-oxoquinoline-3-carboxylic acid hydrochloride (I) (R₁ = cyclopropyl, X = CF, R₂ = R₃ = R₄ = H, A = NH₂ - CH(CH₂CH₂-SO₂CH₃)CO).

### Example 40

Using the process as described in Example 31a, replacing N-t-Boc-L-methionine-p-nitrophenyl ester with N-t-Boc-L-Asparagine-p-nitrophenyl ester, one can obtain 7-(3-N-t-Boc-L-Asparagine-amino-1-pyrrolidinyl)-1-(2,4-difluorophenyl)-6-fluoro-1,4-dihydro-4-oxo-1,8-naphthyridine-3-carboxylic acid (I) (R₁ = 2,4-difluorophenyl, X = N, R₂ = R₃ = R₄ = H, A = t-Boc-Asn). mp. = 147° (dec), MS (m+1)⁺ = 619 m/z, [Alpha]_{D} = +6.0 (MeOH), IR (CDCl₃) CO, cm⁻¹: 1630, 1680, 1720.
NMR (CDCl₃) delta: 1.40 (s) 9H, 1.95 (m) 2H, 2.15 (m) 2H, 2.56 (m) 2H, 2.90 (m) 2H, 4.45 (m) 4H, 5.45 (m) 1H, 5.90 (m) 1H, 6.05 (m) 1H, 7.10 (m) 2H, 7.47 (m) 1H, 8.00 (d) 1H, 8.60 (m) 1H.

### Example 41

Using the process as described in Example 31c, the product of Example 40 yields 7-(3-L-Asparagine-amino-1-pyrrolidinyl)-1-(2,4-difluorophenyl)-6-fluoro-1,4-dihydro-4-oxo-1,8-naphthyridine-3-carboxylic acid hydrochloride (I) (R₁ = 2,4-difluorophenyl, X = N, R₂ = R₃ = R₄ = H, A = Asn). mp. = 207° (dec), MS (m+1)⁺ = 519 m/z, [Alpha]_{D} = -9.4 (MeOH) IR (KBr) CO, cm⁻¹: 1635, 1680, 1720.
NMR (DMSO) delta: 1.83 (m) 1H, 2.05 (m) 1H, 2.63 (m) 2H, 3.95 (m) 2H, 4.30 (m) 2H, 7.20 (m) 1H, 7.35 (m) 1H, 7.63 (m) 1H, 7.80 (m) 1H, 8.09 (d) 1H, 8.17 (m) 2H, 8.78 (m) 1H, 8.82 (s) 1H.

### Example 42

Using the process as described in Example 40, replacing 7-(3-amino-1-pyrrolidinyl)-1-(2,4-difluorophenyl)-6-fluoro -1,4-dihydro-4-oxo-1,8-naphthyridine-3-carboxylic acid with 7-(3-amino-1-pyrrolidinyl)-1-cyclopropyl-6,8-difluoro-1,4-dihydro-4-oxoquinoline-3-carboxylic acid, one obtains 7-(3-N-t-Boc-L-Asparagine-amino-1-pyrrolidinyl)-2-cyclopropyl-6,8-difluoro-1,4-dihydro-4-oxo-quinoline-3-carboxylic acid (I) (R₁ = cyclopropyl X = CF, R₂ = R₃ = R₄ = H, A = t-Boc-Asn). mp. = 148 - 150° C. MS (m+1)⁺ = 564 m/z, [Alpha]_{D} = 21.3 (DMSO) IR (KBr) CO, cm⁻¹: 1625, 1680, 1720.
NMR (DMSO) delta: 1.15 (m) 5H, 1.36 (2-s) 9H, 1.90 (m) 1H, 2.08 (m) 1H, 2.38 (m) 2H, 3.53 (m) 1H, 3.75 (m) 1H, 3.90 (m) 2H, 4.09 (m) 1H, 4.20 (m) 1H, 4.31 (m) 1H, 6.85 (m) 2H, 7.25 (m) 1H, 7.72 (d) 1H, 8.12 (m) 1H, 8.61 (s) 1H.

### Example 43

Using the process as described in Example 31c, the product of Example 42 yields 7-(3-L-Asparagine-amino-1-pyrrolidinyl)-1-cyclopropyl-6,8-difluoro-1,4-dihydro-4-oxoquinoline-3-carboxylic acid hydrochloride (I). (R₁ =cyclopropyl, X = CF, R₂ = R₃ = R₄ = H, A = Asn). IR (KBr) CO, cm⁻¹: 1628, 1680, 1720.
NMR (DMSO) delta: 1.18 (m) 4H, 1.92 (m) 1H, 2.13 (m) 1H, 2.68 (m) 2H, 3.55 (m) 1H, 3.77 (m) 1H, 4.03 (m) 4H, 4.36 (m) 1H, 7.22 (d) 1H, 7.72 (m) 2H, 8.23 (m) 2H, 8.62 (s) 1H, 8.92 (d) 1H.

### Example 44

### 7-[3-L-Histidine-Amino-1-Pyrrolidinyl)-1-(2,4-Difluorophenyl)-6-Fluoro-1,4-Dihydro-4-oxo-1,8-Naphthyridine-3-Carboxylic Acid Dihydrochloride

a) To a cold solution of 7-(3-amino-1-pyrrolidinyl) 1-(2,4-difluorophenyl)-6-fluoro-1,4 dihydro-4-oxo-1,8-naphthyridine-3-carboxylic acid tosylate (5.05g) in dry DMF (75 ml) is added N-t-butoxycarbonyl-N-pi-benzyloxymethyl-L-histidine N-hydroxyphthalimide ester (5.46g) followed by N-methylmorpholine (3.19g). The mixture is stirred in the cold for an additional hour, then at room temperature for 17-18 hours. The resulting solution is poured into 500 ml of 5% NaHCO₃ solution and the mixture is extracted with 2 x 250 ml portion of CHCl₃. The combined extracts are dried over MgSO₄ and evaporated to leave a solution of DMF and product. The residual DMF is removed by co-distillation with toluene, with ethylene dichloride, CHCl₃ and CH₂Cl₂ to leave 5.15 g of a yellow colored foam. Trituration with ether caused the foam to crystallize and yield 3.28 grams 7-[3-(N-2-t-Boc-N-pi-benzyl oxymethyl-L-histidine-amino-1-pyrrolidinyl]-1-(2,4-difluorophenyl)-6-fluoro-1,4-dihydro-4-oxo-1,8-naphthyridine-3-carboxylic acid.
b) To a solution of 7-[3-(N-gamma-t -butoxycarbonyl-N-pi-benzyloxymethyl-L-histidine-amino-1-pyrrolidinyl)]-1-(2,4-difluorophenyl)-6-fluoro-1,4 -dihydro-4-oxo-1,8-naphthyridine-3-carboxylic acid (1.52g) in 1.0M HCl in HOAc (50 ml) is stirred at room temperature for 4 hours. This solution is evaporated to leave a residue which is co-distilled several times with IPA to leave 1.37 g of a light yellow colored solid, 7-[3-(N-pi-benzyloxymethyl-L-histidine-amino-1-pyrrolidinyl]-1-(2,4-difluorophenyl)-6-fluoro-1,4-dihydro-4-oxo-1,8-naphthyridine-3-carboxylic acid. MS (m+1)⁺ = 662 m/z
   IR (KBr) CO cm⁻¹: 1630, 1685, 1720
   NMR (DMSO) delta: 1.77 (m) 1H, 2.05 (m) 1H, 4.14 (m) 1H, 4.30 (m) 1H, 4.60 (d) 1H, 5.77 (m) 1H, 7.30 (m) 5H, 7.58 (m) 1H, 7.80 (m) 1H, 8.08 (m) 1H, 8.57 (m) 2H, 8.83 (s) 1H, 9.32 (m) 1H.
c) A mixture of 7-[3-N-pi-benzyloxymethyl-L-histidine-amino-1-pyrrolidinyl)]-1-(2,4-difluorophenyl)-6-fluoro-1,4-dihydro-4-oxo-1,8-naphthyridine-3-carboxylic acid (1.74g) 10% Pd/c (0.87g) and 100 ml acetic acid is reacted on a Parr Shaker apparatus under 60 psi of hydrogen atmosphere for 24 hours. After the reaction is completed, the hydrogen is vented, the catalyst is removed by filtration and the catalyst mixture is washed thoroughly. The filtrate is evaporated to leave a residue. The residual acetic acid is removed by co-distillation with ethanol and with CH₃OH to leave 1.68 grams of a yellow colored foam, 7-(3-L-histidine-amlno-1-pyrrolidinyl)-1-(2,4-difluorophenyl)-6-fluoro-1,4-dihydro-4-oxo-1,8-naphthyridine-3-carboxylic acid hydrochloride (I) (R₁ = 2,4-difluorophenyl, X=N, R₂ = R₃ = R₄ = H, A = histidine). [Alpha]_{D} = + 13.6 (CH₃OH)
   NMR (DMSO) delta: 1.30 (s) 9H, 1.87 (s) 3H, 1.95 (m) 1H, 2.72 (m) 1H, 2.85 (m) 1H, 4.23 (m) 1H, 4.40 (d) 1H, 5.38 (m) 1H, 6.70 (m) 1H, 6.80 (m) 1H, 7.00 (m) 1H, 7.30 (m) 2H, 7.45 (m) 1H, 7.55 (m) 1H, 7.75 (m) 1H, 8.00 (m) 1H, 8.12 (m) 1H, 8.32 (s) 1H, 8.70 (m) 1H.

### Example 45

Using the process as described in Example 44, replacing N-2-t BOC-N-pi-benzyloxymethyl-L-histidine-N-hydroxyphthalimide ester with N-t-BOC-L-Aspartic acid-b-benzyl-N-hydrosuccinimide ester, one obtains 7-(3-L-Asp-amino-1-pyrrolidinyl)-1-(2,4-difluorophenyl)-6-fluoro-1,4-dihydro-4-oxo-1,8-naphthyridine-3-carboxylic acid hydrochloride (I) (R₁ = 2,4-difluorophenyl, X=N, R₂ = R₃ = R₄ = H, A = Asp). mp. = 186 - 188°C.
MS (m+1)⁺ = 520 m/z, IR (KBr) CO, cm⁻¹: 1630, 1690, 1730
NMR (DMSO) delta: 1.85 (m) 2H, 2.03 (m) 2H, 2.80 (m) 2H, 3.96 (m) 1H, 4.32 (m) 1H, 7.34 (m) 1H, 7.59 (m) 1H, 7.80 (m) 1H, 8.09 (d) 1H, 8.31 (m) 2H, 8.82 (s) 1H.

### Example 46

Using the process as described in Example 2, replacing the N-carbobenzyloxy-L-Norvaline-N-hydroxyphthalimide ester with N,N-dicarbobenzyloxy-L-lysine-N-hydroxysuccinimide ester, one obtains 7-(3-Lys-amino-1-pyrrolidinyl)-1-(2,4-difluorophenyl)-6-fluoro-1,4-dihydro-4-oxo-1,8-naphthyridine-3-carboxylic acid dihydrochloride (I) (R₁ = 2,4-difluorophenyl, X = N, R₂ =R₃ = R₄ = H, A = Lys). mp. = 198 - 200°C.
MS (m+1)⁺ = 533 m/z
[Alpha]_{D} = +3.9 (CH₃OH)
IR (KBr) CO, cm⁻¹: 1630, 1680, 1718
NMR (DMSO) delta: 1.35 (m) 2H, 1.56 (m) 2H, 1.72 (m) 2H, 2.08 (m) 1H, 2.73 (m) 2H, 3.70 (m) 1H, 4.32 (m) 1H, 7.35 (m) 1H, 7.60 (m) 1H, 7.82 (m) 1H, 8.08 (d) 1H, 8.81 (s) 1H, 9.03 (m) 1H.

### Example 47

Using the process described in Example 40, replacing 7-(3-amino-1-pyrrolidinyl)-6-fluoro-1-(2,4-difluorophenyl) -1,4-dihydro-4-oxo-1,8-naphthyridine-3-carboxylic acid with 7-(3-amino-1-pyrrolidinyl)-6-fluoro-1-(2,4-difluorophenyl) -1,4-dihydro-4-oxoquinoline-3-carboxylic acid, one obtains the 7-(3-N-t-Boc-L-Asparagine-amino-1-pyrrolidinyl)-6-fluoro-1 -(2,4-difluorophenyl)-1,4-dihydro-4-oxoquinoline-3-carboxy lic acid (I) (R, =2,4-difluorophenyl, X = CH, R₂ = R₃ = R₄ = H, A = t-Boc-Asn).

### Example 48

Using the process described in Example 41, the product of Example 47 yields 7-(3-L-Asparagine-amino-1-pyrrolidinyl-1-(2,4-difluorophenyl)-6-fluoro-1,4-dihydro-4 -oxoquinoline-3-carboxylic acid hydrochloride (I). (R₁ = 2,4-difluorophenyl, X = CH, R₂ = R₃ = R₄ = H, A = Asn).

### Example 49

Using the process as described in Example 24a, replacing 1-(2,4-difluorophenyl)-6-fluoro-7-(3-amino-1-pyrrolidinyl)-1,4-dihydro-4-oxo-quinoline-3-carboxylic acid with an appropriate quinolone and naphthyridine derivative such as 1-(2,4-difluorophenyl)-6-fluoro-7-(2-methyl-4-amino-1-pyrrolidinyl)-1,4-dihydro-4-oxo-quinoline-3-carboxylic acid, 1-(2,4-difluorophenyl)-6-fluoro-7-(2-methyl-4-amino-1-pyrrolidinyl)-1,4-dihydro-4-oxo-1,8-naphthyridine-3-carboxylic acid, 1-(4-fluoro phenyl)-6-fluoro-7-(2-methyl-4-amino-1-pyrrolidinyl)-1,4-dihydro-4-oxo-quinoline-3-carboxylic acid, 1-(4-fluorophenyl)-6-fluoro-7-(2-methyl-4-amino-1-pyrrolidinyl)-1,4-dihydro-4-oxo-1,8-naphthyridine-3-carboxylic acid, 1-cyclopropyl-6-fluoro-7-(2-methyl-4-amino-1-pyrrolidinyl)-1,4-dihydro-4-oxoquinoline-3-carboxylic acid, 1-cyclopropyl-6-fluoro-7-(2-methyl-4-amino-1-pyrrolidinyl)-1,4-dihydro-4-oxo-naphthyridine-3-carboxylic acid, 1-ethyl-6-fluoro-7-(2-methyl-4-amino-1-pyrrolidinyl)-1,4-dihydro-4-oxo-quinoline-3-carboxylic acid, 1-ethyl-6-fluoro-7-(2-methyl-4-amino-1-pyrrolidinyl)-1,4-dihydro-4-oxo-1,8-naphthyridine-3-carboxylic acid, 1-cyclopropyl-7-(2-methyl-4-amino-1-pyrrolidinyl)-6,8-difluoro-1,4-dihydro-4-oxoquinoline-3-carboxylic acid, 1-cyclopropyl-7-(2-methyl-4-amino-1-pyrrolidinyl)-6-fluoro-8-chloro-1,4-dihydro-4-oxo quinoline 3-carboxylic acid and substituting various amino acids or peptides for one solubilizing group A, one obtains the following compounds as shown in the Table III.

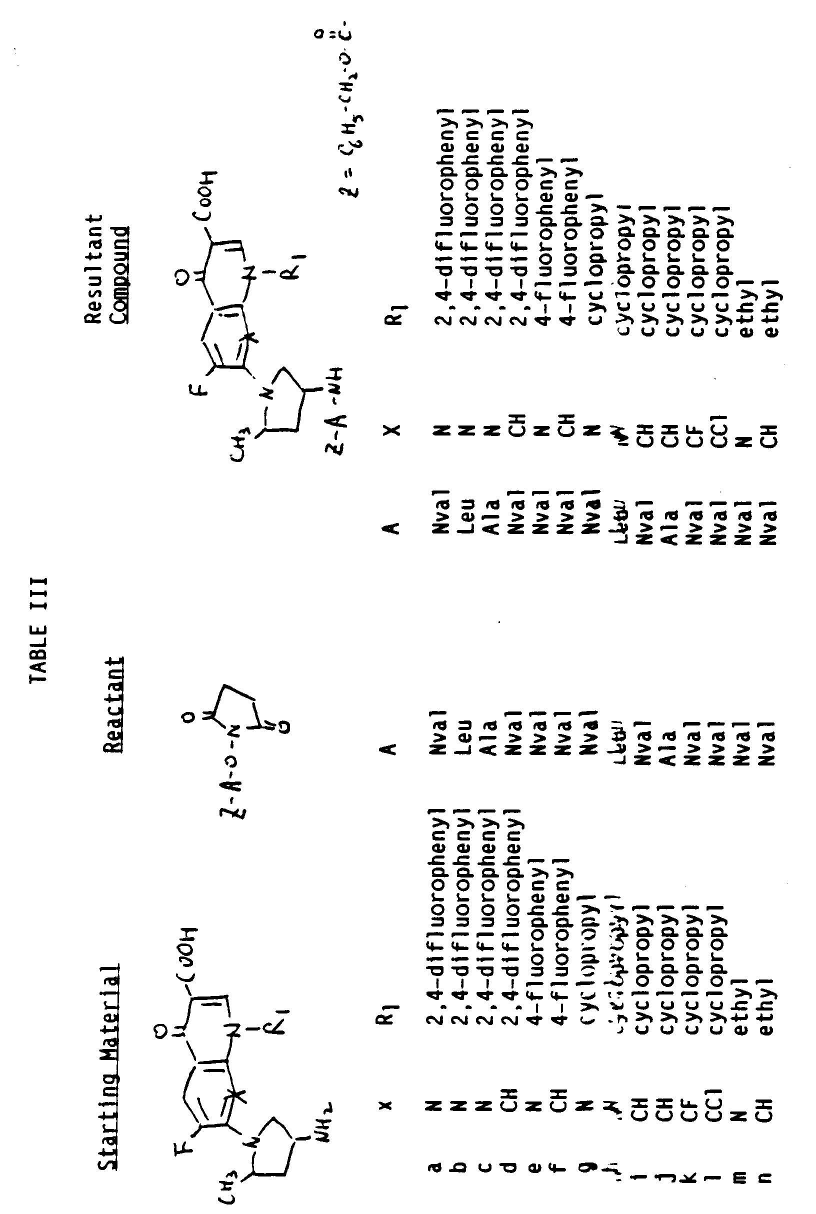

### Example 50

Using the process as described in Example 24(b), the compounds of Example 49 (a-n) yield the following corresponding quinolone and naphthyridine derivatives (a-n) and their hydrochloride salts as shown in Table IV.

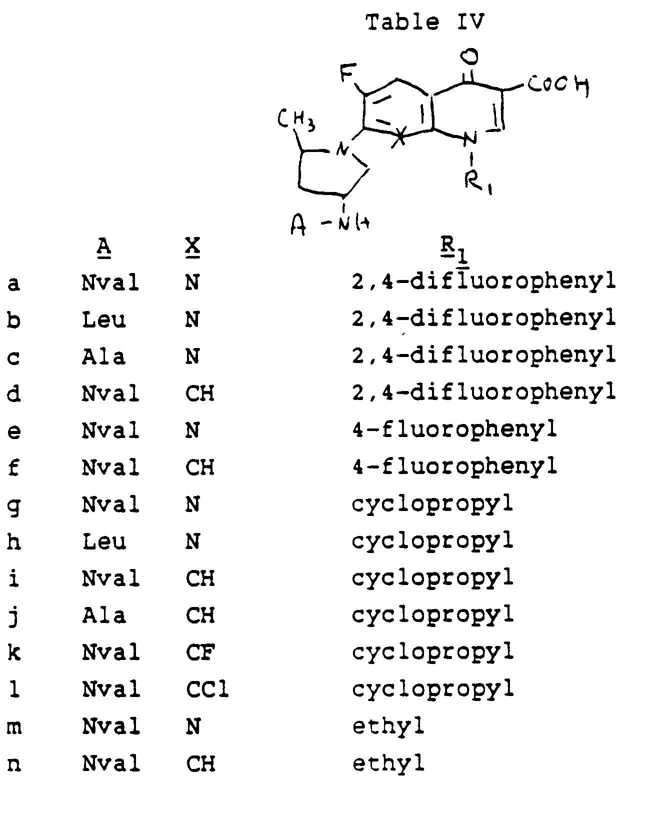

### Example 51

Using the process as described in either Example 1a or 3a and substituting Gly-Nval for the solubilizing group A, yields the protected Gly-Nval compound (I) (R₁ = 2,4-difluorophenyl, X = N, R₂ = R₄ = R₃ = H, A = carbobenzyloxy Gly-Nval). MS (m+1)⁺ = 695 m/z [alpha]_{D} = -11.2 (CDCl₃), mp = 115-117°C.

NMR data: (CDCl₃) delta: 0.90 (m) 3H, 1.30 (m) 3H, 1.65 (m) 3H, 1.85 (m) 1H, 2.03 (m) 2H, 3.30 (m) 2H, 3.85 (m) 4H, 4.57 (m) 4H, 5.05 (s) 2H, 5.52 (m) 1H, 6.62 (m) 1H, 7.10 (m) 4H, 7.32 (m) 6H, 7.85 (d) 1H, 8.43 (d) 1H.

IR data: (CDCl₃) CO, cm⁻¹: 1630, 1670, 1722.

### Example 52

Using the process as described in Example 3b with the compound of Example 51 yields the deblocked Gly-Nval (HCl) salt, (I) (R₁ = 2,4-difluorophenyl, X = N, R₂ = R₃ = R₄ = H, A = Gly-Nval). mp = 180-182°C, MS (m+1)⁺ = 561 m/z, [alpha]_{D} = 5.3° (CH₃OH).

NMR Data: (DMSO) delta: 0.83 (m) 3H, 1.22 (m) 3H, 1.50 (m) 3H, 1.80 (m) 1H, 2.02 (m) 1H, 3.55 (s) 2H, 4.27 (m) 2H, 7.32 (m) 1H, 7.60 (m) 1H, 7.81 (m) 1H, 8.02 (m) 2H, 8.10 (d) 1H, 8.41 (m) 1H, 8.56 (d) 1H, 8.82 (s) 1H.

IR data: (KBr) CO, cm⁻¹: 1630, 1660, 1720.

### Example 53

Using the process as described in either Example 1a or 3a and substituting Gly-Gly for the solubilizing group A, yields the protected Gly-Gly compound (I) (R₁ = 2,4-difluorophenyl, X = N, R₂ = R₄ = R₃ = H, A = carbobenzyloxy Gly-Gly). MS (M+1)⁺ = 653 m/z, mp = 125-128°C.

IR data: (CDCl₃) CO, cm⁻¹: 1630, 1672, 1722.

### Example 54

Using the process as described in Example 3b with the compound of Example 51 yields the deblocked Gly-Gly (HCl) salt, (I) (R₁ = 2,4-difluorophenyl, X = N, R₂ = R₃ = R₄ = H, A = Gly-Gly). (m+1)⁺ 519 m/z, mp = 175-178°C.

NMR data: (DMSO) delta: 1.82 (m) 1H, 2.04 (m) 1H, 3.34 (m) 2H, 3.60 (m) 2H, 3.77 (m) 2H, 4.30 (m) 1H, 7.35 (m) 1H, 7.60 (m) 1H, 7.80 (m) 1H, 8.05 (m) 3H, 8.31 (m) 1H, 8.60 (m) 1H, 8.83 (s) 1H, 15.17 (s) 1H.

IR data: (KBr) CO, cm⁻¹: 1635, 1670, 1720.

### Example 55

Using the process as described in either Example 1a or 3a and substituting Gly-Ala, Ala-Nval, Ala-Met, Ala-Leu, Leu-Nval, Leu-Leu, Leu-Met, Met-Met, Met-Leu, Met-Ala, Met-Nval, Nval-Gly, Nval-Ala, Gly-Gly-Gly, D-Ala-L-Ala, Gly-Nval-Nval, Gly-Gly-Ala, Gly-Ala-Ala, Phe-Ala, Leu-Ala, Val-Ala, Val-Leu, Gly-Gly-Gly-Gly, Gly-Gly-Gly-Ala and Gly-Gly-Gly-Nval for the solubilizing group A and subjecting the products from these reactions with the same process as described in Example 3b, or following a similar procedure as Examples 31a and 31c, substituting the above group, one can obtain the hydrochloride salt of the following compounds.
a. 7-(3-Gly-Ala-amino-1-pyrrolidinyl)-6-fluoro-1-(2,4-difluorophenyl)-1,4-dihydro-4-oxo-1,8-naphthyridine-3-carboxylic acid;
b. 7-(3-Ala-Nval-amino-1-pyrrolidinyl)-6-fluoro-1-(2,4-difluorophenyl)-1,4-dihydro-4-oxo-1,8-naphthyridine-3-carboxylic acid;
c. 7-(3-Leu-Nval-amino-1-pyrrolidinyl)-6-fluoro-1-(2,4-difluorophenyl)-1,4-dihydro-4-oxo-1,8-naphthyridine-3-carboxylic acid;
d. 7-(3-Met-Nval-amino-1-pyrrolidinyl)-6-fluoro-1-(2,4-difluorophenyl)-1,4-dihydro-4-oxo-1,8-naphthyridine-3-carboxylic acid;
e. 7-(3-Nval-Gly-amino-1-pyrrolidinyl)-6-fluoro-1-(2,4-difluorophenyl)-1,4-dihydro-4-oxo-1,8-naphthyridine-3-carboxylic acid;
f. 7-(3-Nval-Ala-amino-1-pyrrolidinyl)-6-fluoro-1-(2,4-difluorophenyl)-1,4-dihydro-4-oxo-1,8-naphthyridine-3-carboxylic acid;
g. 7-(3-Gly-Gly-Gly-amino-1-pyrrolidinyl)-6-fluoro-1-(2,4-difluorophenyl)-1,4-dihydro-4-oxo-1,8-naphthyridine-3-carboxylic acid;
h. 7-(3-Gly-Nval-Nval-amino-1-pyrrolidinyl)-6-fluoro-1-(2,4-difluorophenyl)-1,4-dihydro-4-oxo-1,8-naphthyridine-3-carboxylic acid;
i. 7-(3-Gly-Gly-Ala-amino-1-pyrrolidinyl)-6-fluoro-1-(2,4-difluorophenyl)-1,4-dihydro-4-oxo-1,8-naphthyridine-3-carboxylic acid;
j. 7-(3-Gly-Ala-Ala-amino-1-pyrrolidinyl)-6-fluoro-1-(2,4-difluorophenyl)-1,4-dihydro-4-oxo-1,8-naphthyridine-3-carboxylic acid;
k. 7-(3-Gly-Gly-Gly-Gly-amino-1-pyrrolidinyl)-6-fluoro-1-(2,4-difluorophenyl)-1,4-dihydro-4-oxo-1,8-naphthyridine-3-carboxylic acid;
l. 7-(3-Gly-Gly-Gly-Ala-amino-1-pyrrolidinyl)-6-fluoro-1-(2,4-difluorophenyl)-1,4-dihydro-4-oxo-1,8-naphthyridine-3-carboxylic acid;
m. 7-(3-Gly-Gly-Gly-Nval-amino-1-pyrrolidinyl)-6-fluoro-1-(2,4-difluorophenyl)-1,4-dihydro-4-oxo-1,8-naphthyridine-3-carboxylic acid;
n. 7-(3-Ala-Met-amino-1-pyrrolidinyl)-6-fluoro-1-(2,4-difluorophenyl)-1,4-dihydro-4-oxo-1,8-naphthyridine-3-carboxylic acid;
o. 7-(3-Met-Met-amino-1-pyrrolidinyl)-6-fluoro-1-(2,4-difluorophenyl)-1,4-dihydro-4-oxo-1,8-naphthyridine-3-carboxylic acid;
p. 7-(3-Leu-Met-amino-1-pyrrolidinyl)-6-fluoro-1-(2,4-difluorophenyl)-1,4-dihydro-4-oxo-1,8-naphthyridine-3-carboxylic acid;
q. 7-(3-Leu-Leu-amino-1-pyrrolidinyl)-6-fluoro-1-(2,4-difluorophenyl)-1,4-dihydro-4-oxo-1,8-naphthyridine-3-carboxylic acid;
r. 7-(3-Ala-Leu-amino-1-pyrrolidinyl)-6-fluoro-1-(2,4-difluorophenyl)-1,4-dihydro-4-oxo-1,8-naphthyridine-3-carboxylic acid;
s. 7-(3-Met-Leu-amino-1-pyrrolidinyl)-6-fluoro-1-(2,4-difluorophenyl)-1,4-dihydro-4-oxo-1,8-naphthyridine-3-carboxylic acid;
t. 7-(3-Phe-Ala-amino-1-pyrrolidinyl)-6-fluoro-1-(2,4-difluorophenyl)-1,4-dihydro-4-oxo-1,8-naphthyridine-3-carboxylic acid;
u. 7-(3-Leu-Ala-amino-1-pyrrolidinyl)-6-fluoro-1-(2,4-difluorophenyl)-1,4-dihydro-4-oxo-1,8-naphthyridine-3-carboxylic acid;
v. 7-(3-Met-Ala-amino-1-pyrrolidinyl)-6-fluoro-1-(2,4-difluorophenyl)-1,4-dihydro-4-oxo-1,8-naphthyridine-3-carboxylic acid;
w. 7-(3-Val-Ala-amino-1-pyrrolidinyl)-6-fluoro-1-(2,4-difluorophenyl)-1,4-dihydro-4-oxo-1,8-naphthyridine-3-carboxylic acid;
x. 7-(3-Val-Leu-amino-1-pyrrolidinyl)-6-fluoro-1-(2,4-difluorophenyl)-1,4-dihydro-4-oxo-1,8-naphthyridine-3-carboxylic acid; and
y. 7-(3-D-Ala-L-Ala-amino-1-pyrrolidinyl)-6-fluoro-1-(2,4-difluorophenyl)-1,4-dihydro-4-oxo-1,8-naphthyridine-3-carboxylic acid.

### Table V

The physical data for the hydrochloride salt of the products in the designated Examples is as follows:
Example 55b
   mp = 200°-202°C
   MS (m+1)⁺ = 575 m/z
   [alpha]_{D} = +10.2 (CH₃OH)
   IR (KBr) CO, cm⁻¹: 1630, 1670, 1715
   NMR (DMSO-d₆) delta: 0.80 (m) 3H, 1.25 (m) 5H, 1.47 (m) 2H, 1.78 (m) 1H, 2.00 (m) 1H, 3.80 (m) 2H, 4.18 (m) 2H, 7.30 (m) 1H, 7.53 (m) 1H, 7.75 (m) 1H, 8.05 (d) 1H, 8.34 (d) 1H, 8.47 (d) 1H, 8.77 (s) 1H.
Example 55c
   mp = 180°-182°C
   MS (m+1)⁺ = 617 m/z
   [alpha]_{D} = +19.0 (CH₃OH)
   IR (KBr) CO, cm⁻¹: 1630, 1670, 1710
   NMR (CD₃OD) delta: 0.95 (m) 9H, 1.34 (m) 3H, 1.67 (m) 5H, 1.96 (m) 1H, 2.15 (m) 1H, 3.87 (m) 1H, 4.34 (m) 2H, 7.26 (m) 2H, 7.65 (m) 1H, 8.05 (m) 1H, 8.78 (s) 1H.
Example 55d
   mp = 170°-172°C
   MS (m+1)⁺ = 635 m/z
   [alpha]_{D} = +24.2 (CH₃OH)
   IR (KBr) CO, cm⁻¹: 1630, 1670, 1720
   NMR (DMSO-d₆) delta: 0.85 (m) 3H, 1.28 (m) 3H, 1.53 (m) 3H, 1.95 (m) 2H, 2.05 (m) 3H, 3.85 (m) 1H, 4.25 (m) 1H, 7.33 (m) 1H, 7.55 (m) 1H, 7.80 (m) 1H, 8.10 (m) 1H, 8.15 (m) 2H, 8.38 (m) 1H, 8.60 (m) 1H, 8.82 (s) 1H.
Example 55f
   mp = 195°-197°C
   MS (m+1)⁺ = 575 m/z
   [alpha]_{D} = +25.6 (CH₃OH)
   IR (KBr) CO, cm⁻¹: 1630, 1670, 1720
   NMR (CD₃OD) delta: 0.95 (m) 3H, 1.34 (m) 3H, 1.45 (m) 2H, 1.83 (m) 2H, 1.95 (m) 1H, 2.15 (m) 1H, 3.83 (m) 2H, 4.33 (m) 2H, 7.25 (m) 3H, 7.65 (m) 1H, 8.03 (m) 1H, 8.77 (m) 1H.
Example 55n
   mp = 185°-187°C
   MS (m+1)⁺ = 607 m/z
   [alpha]_{D} = +10.3 (CH₃OH)
   IR (KBr) CO, cm⁻¹: 1630, 1670, 1725
   NMR (CD₃OD) delta: 1.48 (m) 3H, 2.02 (m) 6H, 2.15 (m) 1H, 2.50 (m) 2H, 3.95 (m) 1H, 4.40 (m) 2H, 7.28 (m) 2H, 7.68 (m) 1H, 8.02 (m) 1H, 8.75 (s) 1H.
Example 55o
   mp = 163°-165°C
   MS (m+1)⁺ = 667 m/z
   [alpha]_{D} = +24.6 (CH₃OH)
   IR (KBr) CO, cm⁻¹: 1630, 1670, 1720
   NMR (CD₃OD) delta: 2.10 (m) 11H, 2.55 (m) 4H, 3.95 (m) 1H, 4.40 (m) 2H, 7.28 (m) 2H, 7.65 (m) 1H, 8.05 (m) 1H, 8.77 (s) 1H.
Example 55p
   mp = 180°-183°C
   MS (m+1)⁺ = 649 m/z
   [alpha]_{D} = +18.9 (CH₃OH)
   IR (KBr) CO, cm⁻¹: 1630, 1665, 1720
   NMR (CD₃OD) delta: 0.97 (m) 6H, 1.70 (m) 3H, 1.95 (m) 1H, 2.05 (m) 3H, 2.50 (m) 2H, 3.87 (m) 1H, 4.35 (m) 1H 4.45 (m) 1H, 7.25 (m) 2H, 7.65 (m) 1H, 8.05 (m) 1H, 8.77 (s) 1H.
Example 55q
   mp = 194°-195°C
   MS (m+1)⁺ = 631 m/z
   [alpha]_{D} = +18.5 (CH₃OH)
   IR (KBr) CO, cm⁻¹: 1630, 1675, 1725
   NMR (CD₃OD) delta: 0.95 (m) 12H, 1.63 (m) 7H, 1.94 (m) 1H, 2.15 (m) 1H, 3.85 (m) 1H, 4.37 (m) 2H, 7.25 (m) 2H, 7.65 (m) 1H, 8.05 (m) 1H, 8.77 (s) 1H.
Example 55r
   mp = 200°-202°C
   MS (m+1)⁺ = 589 m/z
   [alpha]_{D} = +7.8 (CH₃OH)
   IR (KBr) CO, cm⁻¹: 1630, 1670, 1720
   NMR (CD₃OD) delta: 0.90 (m) 6H, 1.45 (m) 3H, 1.58 (m) 3H, 1.95 (m) 1H, 2.13 (m) 1H, 3.29 (m) 2H, 3.90 (m) 1H, 4.34 (m) 2H, 7.25 (m) 2H, 7.65 (m) 1H, 8.03 (m) 1H, 8.75 (s) 1H.
Example 55s
   mp = 178°-180°C
   MS (m+1)⁺ = 649 m/z
   [alpha]_{D} = +24.5 (CH₃OH)
   IR (KBr) CO, cm⁻¹: 1630, 1670, 1715
   NMR (CD₃OD) delta: 0.90 (m) 6H, 1.55 (m) 3H, 1.92 (m) 1H, 2.10 (m) 4H, 2.55 (m) 2H, 3.29 (m) 2H, 3.95 (m) 1H, 4.35 (m) 2H, 7.25 (m) 2H, 7.65 (m) 1H, 8.03 (m) 1H, 8.75 (s) 1H.
Example 55t
   mp = 192°-194°C
   MS (m+1)⁺ = 623 m/z
   [alpha]_{D} = +20.8 (CH₃OH)
   IR (KBr) CO, cm⁻¹: 1630, 1670, 1720
   NMR (CD₃OD) delta: 1.34 (m) 3H, 1.95 (m) 1H, 2.16 (m) 1H, 2.95 (m) 1H, 3.20 (m) 1H, 4.08 (m) 1H, 4.35 (m) 2H, 7.30 (m) 7H, 7.63 (m) 1H, 8.04 (m) 1H, 8.75 (m) 1H.
Example 55u
   mp = 197°-200°C
   MS (m+1)⁺ = 589 m/z
   [alpha]_{D} = +25.0 (CH₃OH)
   IR (KBr) CO, cm⁻¹: 1630, 1660, 1720
   NMR (CD₃OD) delta: 0.98 (m) 6H, 1.34 (m) 3H, 1.70 (m) 3H, 1.95 (m) 1H, 2.15 (m) 1H, 3.85 (m) 1H, 4.35 (m) 1H, 7.25 (m) 2H, 7.65 (m) 1H, 8.04 (m) 1H, 8.78 (s) 1H.
Example 55v
   mp = 178°-180°C
   MS (m+1)⁺ = 607 m/z
   [alpha]_{D} = +31.8 (CH₃OH)
   IR (KBr) CO, cm⁻¹: 1630, 1670, 1720
   NMR (CD₃OD) delta: 1.35 (m) 3H, 1.95 (m) 1H, 2.12 (m) 3H, 2.60 (m) 2H, 3.95 (m) 1H, 4.34 (m) 2H, 7.27 (m) 2H, 7.66 (m) 1H, 8.05 (m) 1H, 8.78 (s) 1H.
Example 55w
   mp = 201°-204°C
   MS (m+1)⁺ = 575 m/z
   [alpha]_{D} = +20.6 (CH₃OH)
   IR (KBr) CO, cm⁻¹: 1630, 1665, 1720
   NMR (CD₃OD) delta: 1.03 (m) 6H, 1.35 (m) 3H, 1.95 (m) 1H, 2.18 (m) 1H, 3.65 (m) 1H, 4.34 (m) 1H, 7.25 (m) 2H, 7.65 (m) 1H, 8.05 (m) 1H, 8.76 (s) 1H.
Example 55x
   mp = 192°-195°C
   MS (m+1)⁺ = 617 m/z
   [alpha]_{D} = +17.4 (CH₃OH)
   IR (KBr) CO, cm⁻¹: 1630, 1670, 1720
   NMR (CD₃OD) delta: 0.95 (m) 12H, 1.55 (m) 2H, 1.95 (m) 1H, 2.18 (m) 1H, 3.65 (m) 1H, 4.35 (m) 1H, 7.25 (m) 2H, 7.65 (m) 1H, 8.05 (m) 1H, 8.78 (s) 1H.
Example 55y
   mp = 188-190°C
   MS (M+1)⁺ = 547 m/z
   [alpha]_{D} = +5.1 (CH₃OH)
   IR (KBr) CO, cm⁻¹ = 1630, 1670, 1710
   NMR (CD₃OD) delta: 1.33 (m) 3H, 1.48 (m) 3H, 1.95 (m) 1H, 2.15 (m) 1H, 3.95 (m) 1H, 4.28 (m) 1H, 4.36 (m) 1H, 7.27 (m) 2H, 7.65 (m) 1H, 8.05 (m) 1H, 8.32 (m) 1H, 8.78 (s) 1H.

### Solubility

Some of the dipeptide derivatives of this invention show unexpected improvement in aqueous solubility especially when the N-1 position is substituted by a substituted phenyl group as shown in Table VI below. The amino acid, dipeptide and tripeptide derivatives produced in Examples 1b, 2b, 3b, 5, 11, 13, 15, 17, 19, 21, 30, 31c, 41, 44c, and 45, having a 2,4-difluorophenyl substitution at the N-1 position, are compared to a parent compound which is tosufloxacin (i.e. 7-(3-amino-1-pyrrolidinyl)-6-fluoro,1- (2,4-difluorophenyl)-1,4-dihydro-4-oxo-1,8-naphthyridine-3-carboxylic acid); Example 26i is compared to its parent compound A which is 7-(3-amino-1-pyrrolidinyl)-6-fluoro-1-(4-fluorophenyl)-1,4-dihydro-4-oxo-1,8-naphthyridine-3-carboxylic acid. Further, dipeptide or tripeptide derivatives are generally more soluble than an amino acid derivative (when the polypeptide is compared to any derivative having one amino acid residue included in the polypeptide) at the physiological pH (i.e., 7.4) which may help improve absorption. See, for example, 1b, 3b, 11, 15, 30 compared to 2b, 5, 17 and 52.

**Table VI**

| Example | A | Salt | Water Solubility mg/ml* | pH 7.4 mg/ml |
|---|---|---|---|---|
| tosufloxacin | H | Tosylate | 0.65 | 0.01 |
| 1b | Gly-Phe | HCl | > 98 | 1.037 |
| 2b | Nval | HCl | > 20 | 0.23 |
| 3b | Nval-Nval | HCl | > 40 | 0.68 |
| 5 | Phe | HCl | > 101 | 0.34 |
| 11 | Ala-Phe | HCl | > 20 | 0.59 |
| 13 | Tyr | HCl | > 90 | 0.06 |
| 15 | Ala-Ala | HCl | > 70 | 1.07 |
| 17 | Ala | HCl | > 20 | 0.12 |
| 19 | Val | HCl | > 10 | 0.41 |
| 21 | Leu | HCl | > 35 | 0.14 |
| A | H | HCl | 1.4 | 0.014 |
| 26i | Nval | HCl | > 9 | 0.24 |
| 30 | Gly-Gly-Nval | HCl | > 19 | 0.62 |
| 31c | Met-sulfone | HCl | > 35 | 0.03 |
| 41 | Asn | HCl | > 35 | 0.40 |
| 44c | His | HCl | >100 | 0.30 |
| 45 | Asp | HCl | 4.1 | 2.40 |
| 52 | Gly-Nval | HCl | 6.89 | 0.08 |
| 55b | Ala-Nval | HCl | 26 | 1.03 |
| 54 | Gly-Gly | HCl | 20 | 0.10 |
| 55n | Ala-Met | HCl | 20 | 2.50 |
| 55o | Met-Met | HCl | 22 | 0.31 |
| 55p | Leu-Met | HCl | 23 | 0.06 |
| 55q | Leu-Leu | HCl | 19 | 0.19 |
| 55r | Ala-Leu | HCl | 26 | 2.0 |

| | | | | |
|---|---|---|---|---|
| * the sign > refers to an approximate value and the solubility may be within 10% higher | | | | |

### In Vitro Antibacterial Activity

### In Vitro Tests

The in vitro antibacterial activity of the test compounds was determined by conventional agar dilution procedures. The organisms were grown overnight in brain-heart infusion (BHI) broth (Difco 0037-01-6) at 36° C. Twofold dilutions of the stock solution (2000 mcg/mL) of the test compound were made in BHI agar to obtain a test concentration ranging from 200 to 0.005 mcg/mL. The plate was inoculated with approximately 10⁴ organisms. It was then incubated at 36° C for 18 h. The minimal inhibitory concentration (MIC) was the lowest concentration of the test compound that yielded no visible growth on the plate.

The results of in vitro testing of the compounds are shown in Table VII below where the growth medium for this study was Brain Heart Infusion Agar (BHIA), the parent compound is tosufloxacin. The MIC results are in ug/ml.

**TABLE VII**

| Organism | | MIC (ug/ml) | | | | |
|---|---|---|---|---|---|---|
| | | 15 | 1b | 2b | 5 | Tosufloxacin |
| STAPHYLOCOCCUS AUREUS | ATCC 6538P | 1.56 | .1 | .05 | .2 | 0.02 |
| STAPHYLOCOCCUS AUREUS | CMX 686B | 6.2 | .1 | -- | -- | 0.05 |
| STAPHYLOCOCCUS AUREUS | A5177 | 12.5 | .2 | .2 | .78 | 0.05 |
| STAPHYLOCOCCUS AUREUS | 45 | 12.5 | .1 | .2 | 1.56 | 0.1 |
| STAPHYLOCOCCUS AUREUS | 45 RAR2 | 25 | .1 | .39 | .78 | 0.1 |
| STAPHYLOCOCCUS AUREUS | 642A | -- | -- | .2 | .39 | -- |
| STAPHYLOCOCCUS AUREUS | NCTC 10649 | -- | -- | .1 | .39 | -- |
| STAPHYLOCOCCUS AUREUS | CMX 503A | 12.5 | .1 | -- | -- | 0.05 |
| STAPHYLOCOCCUS AUREUS | CMX 553 | 12.5 | .2 | .39 | .78 | 0.1 |
| STAPHYLOCOCCUS EPIDERMIDIS | 3519 | 12.5 | .78 | .39 | .78 | 0.1 |
| MICROCOCCUS LUTEUS | ATCC 9341 | >100 | 6.25 | 25 | 25 | 1.56 |
| MICROCOCCUS LUTEUS | ATCC 4698 | 100 | .78 | 25 | 12.5 | 0.78 |
| ENTEROCOCCUS FAECIUM | ATCC 8043 | 50 | 1.56 | .78 | 3.1 | 0.2 |
| STREPTOCOCCUS BOVIS | A5169 | 50 | 1.56 | 1.56 | 6.2 | .39 |
| STREPTOCOCCUS AGALACTIAE | CMX 508 | 12.5 | 1.56 | 1.56 | 3.1 | .39 |
| STREPTOCOCCUS PYOGENES | EES61 | 12.5 | 1.56 | 1.56 | 3.1 | .2 |
| STREPTOCOCCUS PYOGENES | 930 CONST | 12.5 | .78 | .78 | 1.56 | .2 |
| STREPTOCOCCUS PYOGENES | 2548 INDUC | 12.5 | .78 | .39 | .78 | .1 |
| ESCHERICHIA COLI | JUHL | 12.5 | 3.12 | .78 | 3.1 | .02 |
| ESCHERICHIA COLI | SS | .39 | .2 | .02 | .1 | .005 |
| ESCHERICHIA COLI | DC-2 | 100 | 100 | 12.5 | 50 | .39 |
| ESCHERICHIA COLI | H560 | 6.2 | 1.56 | .78 | 3.1 | .02 |
| ESCHERICHIA COLI | KNK 437 | 50 | 25 | 6.2 | 100 | .2 |
| ENTEROBACTER AEROGENES | ATCC 13048 | 25 | 12.5 | 3.1 | 12.5 | .05 |
| KLEBSIELLA PNEUMONIAE | ATCC 8045 | 12.5 | 12.5 | .39 | 1.56 | .02 |
| PROVIDENCIA STUARTII | CMX 640 | 100 | 100 | 50 | >100 | 1.56 |
| PSEUDOMONAS AERUGINOSA | BMH10 | 100 | 25 | 6.2 | 25 | .1 |
| PSEUDOMONAS AERUGINOSA | A5007 | 100 | 25 | 12.5 | 50 | .78 |
| PSEUDOMONAS AERUGINOSA | K799/WT | 50 | 25 | 6.2 | 25 | .78 |
| PSEUDOMONAS AERUGINOSA | K799/61 | 3.1 | 12.5 | .39 | 1.56 | .2 |
| PSEUDOMONAS CEPACIA | 296I | 50 | 50 | 50 | >100 | 3.1 |
| ACINETOBACTER SP | CMX 669 | 12.5 | 12.5 | 3.1 | 12.5 | .1 |

In a second in vitro test conducted as described above and shown in Table VIII, the growth media is substituted with Brain Heart Infusion Broth (BHIB), and the MIC level is in ug/ml.

**TABLE VIII**

| Example | MIC (ug/ml) Organism | | | |
|---|---|---|---|---|
| | Stap. Aureus CMX 553 | Strep. pyogenes EES 61 | E.Coli Juhl | Ps.Aeruginosa A5007 |
| 3b | 12.5 | 12.5 | 25 | 50 |
| 7 | 1.56 | 0.78 | 0.78 | 12.5 |
| 11 | 16.0 | -- | 16 | 128 |
| 13 | 12.5 | 12.5 | 12.5 | 50 |
| 17 | 2.0 | -- | 0.5 | 16 |
| 21 | 1.56 | 6.2 | 1.56 | 25 |
| 23 | 3.12 | 3.12 | 3.12 | 5.0 |
| 26b | 0.39 | 1.56 | 0.78 | 6.2 |
| 26h | 0.78 | 1.56 | 0.78 | 6.2 |
| 26i | 1.56 | 1.56 | 0.39 | 6.2 |
| 26o | 0.39 | 3.1 | 0.2 | 1.56 |
| 26p | 0.39 | 3.1 | 0.39 | 1.56 |
| 26ff | 1.56 | 6.2 | 1.56 | 12.5 |
| 26jj | 16 | 32 | 16 | 64 |
| 28 | 1.56 | 1.56 | 1.56 | 12.5 |
| 30 | 25 | 100 | 25 | >100 |
| 31c | 25 | 25 | 12.5 | >100 |
| 44c | 16 | 32 | 64 | > 64 |
| 46 | 100 | 100 | 3.12 | >100 |

### In-vivo Antibacterial Activity

The acute mouse protection test is conducted on ten mice with each of three levels of drug. Mouse mortality is used to calculate an ED₅₀ value, i.e., the dose of drug required to protect 50% of the test animals against death due to the inoculum challenge.

The acute mouse protection test is conducted on female, Swiss albino mice, 18 - 20 grams in weight. The mice are injected intraperitoneally with an 18-hour culture of the indicated test organism diluted sufficiently to provide the desired LD₅₀ value. To check the potency of the inoculum, a titration of the indicated test organism is carried out in control animals. The treatment group of animals is dosed with the test compound at 1 and 5 hours post-infection and observed for 7 days. The ED₅₀ values are calculated using the mortality data collected. Results are indicated in Table IX.

The MIC level is in ug/ml, and the ED₅₀ is in mg/kg/day.

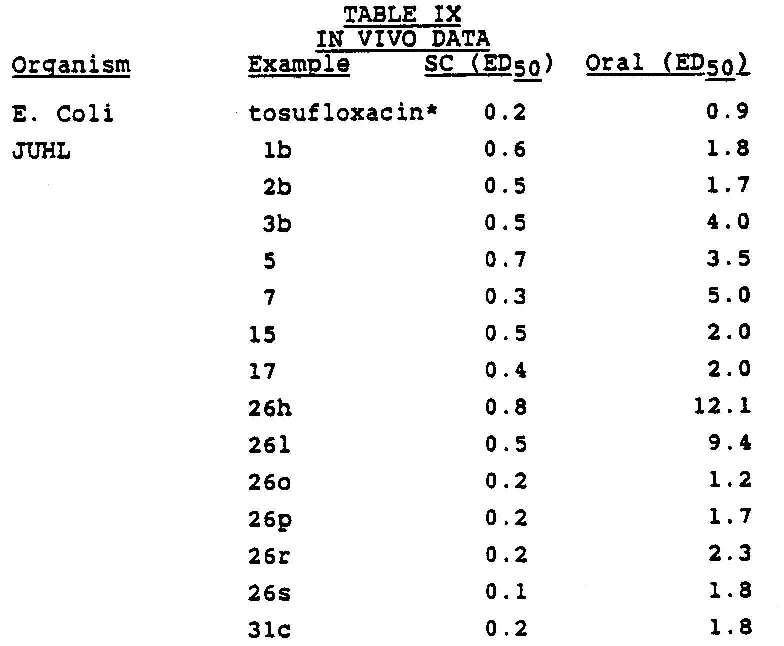

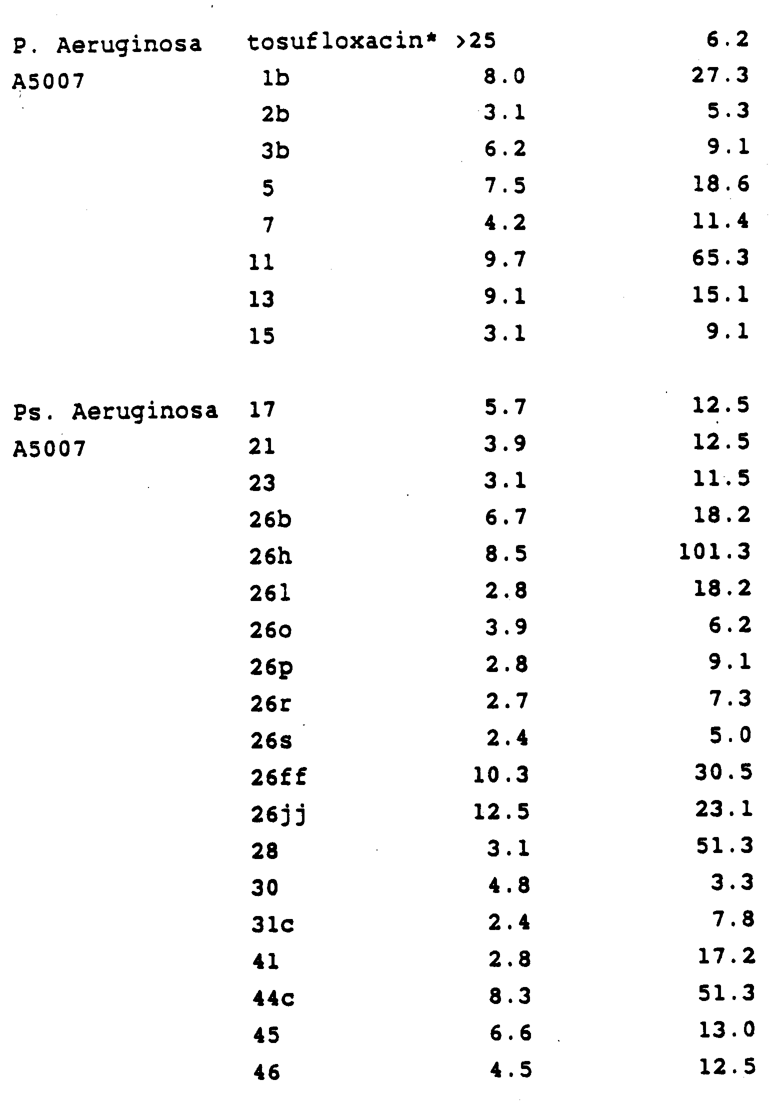

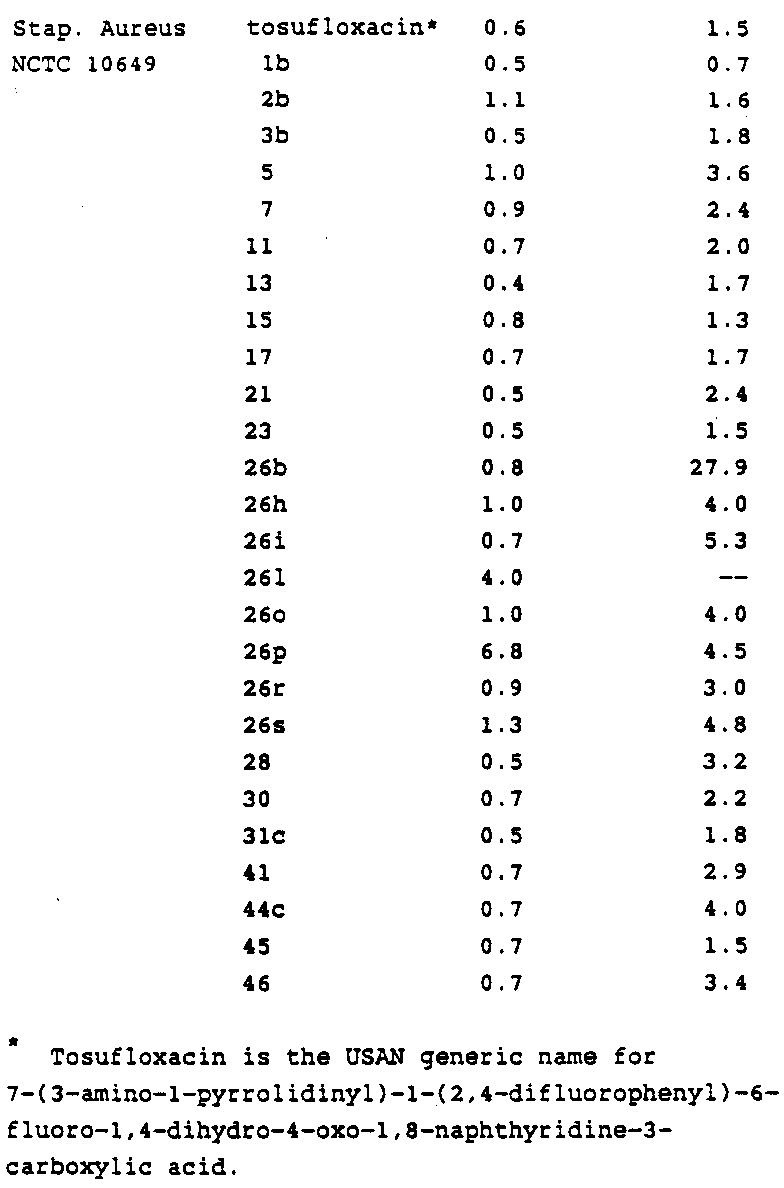

### Blood Levels

The compounds of this invention can also act as prodrugs; i.e., the compounds can hydrolyze rapidly in blood to yield the corresponding parent antibacterial compounds according to the following equation.

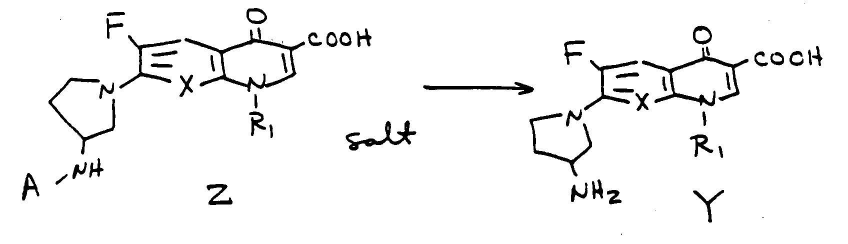

An aqueous solution of prodrug (Z) is spiked into fresh whole blood containing sodium heparin as an anticoagulant. The sample is mixed gently by inversion and placed in a 37° waterbath. At selected time intervals an 0.5 ml aliquot of blood is removed and placed in a chilled 1.5 ml centrifuge tube (ice temperature). Samples are centrifuges at 13,000 rpm for two minutes to separate plasma. An 0.2 ml aliquot of the plasma sample is combined with 0.2 ml internal standard (7-(4-amino-2-methyl-1-pyrrolidinyl)-1-(2,4-difluorophenyl)-6-fluoro-1,4-dihydro-4-oxo-1,8-naphthyridine-3-carboxylic acid in 0.2 M sodium phosphate pH 7.2 buffer) and 6 mls CH₂Cl₂:EtoH (9:1 by volume). Samples are capped and shaken gently for 10 minutes. Samples are centrifuges at 2500 rpm for 10 minutes (4°C). The aqueous layer is aspirated to waste and the organic layer is transferred to a conical centrifuge tube and evaporated to dryness with a stream of dry air and low heat (-35°C). Samples are reconstituted with 0.2 ml mobile phase followed by vortexing. Samples are centrifuges at 2500 rpm for 10 minutes to separate precipitate. The supernatant is transferred to a WISP vial with plastic insert for HPLC analysis.

The parent compound (y), and an internal standard compound and the two peaks from each prodrug are separated from plasma components on a 5 cm 2 4.6 mm 3 um Spherisorb ODS2 column using a mobile phase containing 39% acetonitrile:0.04 M H₃PO₄:0.01 M NaH₂PO₄:0.005 M acetohydroxamic acid:0.2% sodium dodecyl sulfate at a flow rate of 1.0 ml/min with quantitation of the 50-80 ul injection at 270 nm.

The conversion rate of various compound (Z) to parent drug (Y) in whole blood at 37°C. are given in Table X. Included in Table X are data for dipeptide derivatives (Z) of the invention as well as data for amino acid derivatives.

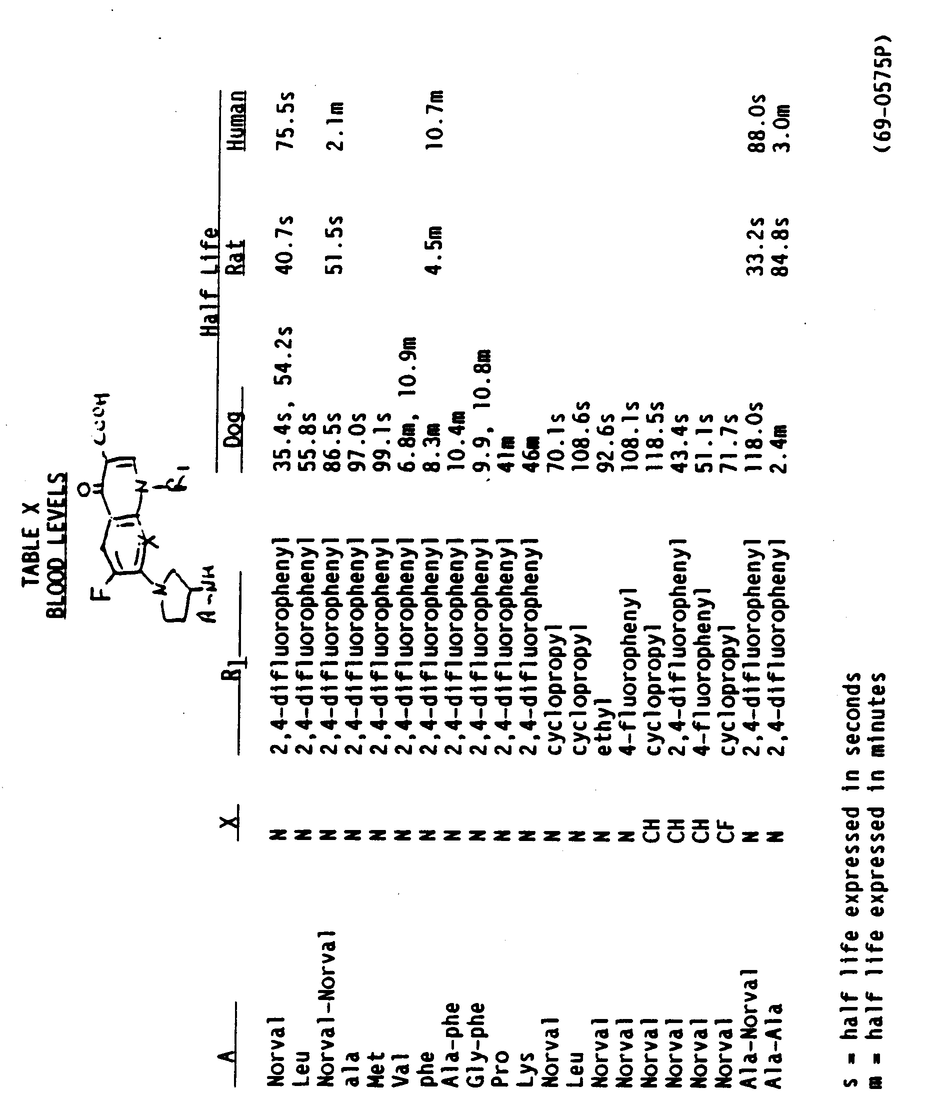

### Plasma Level

The compounds of this invention are found to convert back to the parent antibacterial compound in experimental animals and produce drug plasma level much higher than the parent compound can achieve. Thus they have better pharmacokinetic profiles and can be more effective in treating infections. The maximum plasma concentrations (Cmax) and the total plasma concentration over time (AUC) of tosufloxacin (free base), of several dipeptide derivatives of this invention and of amino acid derivatives in dog after a 10 mg equivalent/kg oral dose are given in Table XI. More than 3 times the plasma level of the parent were achieved by the prodrugs of the invention. The plasma samples were collected and determined by the HPLC analysis.

**TABLE XI**

| Compound A | C max (range) (mcg/ml) | AUC (0-32h) (hr. mcg/mg) | No. of animals |
|---|---|---|---|
| H (Tosufloxacin) | 0.67 (0.60-7.4) | 5.3 | 4 |
| Norval | 2.3 (1.7-3.1) | 15.0 | 6 |
| Gly-phe | 2.1 (1.8-2.4) | 20.2 | 2 |
| Val | 2.2 (1.9-2.4) | 15.2 | 3 |
| Leu | 2.4 (1.5-3.2) | 15.8 | 3 |
| Ala-Norval | 2.3 (1.8-3.0) | 15.4 | 3 |
| Ala-Ala | 1.9 (1.3-3.2) | 10.6 | 7 |
| Norval-Norval | 1.9 (1.5-2.2) | 10.2 | 3 |
| Gly-Norval | 1.9 (1.5-2.2) | 13.9 | 3 |
| Gly-Gly | 1.5 (0.7-2.4) | 11.1 | 3 |

This invention has been described in terms of specific embodiments set forth in detail above. It should be understood, however, that these embodiments are presented by way of illustration only, and that the invention is not necessarily limited thereto. Modifications and variations within the spirit and scope of the claims that follow will be readily apparent from this disclosure, as those skilled in the art will appreciate.

## Claims (Claims for the following Contracting State(s): AT, BE, CH, LI, DE, FR, GB, IT, LU, NL, SE)

1. A compound of the formula 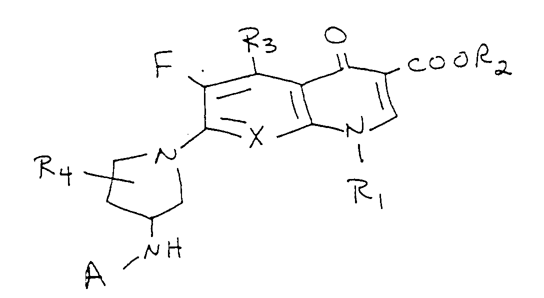 wherein R₁ is alkyl of one to six carbon atoms, haloalkyl of one to six carbon atoms containing one to three halogen substituents, hydroxyalkyl of one to six carbon atoms, cycloalkyl of three to six carbon atoms, vinyl, aryl having five to six atoms in the ring system which may include one to three heteroatoms selected from S, O and N, or said aryl substituted with one to three substituents independently selected from the group consisting of hydrogen, halogen, alkyl of one to six carbon atoms, haloalkyl of one to six carbon atoms containing one to three halogen substituents, alkanoyloxy of formula R₆COO-wherein R₆ is alkyl of one to six carbon atoms, and a group having the formula -Y-R₅, wherein Y is O or S and R₅ is hydrogen or alkyl of one to six carbon atoms;
R₂ is hydrogen, alkyl of one to six carbon atoms, haloalkyl of one to six carbon atoms containing one to three halogen substituents, or a carboxy protecting group;
R₃ is hydrogen or NH₂;
X is N, CH, COH, C-O-alkyl, CF, CCl, C-alkyl or C-NH-alkyl wherein alkyl in each case contains one to six carbon atoms;
R₄ is hydrogen, alkyl of one to six carbon atoms, or haloalkyl of one to six carbon atoms containing one to three halogen substituents;
A is a dipeptidyl group; and pharmaceutically acceptable salts thereof.

2. A compound according to Claim 1 wherein R₁ is a cyclopropyl, ethyl, haloethyl, phenyl, t-butyl or halo-substituted phenyl;
X is N, CH, CF, CCH₃ or CCl; and
R₂ is hydrogen or alkyl of one to six carbon atoms.

3. A compound according to Claim 1 wherein A is Gly-Phe, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Gly, Nval-Ala, Gly-Ala, Phe-Gly, D-Ala-L-Ala, Nval-Nval, Phe-Ala, Val-Ala, Val-Leu, Ala-Ala or Ala-Phe.

4. A compound of the formula 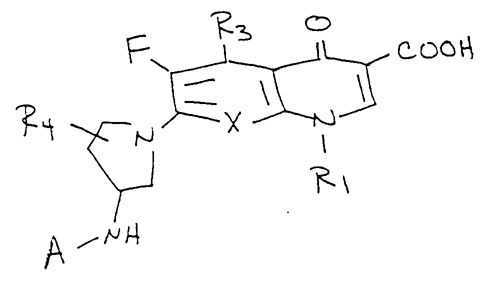 wherein R₁ is ethyl, t-butyl, 2-fluoroethyl, cyclopropyl, 4-fluorophenyl, or 2,4-difluorophenyl;
R₃ is hydrogen or NH₂;
X is N, CH, CF, CCH₃ or CCl; and
R₄ is hydrogen, alkyl of one to six carbon atoms, or haloalkyl of one to six carbon atoms containing one to three halogen substituents;
A is Gly-Phe, Phe-Gly, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Nval, D-Ala-L-Ala, Nval-Gly, Nval-Ala, Gly-Ala, Phe-Ala, Val-Ala, Val-Leu, Ala-Phe or Ala-Ala; and pharmaceutically acceptable salts thereof.

5. A compound according to Claim 4 selected from the group consisting of:
7-(3-Gly-Gly-amino-1-pyrrolidinyl)-6-fluoro-1-cyclopropyl-1,4-dihydro-4-oxo-quinoline-3-carboxylic acid;
7-(3-Gly-Phe-amino-1-pyrrolidinyl)-6-fluoro-1-(2,4-difluorophenyl)-1,4-dihydro-4-oxo-quinoline-3-carboxylic acid;
7-(3-Nval-Nval-amino-1-pyrrolidinyl)-6-fluoro-1-(2,4-difluorophenyl)-1,4-dihydro-4-oxo-quinoline-3-carboxylic acid;
7-(3-Gly-Nval-amino-1-pyrrolidinyl)-6-fluoro-1-cyclopropyl-1,4-dihydro-4-oxo-quinoline-3-carboxylic acid;
7-(3-Gly-Phe-amino-1-pyrrolidinyl)-6-fluoro-1-(2,4-difluorophenyl)-1,4-dihydro-4-oxo-1,8-naphthyridine-3-carboxylic acid;
7-(3-Gly-Gly-amino-1-pyrrolidinyl)-6-fluoro-1-(2,4-difluorophenyl)-1,4-dihydro-4-oxo-1,8-naphthyridien-3-carboxylic acid;
7-(3-Ala-Nval-amino-1-pyrrolidinyl)-6-fluoro-1-(2,4-difluorophenyl)-1,4-dihydro-4-oxo-1,8-naphthyridine-3-carboxylic acid;
7-(3-Ala-Ala-amino-1-pyrrolidinyl)-6-fluoro-1-(2,4-difluorophenyl)-1,4-dihydro-4-oxo-1,8-naphthyridine-3-carboxylic acid;
7-(3-Gly-Nval-amino-1-pyrrolidinyl)-6-fluoro-1-(2,4-difluorophenyl)-1,4-dihydro-4-oxo-1,8-naphthyridine-3-carboxylic acid;
7-(3-Ala-Phe-amino-1-pyrrolidinyl)-6-fluoro-1-(2,4-difluorophenyl)-1,4-dihydro-4-oxo-1,8-naphthyridine-3-carboxylic acid;
7-(3-Leu-Nval-amino-1-pyrrolidinyl)-6-fluoro-1-(2,4-difluorophenyl)-1,4-dihydro-4-oxo-1,8-naphthyridine-3-carboxylic acid;
7-(3-Met-Nval-amino-1-pyrrolidinyl)-6-fluoro-1-(2,4-difluorophenyl)-1,4-dihydro-4-oxo-1,8-naphthyridine-3-caboxylic acid;
7-(3-Nval-Gly-amino-1-pyrrolidinyl)-6-fluoro-1-(2,4-difluorophenyl)-1,4-dihydro-4-oxo-1,8-naphthyridine-3-carboxylic acid;
7-(3-Nval-Ala-amino-1-pyrrolidinyl)-6-fluoro-1-(2,4-difluorophenyl)-1,4-dihydro-4-oxo-1,8-naphthyridine-3-carboxylic acid;
7-(3-Nval-Nval-amino-1-pyrrolidinyl)-6-fluoro-1-(2,4-difluorophenyl)-1,4-dihydro-4-oxo-1,8-naphthyridine-3-carboxylic acid;
7-(3-Nval-Nval-amino-1-pyrrolidinyl)-6-fluoro-1-cyclopropyl-1,4-dihydro-4-oxo-quinoline-3-carboxylic acid;
7-(3-Gly-Phe-amino-1-pyrrolidinyl)-6-fluoro-1-cyclopropyl-1,4-dihydro-4-oxo-quinoline-3-carboxylic acid;
7-(3-Gly-Nval-amino-1-pyrrolidinyl)-6-fluoro-1-cyclopropyl-1,4-dihydro-4-oxo-1,8-naphthyridine-3-carboxylic acid;
7-(3-Gly-Gly-amino-1-pyrrolidinyl)-6-fluoro-1-cyclopropyl-1,4-dihydro-4-oxo-1,8-naphthyridine-3-carboxylic acid;
7-(3-Nval-Nval-amino-1-pyrrolidinyl)-6-fluoro-1-cyclopropyl-1,4-dihydro-4-oxo-1,8-naphthyridine-3-carboxylic acid;
7-(3-Ala-Met-amino-1-pyrrolidinyl)-6-fluoro-1-(2,4-difluorophenyl)-1,4-dihydro-4-oxo-1,8-naphthyridine-3-carboxylic acid;
7-(3-Met-Met-amino-1-pyrrolidinyl)-6-fluoro-1-(2,4-difluorophenyl)-1,4-dihydro-4-oxo-1,8-naphthyridine-3-carboxylic acid;
7-(3-Leu-Met-amino-1-pyrrolidinyl)-6-fluoro-1-(2,4-difluorophenyl)-1,4-dihydro-4-oxo-1,8-naphthyridine-3-carboxylic acid;
7-(3-Leu-Leu-amino-1-pyrrolidinyl)-6-fluoro-1-(2,4-difluorophenyl)-1,4-dihydro-4-oxo-1,8-naphthyridine-3-carboxylic acid;
7-(3-Ala-Leu-amino-1-pyrrolidinyl)-6-fluoro-1-(2,4-difluorophenyl)-1,4-dihydro-4-oxo-1,8-naphthyridine-3-carboxylic acid;
7-(3-Met-Leu-amino-1-pyrrolidinyl)-6-fluoro-1-(2,4-difluorophenyl)-1,4-dihydro-4-oxo-1,8-naphthyridine-3-carboxylic acid;
7-(3-Phe-Ala-amino-1-pyrrolidinyl)-6-fluoro-1-(2,4-difluorophenyl)-1,4-dihydro-4-oxo-1,8-naphthyridine-3-carboxylic acid;
7-(3-Leu-Ala-amino-1-pyrrolidinyl)-6-fluoro-1-(2,4-difluorophenyl)-1,4-dihydro-4-oxo-1,8-naphthyridine-3-carboxylic acid;
7-(3-Met-Ala-amino-1-pyrrolidinyl)-6-fluoro-1-(2,4-difluorophenyl)-1,4-dihydro-4-oxo-1,8-naphthyridine-3-carboxylic acid;
7-(3-Val-Ala-amino-1-pyrrolidinyl)-6-fluoro-1-(2,4-difluorophenyl)-1,4-dihydro-4-oxo-1,8-naphthyridine-3-carboxylic acid;
7-(3-Val-Leu-amino-1-pyrrolidinyl)-6-fluoro-1-(2,4-difluoropheny1)-1,4-dihydro-4-oxo-1,8-naphthyridine-3-carboxylic acid;
7-(3-D-Ala-L-Ala-amino-1-pyrrolidinyl)-6-fluoro-1-(2,4-difluorophenyl)-1,4-dihydro-4-oxo-1,8-naphthyridine-3-carboxylic acid;
7-(3-Gly-Ala-amino-1-pyrrolidinyl)-6-fluoro-1-(2,4-difluorophenyl)-1,4-dihydro-4-oxo-1,8-naphthyridine-3-carboxylic acid; and pharmaceutically acceptable salts thereof.

6. A compound according to Claim 1 for use as a therapeutic agent.

7. An antibacterial composition comprising an antibacterially effective amount of a compound of Claim 1.

8. Use of a compound according to Claim 1 for preparing a medicament for treating a bacterial infection in a mammal.

9. A compound selected from the group consisting of:
7-(3-Norvalylnorvalylamino-1-pyrrolidinyl)-1-(2,4-difluorophenyl)-6-fluoro-1,4-dihydro-4-oxo-1,8-naphthyridine-3-carboxylic acid;
7-(3-Glycylglycylamino-1-pyrrolidinyl)-1-(2,4-difluorophenyl)-6-fluoro-1,4-dihydro-4-oxo-1,8-naphthyridine-3-carboxylic acid;
7-(3-Alanylnorvalylamino-1-pyrrolidinyl)-1-(2,4-difluorophenyl)-6-fluoro-1,4-dihydro-4-oxo-1,8-naphthyridine-3-carboxylic acid;
7-(3-Glycylnorvalylamino-1-pyrrolidinyl)-1-(2,4-difluorophenyl)-6-fluoro-1,4-dihydro-4-oxo-1,8-naphthyridine-3-carboxylic acid;
7-(3-Alanylalanylamino-1-pyrrolidinyl)-1-(2,4-difluorophenyl)-6-fluoro-1,4-dihydro-4-oxo-1,8-naphthyridine-3-carboxylic acid; and
pharmaceutically acceptable salts thereof.

10. A compound according to Claim 9 for use as a therapeutic agent.

11. An antibacterial composition comprising an antibacterially effective amount of a compound of claim 9.

12. Use of a compound according to Claim 9 for preparing a medicament for treating a bacterial infection in a mammal.

13. A method of improving solubility of a compound of the formula: 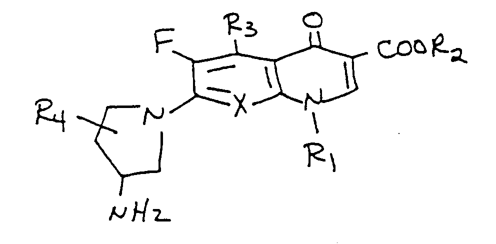 wherein R₁ is aryl having five to six atoms in the ring system which may include one to three heteroatoms selected from S, O and N, or said aryl substituted with one to three substituents independently selected from the group consisting of hydrogen, halogen, alkyl of one to six carbon atoms, haloalkyl of one to six carbon atoms containing one to three halogen substituents, alkanoyloxy of formula R₆COO-wherein R₆ is alkyl of one to six carbon atoms, and a group having the formula -Y-R₅, wherein Y is O or S and R₅ is hydrogen or alkyl of one to six carbon atoms;
R₂ is hydrogen, alkyl of one to six carbon atoms, haloalkyl of one to six carbon atoms containing one to three halogen substituents, or a carboxy protecting group;
R₃ is hydrogen or NH₂;
X is N, CH, COH, C-O-alkyl, CF, CCl, C-alkyl or C-NH-alkyl wherein alkyl in each case contains one to six carbon atoms;
R₄ is hydrogen, alkyl of one to six carbon atoms, or haloalkyl of one to six carbon atoms containing one to three halogen substituents;
the method comprising modifying the compound to a second compound of the formula: 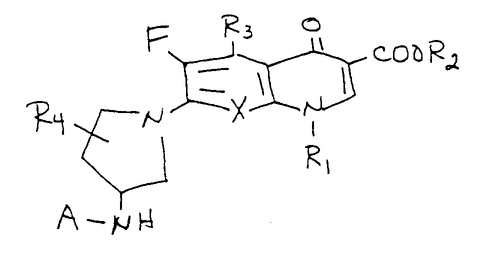 wherein R₁, R₂, R₃, R₄ and X are as above and
A is a dipeptidyl group; and pharmaceutically acceptable salts thereof.

14. A method of making a prodrug which hydrolyzes into the parent drug compound of the prodrug, the method comprising modifying a parent drug of the formula: 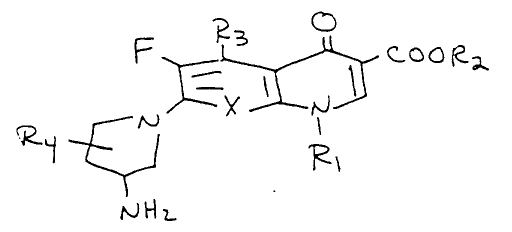 wherein R₁ is alkyl of one to six carbon atoms, haloalkyl of one to six carbon atoms containing one to three halogen substituents, hydroxyalkyl of one to six carbon atoms, cycloalkyl of three to six carbon atoms, vinyl, aryl having five to six atoms in the ring system which may include one to three heteroatoms selected from S, O and N, or said aryl substituted with one to three substituents independently selected from the group consisting of hydrogen, halogen, alkyl of one to six carbon atoms, haloalkyl of one to six carbon atoms containing one to three halogen substituents, alkanoyloxy of formula R₆COO-wherein R₆ is alkyl of one to six carbon atoms, and a group having the formula -Y-R₅, wherein Y is O or S and R₅ is hydrogen or alkyl of one to six carbon atoms;
R₂ is hydrogen, alkyl of one to six carbon atoms, haloalkyl of one to six carbon atoms containing one to three halogen substituents, or a carboxy protecting group;
R₃ is hydrogen or NH₂;
X is N, CH, COH, C-O-alkyl, CF, CCl, C-alkyl or C-NH-alkyl wherein alkyl in each case contains one to six carbon atoms;
R₄ is hydrogen, alkyl of one to six carbon atoms, or haloalkyl of one to six carbon atoms containing one to three halogen substituents;
and pharmaceutlcally acceptable salts thereof; by adding a dipeptidyl residue as a substituent of the amino function of the parent drug.

## Claims (Claims for the following Contracting State(s): ES)

1. A method of improving solubility of a compound of the formula: 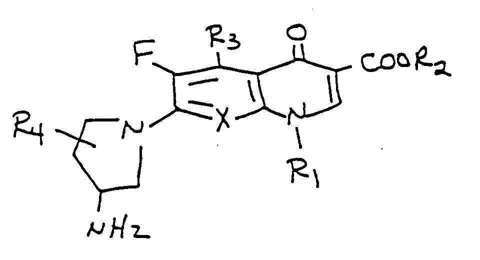 wherein R₁ is alkyl of one to six carbon atoms, haloalkyl of one to six carbon atoms containing one to three halogen substituents, hydroxyalkyl of one to six carbon atoms, cycloalkyl of three to six carbon atoms, vinyl, aryl having five to six atoms in the ring system which may include one to three heteroatoms selected from S, O and N, or said aryl substituted with one to three substituents independently selected from the group consisting of hydrogen, halogen, alkyl of one to six carbon atoms, haloalkyl of one to six carbon atoms containing one to three halogen substituents, alkanoyloxy of formula R₆COO-wherein R₆ is alkyl of one to six carbon atoms, and a group having the formula -Y-R₅, wherein Y is O or S and R₅ is hydrogen or alkyl of one to six carbon atoms;
R₂ is hydrogen, alkyl of one to six carbon atoms, haloalkyl of one to six carbon atoms containing one to three halogen substituents, or a carboxy protecting group;
R₃ is hydrogen or NH₂;
X is N, CH, COH, C-O-alkyl, CF, CCl, C-alkyl or C-NH-alkyl wherein alkyl in each case contains one to six carbon atoms;
R₄ is hydrogen, alkyl of one to six carbon atoms, or haloalkyl of one to six carbon atoms containing one to three halogen substituents;
the method comprising modifying the compound to a second compound of the formula: 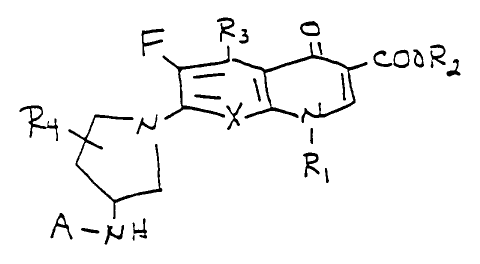 wherein R₁, R₂, R₃, R₄ and X are as above and
A is a dipeptidyl residue; and pharmaceutically acceptable salts thereof.

2. A method of making a prodrug which hydrolyzes into the parent drug compound of the prodrug, the method comprising modifying a parent drug of the formula: 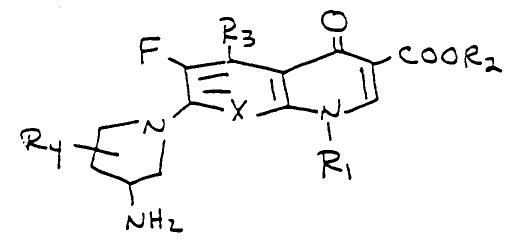 wherein R₁ is alkyl of one to six carbon atoms, haloalkyl of one to six carbon atoms containing one to three halogen substituents, hydroxyalkyl of one to six carbon atoms, cycloalkyl of three to six carbon atoms, vinyl, aryl having five to six atoms in the ring system which may include one to three heteroatoms selected from S, O and N, or said aryl substituted with one to three substituents independently selected from the group consisting of hydrogen, halogen, alkyl of one to six carbon atoms, haloalkyl of one to six carbon atoms containing one to three halogen substituents, alkanoyloxy of formula R₆COO-wherein R₆ is alkyl of one to six carbon atoms, and a group having the formula -Y-R₅, wherein Y is O or S and R₅ is hydrogen or alkyl of one to six carbon atoms;
R₂ is hydrogen, alkyl of one to six carbon atoms, haloalkyl of one to six carbon atoms containing one to three halogen substituents, or a carboxy protecting group;
R₃ is hydrogen or NH₂;
X is N, CH, COH, C-O-alkyl, CF, CCl, C-alkyl or C-NH-alkyl wherein alkyl in each case contains one to six carbon atoms;
R₄ is hydrogen, alkyl of one to six carbon atoms, or haloalkyl of one to six carbon atoms containing one to three halogen substituents;
and pharmaceutically acceptable salts thereof; by adding a dipeptidyl residue as a substituent of the amino function of the parent drug.

3. A method according to Claim 1 or 2 wherein said dipeptidyl residue is Gly-Phe, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Gly, Nval-Ala, Gly-Ala, Phe-Gly, D-Ala-L-Ala, Nval-Nval, Phe-Ala, Val-Ala, Val-Leu, Ala-Ala or Ala-Phe.

4. A method according to Claim 1 or 2 wherein R₁ is ethyl, t-butyl, 2-fluoroethyl, cyclopropyl, 4-fluorophenyl, or 2,4-difluorophenyl;
R₂ is hydrogen;
X is N, CH, CF, CCH₃ or CCl; and said dipeptidyl residue is Gly-Phe, Phe-Gly, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Nval, D-Ala-L-Ala, Nval-Gly, Nval-Ala, Gly-Ala, Phe-Ala, Val-Ala, Val-Leu, Ala-Phe or Ala-Ala.

5. A method according to Claim 1 or 2 wherein said method produces a compound selected from the group consisting of:
7-(3-Norvalylnorvalylamino-1-pyrrolidinyl)-1-(2,4-difluorophenyl)-6-fluoro-1,4-dihydro-4-oxo-1,8-naphthyridine-3-carboxylic acid;
7-(3-Glycylglycylamino-1-pyrrolidinyl)-1-(2,4-difluorophenyl)-6-fluoro-1,4-dihydro-4-oxo-1,8-naphthyridine-3-carboxylic acid;
7-(3-Alanylnorvalylamino-1-pyrrolidinyl)-1-(2,4-difluorophenyl)-6-fluoro-1,4-dihydro-4-oxo- 1,8-naphthyridine-3-carboxylic acid;
7-(3-Glycylnorvalylamino-1-pyrrolidinyl)-1-(2,4-difluorophenyl)-6-fluoro-1,4-dihydro-4-oxo-1,8-naphthyridine-3-carboxylic acid;
7-(3-Alanylalanylamino-1-pyrrolidinyl)-1-(2,4-difluoropnenyl)-6-fluoro-1,4-dihydro-4-oxo-1,8-naphthyridine-3-carboxylic acid; and
pharmaceutically acceptable salts thereof.

## Claims (Claims for the following Contracting State(s): GR)

1. A method of improving solubility of a compound of the formula: 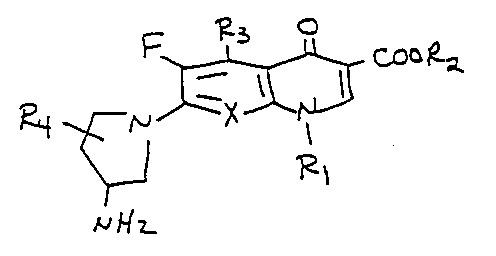 wherein R₁ is alkyl of one to six carbon atoms, haloalkyl of one to six carbon atoms containing one to three halogen substituents, hydroxyalkyl of one to six carbon atoms, cycloalkyl of three to six carbon atoms, vinyl, aryl having five to six atoms in the ring system which may include one to three heteroatoms selected from S, O and N, or said aryl substituted with one to three substituents independently selected from the group consisting of hydrogen, halogen, alkyl of one to six carbon atoms, haloalkyl of one to six carbon atoms containing one to three halogen substituents, alkanoyloxy of formula R₆COO-wherein R₆ is alkyl of one to six carbon atoms, and a group having the formula -Y-R₅, wherein Y is O or S and R₅ is hydrogen or alkyl of one to six carbon atoms;
R₂ is hydrogen, alkyl of one to six carbon atoms, haloalkyl of one to six carbon atoms containing one to three halogen substituents, or a carboxy protecting group;
R₃ is hydrogen or NH₂;
X is N, CH, COH, C-O-alkyl, CF, CCl, C-alkyl or C-NH-alkyl wherein alkyl in each case contains one to six carbon atoms;
R₄ is hydrogen, alkyl of one to six carbon atoms, or haloalkyl of one to six carbon atoms containing one to three halogen substituents;
the method comprising modifying the compound to a second compound of the formula: 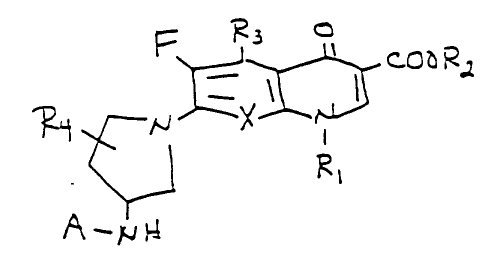 wherein R₁, R₂, R₃, R₄ and X are as above and
A is a dipeptidyl residue; and pharmaceutically acceptable salts thereof.

2. A method of making a prodrug which hydrolyzes into the parent drug compound of the prodrug, the method comprising modifying a parent drug of the formula: 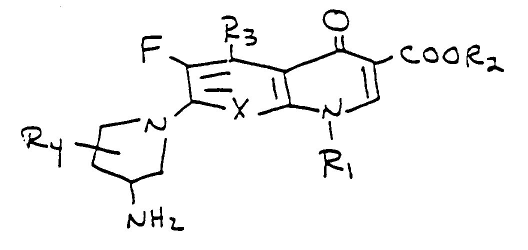 wherein R₁ is alkyl of one to six carbon atoms, haloalkyl of one to six carbon atoms containing one to three halogen substituents, hydroxyalkyl of one to six carbon atoms, cycloalkyl of three to six carbon atoms, vinyl, aryl having five to six atoms in the ring system which may include one to three heteroatoms selected from S, O and N, or said aryl substituted with one to three substituents independently selected from the group consisting of hydrogen, halogen, alkyl of one to six carbon atoms, haloalkyl of one to six carbon atoms containing one to three halogen substituents, alkanoyloxy of formula R₆COO-wherein R₆ is alkyl of one to six carbon atoms, and a group having the formula -Y-R₅, wherein Y is O or S and R₅ is hydrogen or alkyl of one to six carbon atoms;
R₂ is hydrogen, alkyl of one to six carbon atoms, haloalkyl of one to six carbon atoms containing one to three halogen substituents, or a carboxy protecting group;
R₃ is hydrogen or NH₂;
X is N, CH, COH, C-O-alkyl, CF, CCl, C-alkyl or C-NH-alkyl wherein alkyl in each case contains one to six carbon atoms;
R₄ is hydrogen, alkyl of one to six carbon atoms, or haloalkyl of one to six carbon atoms containing one to three halogen substituents;
and pharmaceutically acceptable salts thereof; by adding a dipeptidyl residue as a substituent of the amino function of the parent drug.

3. A method according to Claim 1 or 2 wherein said dipeptidyl residue is Gly-Phe, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Gly, Nval-Ala, Gly-Ala, Phe-Gly, D-Ala-L-Ala, Nval-Nval, Phe-Ala, Val-Ala, Val-Leu, Ala-Ala or Ala-Phe.

4. A method according to Claim 1 or 2 wherein R₁ is ethyl, t-butyl, 2-fluoroethyl, cyclopropyl, 4-fluorophenyl, or 2,4-difluorophenyl;
R₂ is hydrogen;
X is N, CH, CF, CCH₃ or CCl; and said dipeptidyl residue is Gly-Phe, Phe-Gly, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Nval, D-Ala-L-Ala, Nval-Gly, Nval-Ala, Gly-Ala, Phe-Ala, Val-Ala, Val-Leu, Ala-Phe or Ala-Ala.

5. A method according to Claim 1 or 2 wherein said method produces a compound selected from the group consisting of:
7-(3-Norvalylnorvalylamino-1-pyrrolidinyl)-1-(2,4-difluorophenyl)-6-fluoro-1,4-dihydro-4-oxo-1,8-naphthyridine-3-carboxylic acid;
7-(3-Glycylglycylamino-1-pyrrolidinyl)-1-(2,4-difluorophenyl)-6-fluoro-1,4-dihydro-4-oxo-1,8-naphthyridine-3-carboxylic acid;
7-(3-Alanylnorvalylamino-1-pyrrolidinyl)-1-(2,4-difluorophenyl)-6-fluoro-1,4-dihydro-4-oxo- 1,8-naphthyridine-3-carboxylic acid;
7-(3-Glycylnorvalylamino-1-pyrrolidinyl)-1-(2,4-difluorophenyl)-6-fluoro-1,4-dihydro-4-oxo-1,8-naphthyridine-3-carboxylic acid;
7-(3-Alanylalanylamino-1-pyrrolidinyl)-1-(2,4-difluoropnenyl)-6-fluoro-1,4-dihydro-4-oxo-1,8-naphthyridine-3-carboxylic acid; and
pharmaceutically acceptable salts thereof.

6. Use of compound of the formula 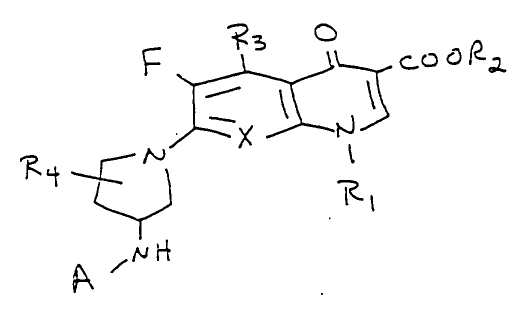 wherein R₁ is alkyl of one to six carbon atoms, haloalkyl of one to six carbon atoms containing one to three halogen substituents, hydroxyalkyl of one to six carbon atoms, cycloalkyl of three to six carbon atoms, vinyl, aryl having five to six atoms in the ring system which may include one to three heteroatoms selected from S, O and N, or said aryl substituted with one to three substituents independently selected from the group consisting of hydrogen, halogen, alkyl of one to six carbon atoms, haloalkyl of one to six carbon atoms containing one to three halogen substituents, alkanoyloxy of formula R₆COO-wherein R₆ is alkyl of one to six carbon atoms, and a group having the formula -Y-R₅, wherein Y is O or S and R₅ is hydrogen or alkyl of one to six carbon atoms;
R₂ is hydrogen, alkyl of one to six carbon atoms, haloalkyl of one to six carbon atoms containing one to three halogen substituents, or a carboxy protecting group;
R₃ is hydrogen or NH₂;
X is N, CH, COH, C-O-alkyl, CF, CCl, C-alkyl or C-NH-alkyl wherein alkyl in each case contains one to six carbon atoms;
R₄ is hydrogen, alkyl of one to six carbon atoms, or haloalkyl of one to six carbon atoms containing one to three halogen substituents;
A is a dipeptidyl group; and pharmaceutically acceptable salts thereof,
for preparing a medicament for treating a bacterial infection in a mammal.

7. The use according to Claim 6 wherein A is Gly-Phe, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Gly, Nval-Ala, Gly-Ala, Phe-Gly, D-Ala-L-Ala, Nval-Nval, Phe-Ala, Val-Ala, Val-Leu, Ala-Ala or Ala-Phe.

8. The use according to Claim 6 wherein R₁ is ethyl, t-butyl, 2-fluoroethyl, cyclopropyl, 4-fluorophenyl, or 2,4-difluorophenyl;
R₂ is hydrogen;
X is N, CH, CF, CCH₃ or CCl; and
A is Gly-Phe, Phe-Gly, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Nval, D-Ala-L-Ala, Nval-Gly, Nval-Ala, Gly-Ala, Phe-Ala, Val-Ala, Val-Leu, Ala-Phe or Ala-Ala.

9. Use according to Claim 6 wherein said compound is selected from the group consisting of:
7-(3-Norvalylnorvalylamino-1-pyrrolidinyl)-1-(2,4-difluorophenyl)-6-fluoro-1,4-dihydro-4-oxo-1,8-naphthyridine-3-carboxylic acid;
7-(3-Glycylglycylamino-1-pyrrolidinyl)-1-(2,4-difluorophenyl)-6-fluoro-1,4-dihydro-4-oxo-1,8-naphthyridine-3-carboxylic acid;
7-(3-Alanylnorvalylamino-1-pyrrolidinyl)-1-(2,4-difluorophenyl)-6-fluoro-1,4-dihydro-4-oxo-1,8-naphthyridine-3-carboxylic acid;
7-(3-Glycylnorvalylamino-1-pyrrolidinyl)-1-(2,4-difluorophenyl)-6-fluoro-1,4-dihydro-4-oxo-1,8-naphthyridine-3-carboxylic acid;
7-(3-Alanylalanylamino-1-pyrrolidinyl)-1-(2,4-difluorophenyl)-6-fluoro-1,4-dihydro-4-oxo-1,8-naphthyridine-3-carboxylic acid; and
pharmaceutically acceptable salts thereof.

## Patentansprüche (Patentansprüche für folgende(n) Vertragsstaat(en): AT, BE, CH, LI, DE, FR, GB, IT, LU, NL, SE)

1. Verbindung nach folgender Formel 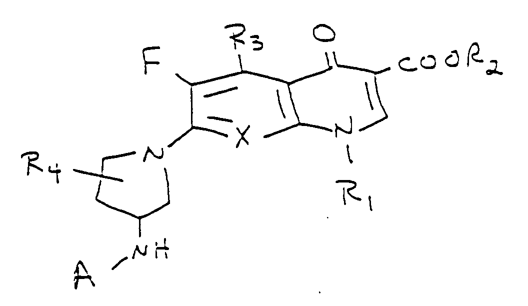 worin R₁ gleich Alkyl mit ein bis sechs Kohlenstoffatomen ist, gleich Haloalkyl mit ein bis sechs Kohlenstoffatomen, das ein bis drei Halogensubstituenten enthält, gleich Hydroxyalkyl mit ein bis sechs Kohlenstoffatomen, gleich Cycloalkyl mit drei bis sechs Kohlenstoffatomen, gleich Vinyl, gleich Aryl, das fünf bis sechs Atome im Ringsystem aufweist, das ein bis drei Heteroatome umfassen kann, die aus S, O und N gewählt sind, oder wobei das Arylsystem mit ein bis drei Substituenten substituiert ist, die unabhängig aus der Gruppe gewählt sind, die aus Wasserstoff, aus Halogen, aus Alkyl mit ein bis sechs Kohlenstoffatomen, aus Haloalkyl mit ein bis sechs Kohlenstoffatomen, das ein bis drei Halogensubstituenten enthält, aus Alkanoyloxy mit der Formel R₆COO-, worin R₆ gleich Alkyl mit ein bis sechs Kohlenstoffatomen ist, und aus einer Gruppe besteht, die die Formel -Y-R₅ aufweist, worin Y gleich O oder S ist, und worin R₅ gleich Wasserstoff oder Alkyl mit ein bis sechs Kohlenstoffatomen ist;
worin R₂ gleich Wasserstoff, gleich Alkyl mit ein bis sechs Kohlenstoffatomen, gleich Haloalkyl mit ein bis sechs Kohlenstoffatomen, das ein bis drei Halogensubstituenten enthält, oder gleich einer Carboxyschutzgruppe ist;
worin R₃ gleich Wasserstoff oder gleich NH₂ ist;
worin X gleich N, CH, COH, C-O-Alkyl, CF, CCl, C-Alkyl oder C-NH-Alkyl ist, worin das Alkyl in jedem Fall ein bis sechs Kohlenstoffatome enthält;
worin R₄ gleich Wasserstoff, gleich Alkyl mit ein bis sechs Kohlenstoffatomen oder gleich Haloalkyl mit ein bis sechs Kohlenstoffatomen ist, das ein bis drei Halogensubstituenten enthält;
worin A eine Dipeptidylguppe ist; und pharmazeutisch verträgliche Salze davon.

2. Verbindung nach Anspruch 1, worin R₁ gleich Cyclopropyl, Ethyl, Haloethyl, Phenyl, t-Butyl oder gleich halosubstituiertem Phenyl ist;
worin X gleich N, CH, CF, CCH₃ oder CCl ist; und
worin R₂ gleich Wasserstoff oder Alkyl mit ein bis sechs Kohlenstoffatomen ist.

3. Verbindung nach Anspruch 1, worin A gleich folgendem ist: Gly-Phe, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Gly, Nval-Ala, Gly-Ala, Phe-Gly, D-Ala-L-Ala, Nval-Nval, Phe-Ala, Val-Ala, Val-Leu, Ala-Ala oder Ala-Phe.

4. Verbindung nach folgender Formel 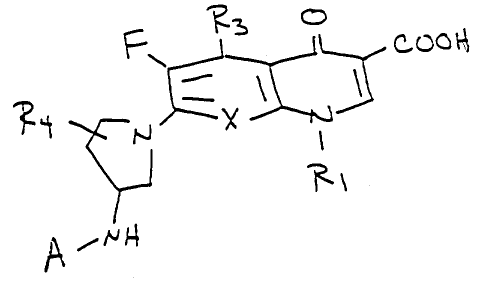 worin R₁ gleich Ethyl, t-Butyl, 2-Fluorethyl, Cyclopropyl, 4-Fluorphenyl oder 2,4-Difluorphenyl ist;
worin R₃ gleich Wasserstoff oder gleich NH₂ ist;
worin X gleich N, CH, CF, CCH₃ oder CCl ist; und
worin R₄ gleich Wasserstoff, gleich Alkyl mit ein bis sechs Kohlenstoffatomen oder gleich Haloalkyl mit ein bis sechs Kohlenstoffatomen ist, das ein bis drei Halogensubstituenten enthält,
worin A gleich folgendem ist Gly-Phe, Phe-Gly, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Nval, D-Ala-L-Ala, Nval-Gly, Nval-Ala, Gly-Ala, Phe-Ala, Val-Ala, Val-Leu, Ala-Phe oder; Ala-Ala; und pharmazeutisch verträgliche Salze davon.

5. Verbindung nach Anspruch 4, die aus der Gruppe gewählt ist, die aus folgendem besteht:
7-(3-Gly-Gly-amino-1-pyrrolidinyl)-6-fluor-1-cyclopropyl-1,4-dihydro-4-oxo-chinolin-3-carbonsäure,
7-(3-Gly-Phe-amino-1-pyrrolidinyl)-6-fluor-1-(2,4-difluorphenyl)-1,4-dihydro-4-oxo-chinolin-3-carbonsäure,
7-(3-Nval-Nval-amino-1-pyrrolidinyl)-6-fluor-1-(2,4-difluorphenyl)-1,4-dihydro-4-oxo-chinolin-3-carbonsäure,
7-(3-Gly-Nval-amino-1-pyrrolidinyl)-6-fluor-1-cyclopropyl-1,4-dihydro-4-oxo-chinolin-3-carbonsäure,
7-(3-Gly-Phe-amino-1-pyrrolidinyl)-6-fluor-1-(2,4-difluorphenyl)-1,4-dihydro-4-oxo-1,8-naphthyridin-3-carbonsäure,
7-(3-Gly-Gly-amino-1-pyrrolidinyl)-6-fluor-1-(2,4-difluorphenyl)-1,4-dihydro-4-oxo-1,8-naphthyridin-3-carbonsäure,
7-(3-Ala-Nval-amino-1-pyrrolidinyl)-6-fluor-1-(2,4-difluorphenyl)-1,4-dihydro-4-oxo-1,8-naphthyridin-3-carbonsäure,
7-(3-Ala-Ala-amino-1-pyrrolidinyl)-6-fluor-1-(2,4-difluorphenyl)-1,4-dihydro-4-oxo-1,8-naphthyridin-3-carbonsäure,
7-(3-Gly-Nval-amino-1-pyrrolidinyl)-6-fluor-1-(2,4-difluorphenyl)-1,4-dihydro-4-oxo-1,8-naphthyridin-3-carbonsäure,
7-(3-Ala-Phe-amino-1-pyrrolidinyl)-6-fluor-1-(2,4-difluorphenyl)-1,4-dihydro-4-oxo-1,8-naphthyridin-3-carbonsäure,
7-(3-Leu-Nval-amino-1-pyrrolidinyl)-6-fluor-1-(2,4-difluorphenyl)-1,4-dihydro-4-oxo-1,8-naphthyridin-3-carbonsäure,
7-(3-Met-Nval-amino-1-pyrrolidinyl)-6-fluor-1-(2,4-difluorphenyl)-1,4-dihydro-4-oxo-1,8-naphthyridin-3-carbonsäure,
7-(3-Nval-Gly-amino-1-pyrrolidinyl)-6-fluor-1-(2,4-difluorphenyl)-1,4-dihydro-4-oxo-1,8-naphthyridin-3-carbonsäure,
7-(3-Nval-Ala-amino-1-pyrrolidinyl)-6-fluor-1-(2,4-difluorphenyl)-1,4-dihydro-4-oxo-1,8-naphthyridin-3-carbonsäure,
7-(3-Nval-Nval-amino-1-pyrrolidinyl)-6-fluor-1-(2,4-difluorphenyl)-1,4-dihydro-4-oxo-1,8-naphthyridin-3-carbonsäure,
7-(3-Nval-Nval-amino-1-pyrrolidinyl)-6-fluor-1-cyclopropyl-1,4-dihydro-4-oxo-chinolin-3-carbonsäure,
7-(3-Gly-Phe-amino-1-pyrrolidinyl)-6-fluor-1-cyclopropyl-1,4-dihydro-4-oxo-chinolin-3-carbonsäure,
7-(3-Gly-Nval-amino-1-pyrrolidinyl)-6-fluor-1-cyclopropyl-1,4-dihydro-4-oxo-1,8-naphthyridin-3-carbonsäure,
7-(3-Gly-Gly-amino-1-pyrrolidinyl)-6-fluor-1-cyclopropyl-1,4-dihydro-4-oxo-1,8-naphthyridin-3-carbonsäure,
7-(3-Nval-Nval-amino-1-pyrrolidinyl)-6-fluor-1-cyclopropyl-1,4-dihydro-4-oxo-1,8-naphthyridin-3-carbonsäure,
7-(3-Ala-Met-amino-1-pyrrolidinyl)-6-fluor-1-(2,4-difluorphenyl)-1,4-dihydro-4-oxo-1,8-naphthyridin-3-carbonsäure,
7-(3-Met-Met-amino-1-pyrrolidinyl)-6-fluor-1-(2,4-difluorphenyl)-1,4-dihydro-4-oxo-1,8-naphthyridin-3-carbonsäure,
7-(3-Leu-Met-amino-1-pyrrolidinyl)-6-fluor-1-(2,4-difluorphenyl)-1,4-dihydro-4-oxo-1,8-naphthyridin-3-carbonsäure,
7-(3-Leu-Leu-amino-1-pyrrolidinyl)-6-fluor-1-(2,4-difluorphenyl)-1,4-dihydro-4-oxo-1,8-naphthyridin-3-carbonsäure,
7-(3-Ala-Leu-amino-1-pyrrolidinyl)-6-fluor-1-(2,4-difluorphenyl)-1,4-dihydro-4-oxo-1,8-naphthyridin-3-carbonsäure,
7-(3-Met-Leu-amino-1-pyrrolidinyl)-6-fluor-1-(2,4-difluorphenyl)-1,4-dihydro-4-oxo-1,8-naphthyridin-3-carbonsäure,
7-(3-Phe-Ala-amino-1-pyrrolidinyl)-6-fluor-1-(2,4-difluorphenyl)-1,4-dihydro-4-oxo-1,8-naphthyridin-3-carbonsäure,
7-(3-Leu-Ala-amino-1-pyrrolidinyl)-6-fluor-1-(2,4-difluorphenyl)-1,4-dihydro-4-oxo-1,8-naphthyridin-3-carbonsäure,
7-(3-Met-Ala-amino-1-pyrrolidinyl)-6-fluor-1-(2,4-difluorphenyl)-1,4-dihydro-4-oxo-1,8-naphthyridin-3-carbonsäure,
7-(3-Val-Ala-amino-1-pyrrolidinyl)-6-fluor-1-(2,4-difluorphenyl)-1,4-dihydro-4-oxo-1,8-naphthyridin-3-carbonsäure,
7-(3-Val-Leu-amino-1-pyrrolidinyl)-6-fluor-1-(2,4-difluorphenyl)-1,4-dihydro-4-oxo-1,8-naphthyridin-3-carbonsäure,
7-(3-D-Ala-L-Ala-amino-1-pyrrolidinyl)-6-fluor-1-(2,4-difluorphenyl)-1,4-dihydro-4-oxo-1,8-naphthyridin-3-carbonsäure,
7-(3-Gly-Ala-amino-1-pyrrolidinyl)-6-fluor-1-(2,4-difluorphenyl)-1,4-dihydro-4-oxo-1,8-naphthyridin-3-carbonsäure
und aus pharmazeutisch verträglichen Salzen davon.

6. Verbindung nach Anspruch 1 zur Verwendung als therapeutisches Agens.

7. Antibakterielle Zusammensetzung, die eine antibakteriell wirksame Menge einer Verbindung nach Anspruch 1 umfaßt.

8. Verwendung einer Verbindung nach Anspruch 1 zur Zubereitung eines Medikamentes zur Behandlung einer bakteriellen Infektion bei einem Säuger.

9. Verbindung, die aus der Gruppe gewählt ist, die aus folgendem besteht:
7-(3-Norvalylnorvalylamino-1-pyrrolidinyl)-1-(2,4-difluorphenyl)-6-fluor-1,4-dihydro-4-oxo-1,8-naphthyridin-3-carbonsäure,
7-(3-Glycylglycylamino-1-pyrrolidinyl)-1-(2,4-difluorphenyl)-6-fluor-1,4-dihydro-4-oxo-1,8-naphthyridin-3-carbonsäure,
7-(3-Alanylnorvalylamino-1-pyrrolidinyl)-1-(2,4-difluorphenyl)-6-fluor-1,4-dihydro-4-oxo-1,8-naphthyridin-3-carbonsäure,
7-(3-Glycylnorvalylamino-1-pyrrolidinyl)-1-(2,4-difluorphenyl)-6-fluor-1,4-dihydro-4-oxo-1,8-naphthyridin-3-carbonsäure,
7-(3-Alanylalanylamino-1-pyrrolidinyl)-1-(2,4-difluorphenyl)-6-fluor-1,4-dihydro-4-oxo-1,8-naphthyridin-3-carbonsäure und aus pharmazeutisch verträglichen Salzen davon.

10. Verbindung nach Anspruch 9 zur Verwendung als therapeutisches Agens.

11. Antibakterielle Zusammensetzung, die eine antibakteriell wirksame Menge einer Verbindung nach Anspruch 9 umfaßt.

12. Verwendung einer Verbindung nach Anspruch 9 zur Zubereitung eines Medikamentes zur Behandlung einer bakteriellen Infektion bei einem Säuger.

13. Verfahren zur Verbesserung der Löslichkeit einer Verbindung nach folgender Formel: 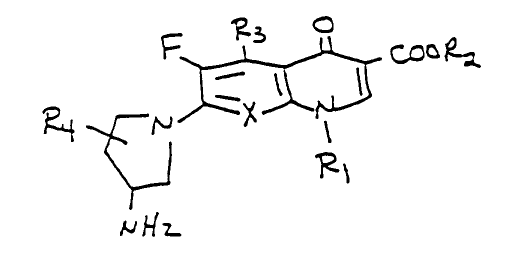 worin R₁ gleich Aryl ist, das fünf bis sechs Atome im Ringsystem aufweist, das ein bis drei Heteroatome umfassen kann, die aus S, O und N gewählt sind, oder wobei das Arylsystem mit ein bis drei Substituenten substituiert ist, die unabhängig aus der Gruppe gewählt sind, die aus Wasserstoff, aus Halogen, aus Alkyl mit ein bis sechs Kohlenstoffatomen, aus Haloalkyl mit ein bis sechs Kohlenstoffatomen, das ein bis drei Halogensubstituenten enthält, aus Alkanoyloxy mit der Formel R₆COO-, worin R₆ gleich Alkyl mit ein bis sechs Kohlenstoffatomen ist, und aus einer Gruppe besteht, die die Formel -Y-R₅ aufweist, worin Y gleich O oder S ist, und worin R₅ gleich Wasserstoff oder Alkyl mit ein bis sechs Kohlenstoffatomen ist;
worin R₂ gleich Wasserstoff, gleich Alkyl mit ein bis sechs Kohlenstoffatomen, gleich Haloalkyl mit ein bis sechs Kohlenstoffatomen, das ein bis drei Halogensubstituenten enthält, oder gleich einer Carboxyschutzgruppe ist;
worin R₃ gleich Wasserstoff oder gleich NH₂ ist;
worin X gleich N, CH, COH, C-O-Alkyl, CF, CCl, C-Alkyl oder C-NH-Alkyl ist, worin Alkyl in jedem Fall ein bis sechs Kohlenstoffatome enthält;
worin R₄ gleich Wasserstoff, gleich Alkyl mit ein bis sechs Kohlenstoffatomen oder gleich Haloalkyl mit ein bis sechs Kohlenstoffatomen ist, das ein bis drei Halogensubstituenten enthält;
wobei das Verfahren die Abwandlung der Verbindung zu einer zweiten Verbindung nach folgender Formel umfaßt: 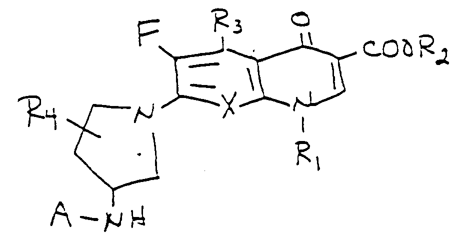 worin R₁, R₂, R₃, R₄ und X die oben genannte Bedeutung haben, und worin
A eine Dipeptidylgruppe ist; und pharmazeutisch verträgliche Salze davon.

14. Verfahren zur Herstellung einer "prodrug"-Substanz, die zu der Stammwirkstoffverbindung der "prodrug"-Substanz hydrolysiert, wobei das Verfahren die Abwandlung eines Stammwirkstoffes nach folgender Formel umfaßt: 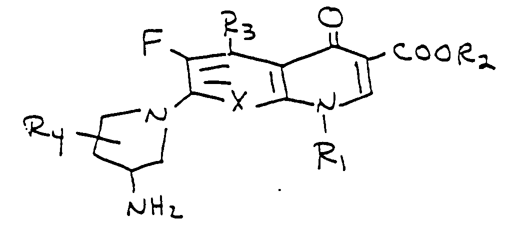 worin R₁ gleich Alkyl mit ein bis sechs Kohlenstoffatomen ist, gleich Haloalkyl mit ein bis sechs Kohlenstoffatomen, das ein bis drei Halogensubstituenten enthält, gleich Hydroxyalkyl mit ein bis sechs Kohlenstoffatomen, gleich Cycloalkyl mit drei bis sechs Kohlenstoffatomen, gleich Vinyl, gleich Aryl, das fünf bis sechs Atome im Ringsystem aufweist, das ein bis drei Heteroatome umfassen kann, die aus S, O und N gewählt sind, oder wobei das Arylsystem mit ein bis drei Substituenten substituiert ist, die unabhängig aus der Gruppe gewählt sind, die aus Wasserstoff, aus Halogen, aus Alkyl mit ein bis sechs Kohlenstoffatomen, aus Haloalkyl mit ein bis sechs Kohlenstoffatomen, das ein bis drei Halogensubstituenten enthält, aus Alkanoyloxy mit der Formel R₆COO-, worin R₆ gleich Alkyl mit ein bis sechs Kohlenstoffatomen ist, und aus einer Gruppe besteht, die die Formel -Y-R₅ aufweist, worin Y gleich O oder S ist, und worin R₅ gleich Wasserstoff oder gleich Alkyl mit ein bis sechs Kohlenstoffatomen ist;
worin R₂ gleich Wasserstoff, gleich Alkyl mit ein bis sechs Kohlenstoffatomen, gleich Haloalkyl mit ein bis sechs Kohlenstoffatomen, das ein bis drei Halogensubstituenten enthält, oder gleich einer Carboxyschutzgruppe ist;
worin R₃ gleich Wasserstoff oder gleich NH₂ ist;
worin X gleich N, CH, COH, C-O-Alkyl, CF, CCl, C-Alkyl oder C-NH-Alkyl ist, worin das Alkyl in jedem Fall ein bis sechs Kohlenstoffatome enthält;
worin R₄ gleich Wasserstoff, gleich Alkyl mit ein bis sechs Kohlenstoffatomen oder gleich Haloalkyl mit ein bis sechs Kohlenstoffatomen ist, das ein bis drei Halogensubstituenten enthält;
und pharmazeutisch verträgliche Salze davon, indem ein Dipeptidylrest als Substituent an die Aminofunktion des Stammwirkstoffes addiert wird.

## Patentansprüche (Patentansprüche für folgende(n) Vertragsstaat(en): ES)

1. Verfahren zur Verbesserung der Löslichkeit einer Verbindung nach folgender Formel: 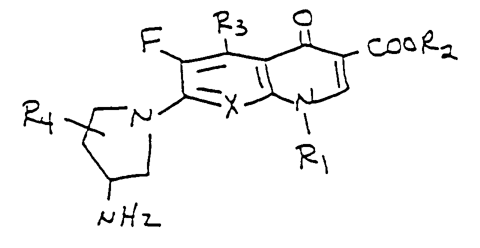 worin R₁ gleich Alkyl mit ein bis sechs Kohlenstoffatomen ist, gleich Haloalkyl mit ein bis sechs Kohlenstoffatomen, das ein bis drei Halogensubstituenten enthält, gleich Hydroxyalkyl mit ein bis sechs Kohlenstoffatomen, gleich Cycloalkyl mit drei bis sechs Kohlenstoffatomen, gleich Vinyl, gleich Aryl, das fünf bis sechs Atome im Ringsystem aufweist, das ein bis drei Heteroatome umfassen kann, die aus S, O und N gewählt sind, oder wobei das Arylsystem mit ein bis drei Substituenten substituiert ist, die unabhängig aus der Gruppe gewählt sind, die aus Wasserstoff, aus Halogen, aus Alkyl mit ein bis sechs Kohlenstoffatomen, aus Haloalkyl mit ein bis sechs Kohlenstoffatomen, das ein bis drei Halogensubstituenten enthält, aus Alkanoyloxy mit der Formel R₆COO-, worin R₆ gleich Alkyl mit ein bis sechs Kohlenstoffatomen ist, und aus einer Gruppe besteht, die die Formel -Y-R₅ aufweist, worin Y gleich O oder S ist, und worin R₅ gleich Wasserstoff oder Alkyl mit ein bis sechs Kohlenstoffatomen ist;
worin R₂ gleich Wasserstoff, gleich Alkyl mit ein bis sechs Kohlenstoffatomen, gleich Haloalkyl mit ein bis sechs Kohlenstoffatomen, das ein bis drei Halogensubstituenten enthält, oder gleich einer Carboxyschutzgruppe ist;
worin R₃ gleich Wasserstoff oder gleich NH₂ ist;
worin X gleich N, CH, COH, C-O-Alkyl, CF, CCl, C-Alkyl oder C-NH-Alkyl ist, worin Alkyl in jedem Fall ein bis sechs Kohlenstoffatome enthält;
worin R₄ gleich Wasserstoff, gleich Alkyl mit ein bis sechs Kohlenstoffatomen oder gleich Haloalkyl mit ein bis sechs Kohlenstoffatomen ist, das ein bis drei Halogensubstituenten enthält;
wobei das Verfahren die Abwandlung der Verbindung zu einer zweiten Verbindung nach folgender Formel umfaßt: 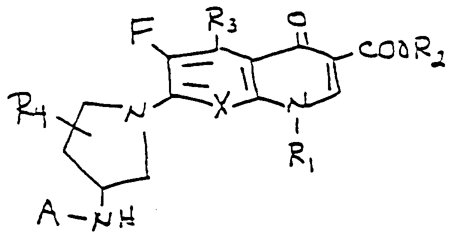 worin R₁, R₂, R₃, R₄ und X die oben genannte Bedeutung haben, und worin
A ein Dipeptidylrest ist; und pharmazeutisch verträgliche Salze davon.

2. Verfahren zur Herstellung einer "prodrug"-Substanz, die zu der Stammwirkstoffverbindung der "prodrug"-Substanz hydrolysiert, wobei das Verfahren die Abwandlung eines Stammwirkstoffes nach folgender Formel umfaßt: 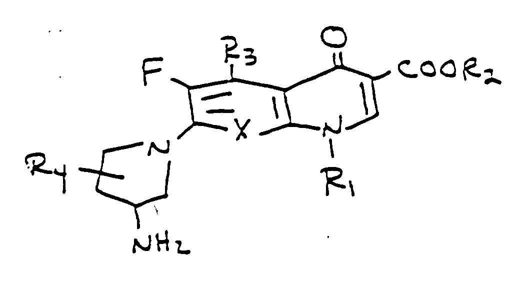 worin R₁ gleich Alkyl mit ein bis sechs Kohlenstoffatomen ist, gleich Haloalkyl mit ein bis sechs Kohlenstoffatomen, das ein bis drei Halogensubstituenten enthält, gleich Hydroxyalkyl mit ein bis sechs Kohlenstoffatomen, gleich Cycloalkyl mit drei bis sechs Kohlenstoffatomen, gleich Vinyl, gleich Aryl, das fünf bis sechs Atome im Ringsystem aufweist, das ein bis drei Heteroatome umfassen kann, die aus S, O und N gewählt sind, oder wobei das Arylsystem mit ein bis drei Substituenten substituiert ist, die unabhängig aus der Gruppe gewählt sind, die aus Wasserstoff, aus Halogen, aus Alkyl mit ein bis sechs Kohlenstoffatomen, aus Haloalkyl mit ein bis sechs Kohlenstoffatomen, das ein bis drei Halogensubstituenten enthält, aus Alkanoyloxy mit der Formel R₆COO-, worin R₆ gleich Alkyl mit ein bis sechs Kohlenstoffatomen ist, und aus einer Gruppe besteht, die die Formel -Y-R₅ aufweist, worin Y gleich O oder S ist, und worin R₅ gleich Wasserstoff oder gleich Alkyl mit ein bis sechs Kohlenstoffatomen ist;
worin R₂ gleich Wasserstoff, gleich Alkyl mit ein bis sechs Kohlenstoffatomen, gleich Haloalkyl mit ein bis sechs Kohlenstoffatomen, das ein bis drei Halogensubstituenten enthält, oder gleich einer Carboxyschutzgruppe ist;
worin R₃ gleich Wasserstoff oder gleich NH₂ ist;
worin X gleich N, CH, COH, C-O-Alkyl, CF, CCl, C-Alkyl oder C-NH-Alkyl ist, worin das Alkyl in jedem Fall ein bis sechs Kohlenstoffatome enthält;
worin R₄ gleich Wasserstoff, gleich Alkyl mit ein bis sechs Kohlenstoffatomen oder gleich Haloalkyl mit ein bis sechs Kohlenstoffatomen ist, das ein bis drei Halogensubstituenten enthält;
und pharmazeutisch verträgliche Salze davon, indem ein Dipeptidylrest als Substituent an die Aminofunktion des Stammwirkstoffes addiert wird.

3. Verfahren nach Anspruch 1 oder 2, worin der Dipeptidylrest gleich folgendem ist: Gly-Phe, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Gly, Nval-Ala, Gly-Ala, Phe-Gly, D-Ala-L-Ala, Nval-Nval, Phe-Ala, Val-Ala, Val-Leu, Ala-Ala oder Ala-Phe.

4. Verfahren nach Anspruch 1 oder 2, worin R₁ gleich Ethyl, t-Butyl, 2-Fluorethyl, Cyclopropyl oder gleich 4-Fluorphenyl oder 2,4-Difluorphenyl ist;
worin R₂ gleich Wasserstoff ist;
worin X gleich N, CH, CF, CCH₃ oder CCl ist; und worin der Dipeptidylrest gleich folgendem ist Gly-Phe, Phe-Gly, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Nval, D-Ala-L-Ala, Nval-Gly, Nval-Ala, Gly-Ala, Phe-Ala, Val-Ala, Val-Leu, Ala-Phe oder Ala-Ala.

5. Verfahren nach Anspruch 1 oder 2, wobei das Verfahren eine Verbindung erzeugt, die aus der Gruppe gewählt ist, die aus folgendem besteht:
7-(3-Norvalylnorvalylamino-1-pyrrolidinyl)-1-(2,4-difluorphenyl)-6-fluor-1,4-dihydro-4-oxo-1,8-naphthyridin-3-carbonsäure,
7-(3-Glycylglycylamino-1-pyrrolidinyl)-1-(2,4-difluorphenyl)-6-fluor-1,4-dihydro-4-oxo-1,8-naphthyridin-3-carbonsäure,
7-(3-Alanylnorvalylamino-1-pyrrolidinyl)-1-(2,4-difluorphenyl)-6-fluor-1,4-dihydro-4-oxo-1,8-naphthyridin-3-carbonsäure,
7-(3-Glycylnorvalylamino-1-pyrrolidinyl)-1-(2,4-difluorphenyl)-6-fluor-1,4-dihydro-4-oxo-1,8-naphthyridin-3-carbonsäure,
7-(3-Alanylalanylamino-1-pyrrolidinyl)-1-(2,4-difluorphenyl)-6-fluor-1,4-dihydro-4-oxo-1,8-naphthyridin-3-carbonsäure und aus
pharmazeutisch verträglichen Salzen davon.

## Patentansprüche (Patentansprüche für folgende(n) Vertragsstaat(en): GR)

1. Verfahren zur Verbesserung der Löslichkeit einer Verbindung nach folgender Formel: 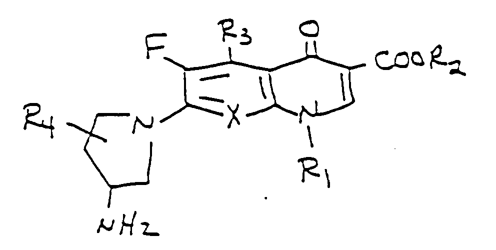 worin R₁ gleich Alkyl mit ein bis sechs Kohlenstoffatomen ist, gleich Haloalkyl mit ein bis sechs Kohlenstoffatomen, das ein bis drei Halogensubstituenten enthält, gleich Hydroxyalkyl mit ein bis sechs Kohlenstoffatomen, gleich Cycloalkyl mit drei bis sechs Kohlenstoffatomen, gleich Vinyl, gleich Aryl, das fünf bis sechs Atome im Ringsystem aufweist, das ein bis drei Heteroatome umfassen kann, die aus S, O und N gewählt sind, oder wobei das Arylsystem mit ein bis drei Substituenten substituiert ist, die unabhängig aus der Gruppe gewählt sind, die aus Wasserstoff, aus Halogen, aus Alkyl mit ein bis sechs Kohlenstoffatomen, aus Haloalkyl mit ein bis sechs Kohlenstoffatomen, das ein bis drei Halogensubstituenten enthält, aus Alkanoyloxy mit der Formel R₆COO-, worin R₆ gleich Alkyl mit ein bis sechs Kohlenstoffatomen ist, und aus einer Gruppe besteht, die die Formel -Y-R₅ aufweist, worin Y gleich O oder S ist, und worin R₅ gleich Wasserstoff oder Alkyl mit ein bis sechs Kohlenstoffatomen ist;
worin R₂ gleich Wasserstoff, gleich Alkyl mit ein bis sechs Kohlenstoffatomen, gleich Haloalkyl mit ein bis sechs Kohlenstoffatomen, das ein bis drei Halogensubstituenten enthält, oder gleich einer Carboxyschutzgruppe ist;
worin R₃ gleich Wasserstoff oder gleich NH₂ ist;
worin X gleich N, CH, COH, C-O-Alkyl, CF, CCl, C-Alkyl oder C-NH-Alkyl ist, worin Alkyl in jedem Fall ein bis sechs Kohlenstoffatome enthält;
worin R₄ gleich Wasserstoff, gleich Alkyl mit ein bis sechs Kohlenstoffatomen oder gleich Haloalkyl mit ein bis sechs Kohlenstoffatomen ist, das ein bis drei Halogensubstituenten enthält;
wobei das Verfahren die Abwandlung der Verbindung zu einer zweiten Verbindung nach folgender Formel umfaßt: 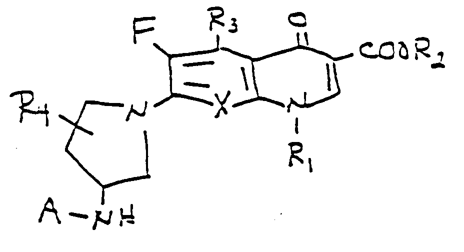 worin R₁, R₂, R₃, R₄ und X die oben genannte Bedeutung haben, und worin
A ein Dipeptidylrest ist; und pharmazeutisch verträgliche Salze davon.

2. Verfahren zur Herstellung einer "prodrug"-Substanz, die zu der Stammwirkstoffverbindung der "prodrug"-Substanz hydrolysiert, wobei das Verfahren die Abwandlung eines Stammwirkstoffes nach folgender Formel umfaßt: 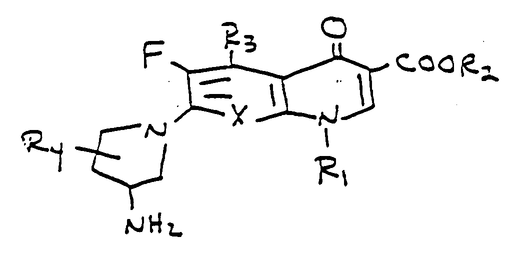 worin R₁ gleich Alkyl mit ein bis sechs Kohlenstoffatomen ist, gleich Haloalkyl mit ein bis sechs Kohlenstoffatomen, das ein bis drei Halogensubstituenten enthält, gleich Hydroxyalkyl mit ein bis sechs Kohlenstoffatomen, gleich Cycloalkyl mit, drei bis sechs Kohlenstoffatomen, gleich Vinyl, gleich Aryl, das fünf bis sechs Atome im Ringsystem aufweist, das ein bis drei Heteroatome umfassen kann, die aus S, O und N gewählt sind, oder wobei das Arylsystem mit ein bis drei Substituenten substituiert ist, die unabhängig aus der Gruppe gewählt sind, die aus Wasserstoff, aus Halogen, aus Alkyl mit ein bis sechs Kohlenstoffatomen, aus Haloalkyl mit ein bis sechs Kohlenstoffatomen, das ein bis drei Halogensubstituenten enthält, aus Alkanoyloxy mit der Formel R₆COO-, worin R₆ gleich Alkyl mit ein bis sechs Kohlenstoffatomen ist, und aus einer Gruppe besteht, die die Formel -Y-R₅ aufweist, worin Y gleich O oder S ist, und worin R₅ gleich Wasserstoff oder gleich Alkyl mit ein bis sechs Kohlenstoffatomen ist;
worin R₂ gleich Wasserstoff, gleich Alkyl mit ein bis sechs Kohlenstoffatomen, gleich Haloalkyl mit ein bis sechs Kohlenstoffatomen, das ein bis drei Halogensubstituenten enthält, oder gleich einer Carboxyschutzgruppe ist;
worin R₃ gleich Wasserstoff oder gleich NH₂ ist;
worin X gleich N, CH, COH, C-O-Alkyl, CF, CCl, C-Alkyl oder C-NH-Alkyl ist, worin das Alkyl in jedem Fall ein bis sechs Kohlenstoffatome enthält;
worin R₄ gleich Wasserstoff, gleich Alkyl mit ein bis sechs Kohlenstoffatomen oder gleich Haloalkyl mit ein bis sechs Kohlenstoffatomen ist, das ein bis drei Halogensubstituenten enthält;
und pharmazeutisch verträgliche Salze davon, indem ein Dipeptidylrest als Substituent an die Aminofunktion des Stammwirkstoffes addiert wird.

3. Verfahren nach Anspruch 1 oder 2, worin der Dipeptidylrest gleich folgendem ist: Gly-Phe, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Gly, Nval-Ala, Gly-Ala, Phe-Gly, D-Ala-L-Ala, Nval-Nval, Phe-Ala, Val-Ala, Val-Leu, Ala-Ala oder Ala-Phe.

4. Verfahren nach Anspruch 1 oder 2, worin R₁ gleich Ethyl, t-Butyl, 2-Fluorethyl, Cyclopropyl oder gleich 4-Fluorphenyl oder 2,4-Difluorphenyl ist;
worin R₂ gleich Wasserstoff ist;
worin X gleich N, CH, CF, CCH₃ oder CCl ist; und worin der Dipeptidylrest gleich folgendem ist Gly-Phe, Phe-Gly, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Nval, D-Ala-L-Ala, Nval-Gly, Nval-Ala, Gly-Ala, Phe-Ala, Val-Ala, Val-Leu, Ala-Phe oder Ala-Ala.

5. Verfahren nach Anspruch 1 oder 2, wobei das Verfahren eine Verbindung erzeugt, die aus der Gruppe gewählt ist, die aus folgendem besteht:
7-(3-Norvalylnorvalylamino-1-pyrrolidinyl)-1-(2,4-difluorphenyl)-6-fluor-1,4-dihydro-4-oxo-1,8-naphthyridin-3-carbonsäure,
7-(3-Glycylglycylamino-1-pyrrolidinyl)-1-(2,4-difluorphenyl)-6-fluor-1,4-dihydro-4-oxo-1,8-naphthyridin-3-carbonsäure,
7-(3-Alanylnorvalylamino-1-pyrrolidinyl)-1-(2,4-difluorphenyl)-6-fluor-1,4-dihydro-4-oxo-1,8-naphthyridin-3-carbonsäure,
7-(3-Glycylnorvalylamino-1-pyrrolidinyl)-1-(2,4-difluorphenyl)-6-fluor-1,4-dihydro-4-oxo-1,8-naphthyridin-3-carbonsäure,
7-(3-Alanylalanylamino-1-pyrrolidinyl)-1-(2,4-difluorphenyl)-6-fluor-1,4-dihydro-4-oxo-1,8-naphthyridin-3-carbonsäure und aus
pharmazeutisch verträglichen Salzen davon.

6. Verwendung einer Verbindung nach folgender Formel 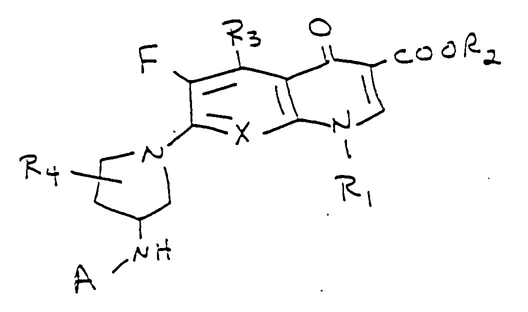 worin R₁ gleich Alkyl mit ein bis sechs Kohlenstoffatomen ist, gleich Haloalkyl mit ein bis sechs Kohlenstoffatomen, das ein bis drei Halogensubstituenten enthält, gleich Hydroxyalkyl mit ein bis sechs Kohlenstoffatomen, gleich Cycloalkyl mit drei bis sechs Kohlenstoffatomen, gleich Vinyl, gleich Aryl, das fünf bis sechs Atome im Ringsystem aufweist, das ein bis drei Heteroatome umfassen kann, die aus S, O und N gewählt sind, oder wobei das Arylsystem mit ein bis drei Substituenten substituiert ist, die unabhängig aus der Gruppe gewählt sind, die aus Wasserstoff, aus Halogen, aus Alkyl mit ein bis sechs Kohlenstoffatomen, aus Haloalkyl mit ein bis sechs Kohlenstoffatomen, das ein bis drei Halogensubstituenten enthält, aus Alkanoyloxy mit der Formel R₆COO-, worin R₆ gleich Alkyl mit ein bis sechs Kohlenstoffatomen ist, und aus einer Gruppe besteht, die die Formel -Y-R₅ aufweist, worin Y gleich O oder S ist, und worin R₅ gleich Wasserstoff oder gleich Alkyl mit ein bis sechs Kohlenstoffatomen ist;
worin R₂ gleich Wasserstoff, gleich Alkyl mit ein bis sechs Kohlenstoffatomen, gleich Haloalkyl mit ein bis sechs Kohlenstoffatomen, das ein bis drei Halogensubstituenten enthält, oder gleich einer Carboxyschutzgruppe ist;
worin R₃ gleich Wasserstoff oder gleich NH₂ ist;
worin X gleich N, CH, COH, C-O-Alkyl, CF, CCl, C-Alkyl oder C-NH-Alkyl ist, worin das Alkyl in jedem Fall ein bis sechs Kohlenstoffatome enthält;
worin R₄ gleich Wasserstoff, gleich Alkyl mit ein bis sechs Kohlenstoffatomen oder gleich Haloalkyl mit ein bis sechs Kohlenstoffatomen ist, das ein bis drei Halogensubstituenten enthält;
worin A eine Dipeptidylgruppe ist;
und von pharmazeutisch verträglichen Salzen davon zur Zubereitung eines Medikamentes zur Behandlung einer bakteriellen Infektion bei einem Säuger.

7. Verwendung gemäß Anspruch 6, wobei A gleich folgendem ist Gly-Phe, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Gly, Nval-Ala, Gly-Ala, Phe-Gly, D-Ala-L-Ala, Nval-Nval, Phe-Ala, Val-Ala, Val-Leu, Ala-Ala oder Ala-Phe.

8. Verwendung gemäß Anspruch 6, wobei
R₁ gleich Ethyl, t-Butyl, 2-Fluorethyl, Cyclopropyl oder gleich 4-Fluorphenyl oder 2,4-Difluorphenyl ist;
wobei R₂ gleich Wasserstoff ist;
wobei X gleich N, CH, CF, CCH₃ oder CCl ist; und wobei A gleich folgendem ist Gly-Phe, Phe-Gly, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Nval, D-Ala-L-Ala, Nval-Gly, Nval-Ala, Gly-Ala, Phe-Ala, Val-Ala, Val-Leu, Ala-Phe oder Ala-Ala.

9. Verwendung gemäß Anspruch 6, wobei die Verbindung aus der Gruppe gewählt ist, die aus folgendem besteht:
7-(3-Norvalylnorvalylamino-1-pyrrolidinyl)-1-(2,4-difluorphenyl)-6-fluor-1,4-dihydro-4-oxo-1,8-naphthyridin-3-carbonsäure,
7-(3-Glycylglycylamino-1-pyrrolidinyl)-1-(2,4-difluorphenyl)-6-fluor-1,4-dihydro-4-oxo-1,8-naphthyridin-3-carbonsäure,
7-(3-Alanylnorvalylamino-1-pyrrolidinyl)-1-(2,4-difluorphenyl)-6-fluor-1,4-dihydro-4-oxo-1,8-naphthyridin-3-carbonsäure,
7-(3-Glycylnorvalylamino-1-pyrrolidinyl)-1-(2,4-difluorphenyl)-6-fluor-1,4-dihydro-4-oxo-1,8-naphthyridin-3-carbonsäure,
7-(3-Alanylalanylamino-1-pyrrolidinyl)-1-(2,4-difluorphenyl)-6-fluor-1,4-dihydro-4-oxo-1,8-naphthyridin-3-carbonsäure und aus
pharmazeutisch verträglichen Salzen davon.

## Revendications (Revendications pour l'(les) Etat(s) contractant(s) suivant(s): AT, BE, CH, LI, DE, FR, GB, IT, LU, NL, SE)

1. Composé de formule : 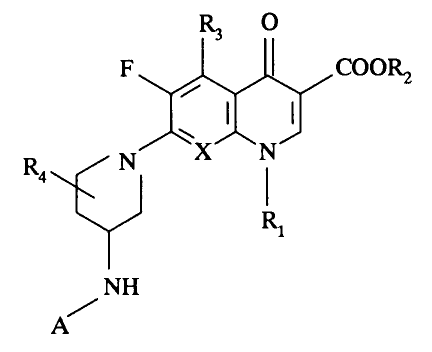 dans laquelle R₁ représente un groupement alkyle ayant de 1 à 6 atomes de carbone, un groupement halogénoalkyle ayant de 1 à 6 atomes de carbone, contenant de 1 à 3 substituants halogène, un groupement hydroxyalkyle ayant de 1 à 6 atomes de carbone, un groupement cycloalkyle ayant de 3 à 6 atomes de carbone, un groupement vinyle, un groupement aryle ayant 5 ou 6 atomes dans le système de cycle qui peut inclure de 1 à 3 hétéroatomes choisis parmi S, O et N, ou bien ledit groupement aryle substitué par 1 à 3 substituants choisis indépendamment dans le groupe constitué des suivants : atome d'hydrogène, atome d'halogène, groupement alkyle ayant de 1 à 6 atomes de carbone, groupement halogénoalkyle ayant de 1 à 6 atomes de carbone, contenant de 1 à 3 substituants halogène, groupement alcanoyloxy de formule R₆COO-, dans laquelle R₆ représente un groupement alkyle ayant de 1 à 6 atomes de carbone, et groupement de formule -Y-R₅, dans laquelle Y représente O ou S et R₅ représente l'atome d'hydrogène ou un groupement alkyle ayant de 1 à 6 atomes de carbone ;
R₂ représente l'atome d'hydrogène, un groupement alkyle ayant de 1 à 6 atomes de carbone, un groupement halogénoalkyle ayant de 1 à 6 atomes de carbone, contenant de 1 à 3 substituants halogène, ou un groupement protecteur du carboxy ;
R₃ représente l'atome d'hydrogène ou NH₂ ;
X représente N, CH, COH, C-O-alkyle, CF, CCl, C-alkyle ou C-NH-alkyle où, dans chaque cas, le groupement alkyle contient de 1 à 6 atomes de carbone ;
R₄ représente l'atome d'hydrogène, un groupement alkyle ayant de 1 à 6 atomes ou un groupement halogénoalkyle ayant de 1 à 6 atomes de carbone, contenant de 1 à 3 substituants halogène ;
A représente un groupement dipeptidyle ; et ses sels pharmaceutiquement acceptables.

2. Composé selon la revendication 1, pour lequel R₁ représente un groupement du type cyclopropyle, éthyle, halogénoéthyle, phényle, t-butyle ou phényle halogéno-substitué ;
X représente N, CH, CF, CCH₃ ou CCl ; et
R₂ représente l'atome d'hydrogène ou un groupement alkyle ayant de 1 à 6 atomes de carbone.

3. Composé selon la revendication 1, pour lequel A représente Gly-Phe, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Gly, Nval-Ala, Gly-Ala, Phe-Gly, D-Ala-L-Ala, Nval-Nval, Phe-Ala, Val-Ala, Val-Leu, Ala-Ala ou Ala-Phe.

4. Composé de formule : 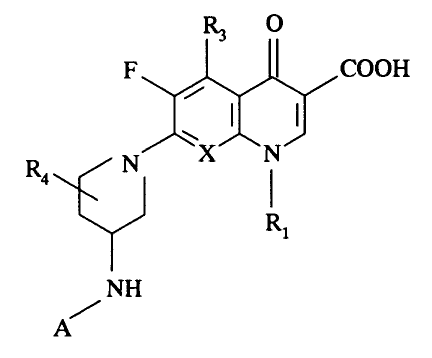 dans laquelle R₁ représente un groupement du type éthyle, t-butyle, 2-fluoroéthyle, cyclopropyle, 4-fluorophényle ou 2,4-difluorophényle ;
R₃ représente l'atome d'hydrogène ou NH₂ ;
X représente N, CH, CF, CCH₃ ou CCl ; et
R₄ représente l'atome d'hydrogène, un groupement alkyle ayant de 1 à 6 atomes de carbone ou un groupement halogénoalkyle ayant de 1 à 6 atomes de carbone, contenant de 1 à 3 substituants halogène ;
A représente Gly-Phe, Phe-Gly, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Nval, D-Ala-L-Ala, Nval-Gly, Nval-Ala, Gly-Ala, Phe-Ala, Val-Ala, Val-Leu, Ala-Phe ou Ala-Ala ; et ses sels pharmaceutiquement acceptables.

5. Composé selon la revendication 4, choisi dans le groupe constitué des suivants :
acide 7-(3-Gly-Gly-amino-1-pyrrolidinyl)-6-fluoro-1-cyclopropyl-1,4-dihydro-4-oxo-quinoléine-3-carboxylique ;
acide 7-(3-Gly-Phe-amino-1-pyrrolidinyl)-6-fluoro-1-(2,4-difluorophényl)-1,4-dihydro-4-oxo-quinoléine-3-carboxylique ;
acide 7-(3-Nval-Nval-amino-1-pyrrolidinyl)-6-fluoro-1-(2,4-difluorophényl)-1,4-dihydro-4-oxo-quinoléine-3-carboxylique ;
acide 7-(3-Gly-Nval-amino-1-pyrrolidinyl)-6-fluoro-1-cyclopropyl-1,4-dihydro-4-oxo-quinoléine-3-carboxylique ;
acide 7-(3-Gly-Phe-amino-1-pyrrolidinyl)-6-fluoro-1-(2,4-difluorophényl)-1,4-dihydro-4-oxo-1,8-naphtyridine-3-carboxylique ;
acide 7-(3-Gly-Gly-amino-1-pyrrolidinyl)-6-fluoro-1-(2,4-difluorophényl)-1,4-dihydro-4-oxo-1,8-naphtyridine-3-carboxylique ;
acide 7-(3-Ala-Nval-amino-1-pyrrolidinyl)-6-fluoro-1-(2,4-difluorophényl)-1,4-dihydro-4-oxo-1,8-naphtyridine-3-carboxylique ;
acide 7-(3-Ala-Ala-amino-1-pyrrolidinyl)-6-fluoro-1-(2,4-difluorophényl)-1,4-dihydro-4-oxo-1,8-naphtyridine-3-carboxylique ;
acide 7-(3-Gly-Nval-amino-1-pyrrolidinyl)-6-fluoro-1-(2,4-difluorophényl)-1,4-dihydro-4-oxo-1,8-naphtyridine-3-carboxylique ;
acide 7-(3-Ala-Phe-amino-1-pyrrolidinyl)-6-fluoro-1-(2,4-difluorophényl)-1,4-dihydro-4-oxo-1,8-naphtyridine-3-carboxylique ;
acide 7-(3-Leu-Nval-amino-1-pyrrolidinyl)-6-fluoro-1-(2,4-difluorophényl)-1,4-dihydro-4-oxo-1,8-naphtyridine-3-carboxylique ;
acide 7-(3-Met-Nval-amino-1-pyrrolidinyl)-6-fluoro-1-(2,4-difluorophényl)-1,4-dihydro-4-oxo-1,8-naphtyridine-3-carboxylique ;
acide 7-(3-Nval-Gly-amino-1-pyrrolidinyl)-6-fluoro-1-(2,4-difluorophényl)-1,4-dihydro-4-oxo-1,8-naphtyridine-3-carboxylique ;
acide 7-(3-Nval-Ala-amino-1-pyrrolidinyl)-6-fluoro-1-(2,4-difluorophényl)-1,4-dihydro-4-oxo-1,8-naphtyridine-3-carboxylique ;
acide 7-(3-Nval-Nval-amino-1-pyrrolidinyl)-6-fluoro-1-(2,4-difluorophényl)-1,4-dihydro-4-oxo-1,8-naphtyridine-3-carboxylique ;
acide 7-(3-Nval-Nval-amino-1-pyrrolidinyl)-6-fluoro-1-cyclopropyl-1,4-dihydro-4-oxo-quinoléine-3-carboxylique ;
acide 7-(3-Gly-Phe-amino-1-pyrrolidinyl)-6-fluoro-1-cyclopropyl-1,4-dihydro-4-oxo-quinoléine-3-carboxylique ;
acide 7-(3-Gly-Nval-amino-1-pyrrolidinyl)-6-fluoro-1-cyclopropyl-1,4-dihydro-4-oxo-1,8-naphtyridine-3-carboxylique ;
acide 7-(3-Gly-Gly-amino-1-pyrrolidinyl)-6-fluoro-1-cyclopropyl-1,4-dihydro-4-oxo-1,8-naphtyridine-3-carboxylique ;
acide 7-(3-Nval-Nval-amino-1-pyrrolidinyl)-6-fluoro-1-cyclopropyl-1,4-dihydro-4-oxo-1,8-naphtyridine-3-carboxylique ;
acide 7-(3-Ala-Met-amino-1-pyrrolidinyl)-6-fluoro-1-(2,4-difluorophényl)-1,4-dihydro-4-oxo-1,8-naphtyridine-3-carboxylique ;
acide 7-(3-Met-Met-amino-1-pyrrolidinyl)-6-fluoro-1-(2,4-difluorophényl)-1,4-dihydro-4-oxo-1,8-naphtyridine-3-carboxylique ;
acide 7-(3-Leu-Met-amino-1-pyrrolidinyl)-6-fluoro-1-(2,4-difluorophényl)-1,4-dihydro-4-oxo-1,8-naphtyridine-3-carboxylique ;
acide 7-(3-Leu-Leu-amino-1-pyrrolidinyl)-6-fluoro-1-(2,4-difluorophényl)-1,4-dihydro-4-oxo-1,8-naphtyridine-3-carboxylique ;
acide 7-(3-Ala-Leu-amino-1-pyrrolidinyl)-6-fluoro-1-(2,4-difluorophényl)-1,4-dihydro-4-oxo-1,8-naphtyridine-3-carboxylique ;
acide 7-(3-Met-Leu-amino-1-pyrrolidinyl)-6-fluoro-1-(2,4-difluorophényl)-1,4-dihydro-4-oxo-1,8-naphtyridine-3-carboxylique ;
acide 7-(3-Phe-Ala-amino-1-pyrrolidinyl)-6-fluoro-1-(2,4-difluorophényl)-1,4-dihydro-4-oxo-1,8-naphtyridine-3-carboxylique ;
acide 7-(3-Leu-Ala-amino-1-pyrrolidinyl)-6-fluoro-1-(2,4-difluorophényl)-1,4-dihydro-4-oxo-1,8-naphtyridine-3-carboxylique ;
acide 7-(3-Met-Ala-amino-1-pyrrolidinyl)-6-fluoro-1-(2,4-difluorophényl)-1,4-dihydro-4-oxo-1,8-naphtyridine-3-carboxylique ;
acide 7-(3-Val-Ala-amino-1-pyrrolidinyl)-6-fluoro-1-(2,4-difluorophényl)-1,4-dihydro-4-oxo-1,8-naphtyridine-3-carboxylique ;
acide 7-(3-Val-Leu-amino-1-pyrrolidinyl)-6-fluoro-1-(2,4-difluorophényl)-1,4-dihydro-4-oxo-1,8-naphtyridine-3-carboxylique ;
acide 7-(3-D-Ala-L-Ala-amino-1-pyrrolidinyl)-6-fluoro-1-(2,4-difluorophényl)-1,4-dihydro-4-oxo-1,8-naphtyridine-3-carboxylique ;
acide 7-(3-Gly-Ala-amino-1-pyrrolidinyl)-6-fluoro-1-(2,4-difluorophényl)-1,4-dihydro-4-oxo-1,8-naphtyridine-3-carboxylique ; et de leurs sels pharmaceutiquement acceptables.

6. Composé selon la revendication 1, à utiliser en tant qu'agent thérapeutique.

7. Composition antibactérienne comprenant une quantité antibactérienne efficace d'un composé selon la revendication 1.

8. Utilisation d'un composé selon la revendication 1 pour la préparation d'un médicament destiné au traitement d'une infection bactérienne chez un mammifère.

9. Composé choisi dans le groupe constitué des suivants :
acide 7-(3-norvalylnorvalylamino-1-pyrrolidinyl)-1-(2,4-difluorophényl)-6-fluoro-1,4-dihydro-4-oxo-1,8-naphtyridine-3-carboxylique ;
acide 7-(3-glycylglycylamino-1-pyrrolidinyl)-1-(2,4-difluorophényl)-6-fluoro-1,4-dihydro-4-oxo-1,8-naphtyridine-3-carboxylique ;
acide 7-(3-alanylnorvalylamino-1-pyrrolidinyl)-1-(2,4-difluorophényl)-6-fluoro-1,4-dihydro-4-oxo-1,8-naphtyridine-3-carboxylique ;
acide 7-(3-glycylnorvalylamino-1-pyrrolidinyl)-1-(2,4-difluorophényl)-6-fluoro-1,4-dihydro-4-oxo-1,8-naphtyridine-3-carboxylique ;
acide 7-(3-alanylalanylamino-1-pyrrolidinyl)-1-(2,4-difluorophényl)-6-fluoro-1,4-dihydro-4-oxo-1,8-naphtyridine-3-carboxylique ; et de leurs sels
pharmaceutiquement acceptables.

10. Composé selon la revendication 9, à utiliser en tant qu'agent thérapeutique.

11. Composition antibactérienne comprenant une quantité antibactérienne efficace d'un composé selon la revendication 9.

12. Utilisation d'un composé selon la revendication 9 pour la préparation d'un médicament destiné au traitement d'une infection bactérienne chez un mammifère.

13. Procédé d'amélioration de la solubilité d'un composé de formule : 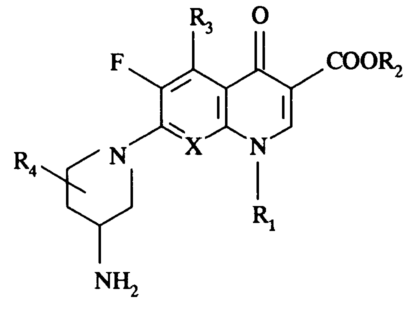 dans laquelle R₁ représente un groupement aryle ayant 5 ou 6 atomes dans le système de cycle peut inclure de 1 à 3 hétéroatomes choisis parmi S, O et N, ou bien ledit aryle substitué par de 1 à 3 substituants choisis indépendamment dans le groupe constitué des suivants : atome d'hydrogène, atome d'halogène, groupement alkyle ayant de 1 à 6 atomes de carbone, groupement halogénoalkyle ayant de 1 à 6 atomes de carbone, contenant de 1 à 3 substituants halogène, groupement alcanoyloxy de formule R₆COO-, dans laquelle R₆ représente un groupement alkyle ayant de 1 à 6 atomes de carbone, et groupement de formule -Y-R₅, dans laquelle Y représente O ou S et R₅ représente l'atome d'hydrogène ou un groupement alkyle ayant de 1 à 6 atomes de carbone ;
R₂ représente l'atome d'hydrogène, un groupement alkyle ayant de 1 à 6 atomes de carbone, un groupement halogénoalkyle ayant de 1 à 6 atomes de carbone, contenant de 1 à 3 substituants halogène, ou un groupement protecteur du carboxy ;
R₃ représente l'atome d'hydrogène ou NH₂ ;
X représente N, CH, COH, C-O-alkyle, CF, CCl, C-alkyle ou C-NH-alkyle où, dans chaque cas, le groupement alkyle contient de 1 à 6 atomes de carbone ;
R₄ représente l'atome d'hydrogène, un groupement alkyle ayant de 1 à 6 atomes de carbone ou un groupement halogénoalkyle ayant de 1 à 6 atomes de carbone, contenant de 1 à 3 substituants halogène ;
le procédé comprenant la modification du composé en un deuxième composé de formule : 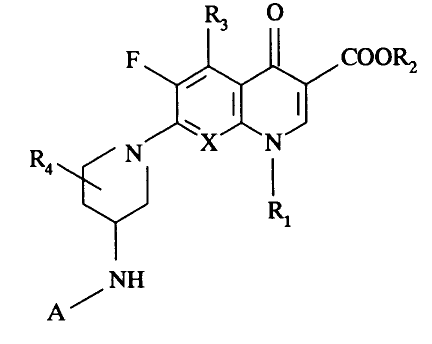 dans laquelle R₁, R₂, R₃, R₄ et X sont tels que définis ci-dessus et
A représente un groupement dipeptidyle ; et de ses sels pharmaceutiquement acceptables.

14. Procédé de préparation d'un précurseur de médicament qui s'hydrolyse en le composé médicament apparenté du précurseur de médicament, le procédé comprenant la modification d'un médicament apparenté de formule : 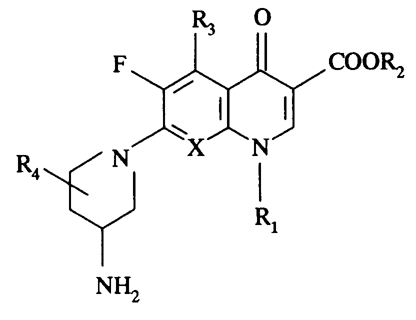 dans laquelle R₁ représente un groupement alkyle ayant de 1 à 6 atomes de carbone, un groupement halogénoalkyle ayant de 1 à 6 atomes de carbone, contenant de 1 à 3 substituants halogène, un groupement hydroxyalkyle ayant de 1 à 6 atomes de carbone, un groupement cycloalkyle ayant de 3 à 6 atomes de carbone, un groupement vinyle, un groupement aryle ayant 5 ou 6 atomes dans le système de cycle qui peut inclure de 1 à 3 hétéroatomes choisis parmi S, O et N, ou ledit groupement aryle substitué par 1 à 3 substituants choisis indépendamment dans le groupe constitué des suivants : atome d'hydrogène, atome d'halogène, groupement alkyle ayant de 1 à 6 atomes de carbone, groupement halogénoalkyle ayant de 1 à 6 atomes de carbone, contenant de 1 à 3 substituants halogène, groupement alcanoyloxy de formule R₆COO-, dans laquelle R₆ représente un groupement alkyle ayant de 1 à 6 atomes de carbone, et groupement de formule -Y-R₅, dans laquelle Y représente O ou S et R₅ représente l'atome d'hydrogène ou un groupement alkyle ayant de 1 à 6 atomes de carbone ;
R₂ représente l'atome d'hydrogène, un groupement alkyle ayant de 1 à 6 atomes de carbone, un groupement halogénoalkyle ayant de 1 à 6 atomes de carbone, contenant de 1 à 3 substituants halogène, ou un groupement protecteur du carboxy ;
R₃ représente l'atome d'hydrogène ou NH₂ ;
X représente N, CH, COH, C-O-alkyle, CF, CCl, C-alkyle ou C-NH-alkyle où, dans chaque cas, le groupement alkyle contient de 1 à 6 atomes de carbone ;
R₄ représente l'atome d'hydrogène, un groupement alkyle ayant de 1 à 6 atomes de carbone ou un groupement halogénoalkyle ayant de 1 à 6 atomes de carbone, contenant de 1 à 3 substituants halogène ;
et de ses sels pharmaceutiquement acceptables ;
en ajoutant un résidu dipeptidyle en tant que substituant de la fonction amino du médicament apparenté.

## Revendications (Revendications pour l'(les) Etat(s) contractant(s) suivant(s): ES)

1. Procédé d'amélioration de la solubilité d'un composé de formule : 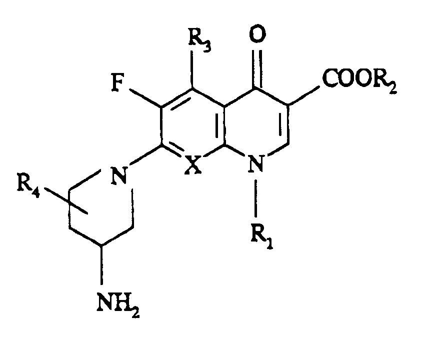 dans laquelle R₁ représente un groupement alkyle ayant de 1 à 6 atomes de carbone, un groupement halogénoalkyle ayant de 1 à 6 atomes de carbone, contenant de 1 à 3 substituants halogène, un groupement hydroxyalkyle ayant de 1 a 6 atomes de carbone, un groupement cycloalkyle ayant de 3 à 6 atomes de carbone, un groupement vinyle, un groupement aryle ayant 5 ou 6 atomes dans le système de cycle qui peut inclure de 1 à 3 hétéroatomes choisis parmi S, O et N, ou bien ledit groupement aryle substitué par 1 à 3 substituants choisis indépendamment dans le groupe constitué des suivant : atome d'hydrogène, atome d'halogène, groupement alkyle ayant de 1 à 6 atomes de carbone, groupement halogénoalkyle ayant de 1 à 6 atomes de carbone, contenant de 1 à 3 substituants halogène, groupement alcanoyloxy de formule R₆COO-, dans laquelle R₆ représente un groupement alkyle ayant de 1 à 6 atomes de carbone, et groupement de formule -Y-R₅, dans laquelle Y représente O ou S et R₅ représente l'atome d'hydrogène ou un groupement alkyle ayant de 1 à 6 atomes de carbone ;
R₂ représente l'atome d'hydrogène, un groupement alkyle ayant de 1 à 6 atomes de carbone, un groupement halogénoalkyle ayant de 1 à 6 atomes de carbone, contenant de 1 à 3 substituants halogène, ou un groupement protecteur du carboxy ;
R₃ représente l'atome d'hydrogène ou NH₂ ;
X représente N, CH, COH, C-O-alkyle, CF, CCl, C-alkyle ou C-NH-alkyle où, dans chaque cas, le groupement alkyle contient de 1 à 6 atomes de carbone ;
R₄ représente l'atome d'hydrogène, un groupement alkyle ayant de 1 à 6 atomes de carbone ou un groupement halogénalkyle ayant de 1 à 6 atomes de carbone, contenant de 1 à 3 substituant halogène ;
le procédé comprenant la modification du composé en un deuxième composé de formule : 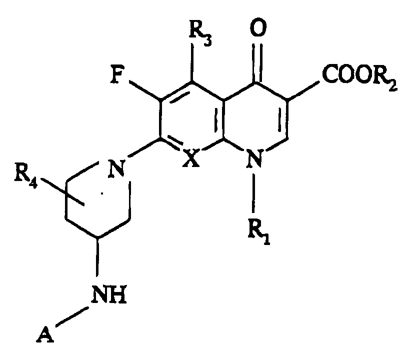 dans laquelle R₁, R₂, R₃, R₄ et X sont tels que définis ci-dessus et A représente un résidu dipeptidyle ; et de ses sels pharmaceutiquement acceptables.

2. Procédé de préparation d'un précurseur de médicament qui s'hydrolyse en le composé médicament apparenté du précurseur de médicament, le procédé comprenant la modification d'un médicament apparenté de formule : 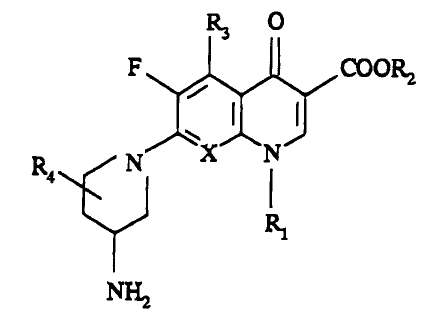 dans laquelle R₁ représente un groupement alkyle ayant de 1 à 6 atomes de carbone, un groupement halogénoalkyle ayant de 1 à 6 atomes de carbone, contenant de 1 à 3 substituants halogène, un groupement hydroxyalkyle ayant de 1 à 6 atomes de carbone, un groupement cycloalkyle ayant de 3 à 6 atomes de carbone, un groupement vinyle, un groupement aryle ayant 5 ou 6 atomes dans le système de cycle qui peut inclure de 1 à 3 hétéroatomes choisis parmi S, O et N, ou ledit groupement aryle substitué par 1 à 3 substituants choisis indépendamment dans le groupe constitué des suivants : atome d'hydrogène, atome d'halogène, groupement alkyle ayant de 1 à 6 atomes de carbone, groupement halogénalkyle ayant de 1 à 6 atomes de carbone, contenant de 1 à 3 substituants halogène, groupement alcanoyloxy de formule R₆COO-, dans laquelle R₆ représente un groupement alkyle ayant de 1 à 6 atomes de carbone, et groupement de formule -Y-R₅, dans laquelle Y représente O ou S et R₅ représente l'atome d'hydrogène ou un groupement alkyle ayant de 1 à 6 atomes de carbone ;
R₂ représente l'atome d'hydrogène, un groupement alkyle ayant de 1 à 6 atomes de carbone, un groupement halogénoalkyle ayant de 1 à 6 atomes de carbone, contenant de 1 à 3 substituants halogène, ou un groupement protecteur du carboxy ;
R₃ représente l'atome d'hydrogène ou NH₂ ;
X représente N, CH, COH, C-O-alkyle, CF, CCl, C-alkyle ou C-NH-alkyle où dans chaque cas, le groupement alkyle contient de 1 à 6, atomes de carbone ;
R₄ représente l'atome d'hydrogène, un groupement alkyle ayant de 1 à 6 atomes de carbone ou un groupement halogénoalkyle ayant de 1 à 6 atomes de carbone, contenant de 1 à 3 substituants halogène ;
et de ses sels pharmaceutiquement acceptables ;
en ajoutant a résidu dipeptidyle en tant que substituant de la fonction amino du médicament apparenté.

3. Procédé selon la revendication 1 ou 2, dans lequel ledit résidu dipeptidyle représente Gly-Phe, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Gly, Nval-Ala, Gly-Ala, Phe-Gly, D-Ala-L-Ala, Nval-Nval, Phe-Ala, Val-Ala, Val-Leu, Ala-Ala ou Ala-Phe.

4. Procédé selon la revendication 1 ou 2, dans lequel R₁ représente un groupement du type éthyle, t-butyle, 2-fluoroéthyle, cyclopropyle, 4-fluorophényle ou 2,4-difluorophényle ;
R₂ représente l'atome d'hydrogène ;
X représente N, CH, CF, CCH₃ ou CCl ; et
ledit résidu dipeptidyle représente Gly-Phe, Phe-Gly, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Nval, D-Ala-L-Ala, Nval-Gly, Nval-Ala, Gly-Ala, Phe-Ala, Val-Ala, Val-Leu, Ala-Phe ou Ala-Ala.

5. Procédé selon la revendication 1 ou 2, dans lequel ledit procédé produit un composé choisi dans le groupe constitué des suivants :
acide 7-(3-norvalylnorvalylamino-1-pyrrolidinyl)-1-(2,4-difluorophényl)-6-fluoro-1,4-dihydro-4-oxo-1,8-naphtyridine-3-carboxylique ;
acide 7-(3-glycylglycylamino-1-pyrrolidinyl)-1-(2,4-difluorophényl)-6-fluoro-1,4-dihydro-4-oxo-1,8-naphtyridine-3-carboxylique ;
acide 7-(3-alanynorvalylamino-1-pyrrolidinyl)-1-(2,4-difluorophényl)-6-fluoro-1,4-dihydro-4-oxo-1,8-naphtyridine-3-carboxylique ;
acide 7-(3-glycylnorvalylamino-1-pyrrolidinyl)-1-(2,4-difluorophényl)-6-fluoro-1,4-dihydro-4-oxo-1,8-naphtyridine-3-carboxylique ;
acide 7-(3-alanyalanylamino-1-pyrrolidinyl)-1-(2,4-difluorophényl)-6-fluoro-1,4-dihydro-4-oxo-1,8-naphtyridine-3-carboxylique ; et de leurs sels
pharmaceutiquement acceptables.

## Revendications (Revendications pour l'(les) Etat(s) contractant(s) suivant(s): GR)

1. Procédé d'amélioration la solubilité d'un composé de formule : 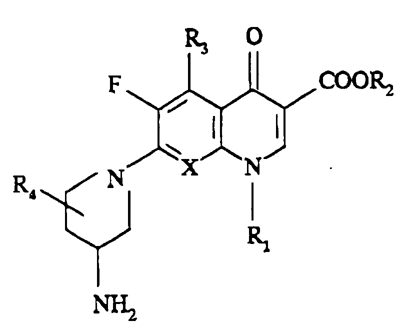 dans laquelle R₁ représente un groupement alkyle ayant de 1 à 6 atomes de carbone, un groupement halogénoalkyle ayant de 1 à 6 atomes de carbone, contenant de 1 à 3 substituants halogène, un groupement hydroxyalkyle ayant de 1 à 6 atomes de carbone, un groupement cycloalkyle ayant de 3 à 6 atomes de carbone, un groupement vinyle, un groupement aryle ayant 5 ou 6 atomes dans le système de cycle qui peut inclure de 1 à 3 hétéroatomes choisis parmi S, O et N, ou bien ledit groupement aryle substitué par 1 à 3 substituants choisis indépendamment dans le groupe constitué des suivants : atome d'hydrogène, atome d'halogène, groupement alkyle ayant de 1 à 6 atomes de carbone, groupement halogénoalkyle ayant de 1 à 6 atomes de carbone, contenant de 1 à 3 substituants halogène, groupement alcanoyloxy de formule R₆COO-, dans laquelle R₆ représente un groupement alkyle ayant de 1 à 6 atomes de carbone, et groupement de formule -Y-R₅, dans laquelle Y représente O ou S et R₅ représente l'atome d'hydrogène ou un groupement alkyle ayant de 1 à 6 atomes de carbone ;
R₂ représente l'atome d'hydrogène, a groupement alkyle ayant de 1 à 6 atomes de carbone, un groupement halogénoalkyle ayant de 1 à 6 atomes de carbone, contenant de 1 à 3 substituants halogène, ou un groupement protecteur du carboxy ;
R₃ représente l'atome d'hydrogène ou NH₂ ;
X représente N, CH, COH, C-O-alkyle, CF, CCl, C-alkyle ou C-NH-alkyle où, dans chaque cas, le groupement alkyle contient de 1 à 6 atomes de carbone ;
R₄ représente l'atome d'hydrogène, un groupement alkyle ayant de 1 à 6 atomes de carbone ou un groupement halogénoalkyle ayant de 1 à 6 atomes de carbone, contenant de 1 à 3 substituants halogène ;
le procédé comprenant la modification du composé a un deuxième composé de formule : 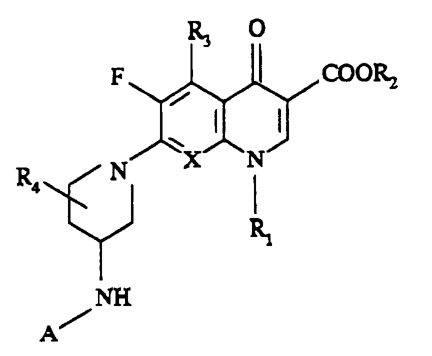 dans laquelle R₁, R₂, R₃, R₄ et X sont tels que définis ci-dessus et A représente un résidu dipeptidyle : et de ses sels pharmaceutiquement acceptables.

2. Procédé de préparation d'un précurseur de médicament qui s'hydrolyse en le composé médicament apparenté du précurseur de médicament, le procédé comprenant la modification d'un médicament apparenté de formule : 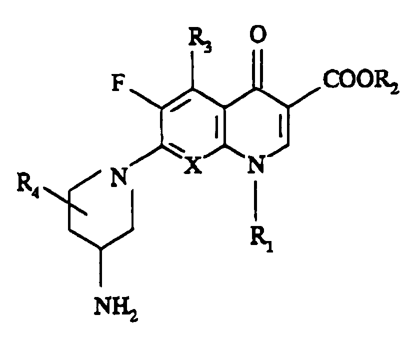 dans laquelle R₁ représente un groupement alkyle ayant de 1 à 6 atomes de carbone, un groupement halogénoalkyle ayant de 1 à 6 atomes de carbone, contenant de 1 à 3 substituants halogène, a groupement hydroxyalkyle ayant de 1 à 6 atomes de carbone, un groupement cycloalkyle ayant de 3 à 6 atomes de carbone, un groupement vinyle, un groupement aryle ayant 5 ou 6 atomes dans le système de cycle qui peut inclure de 1 à 3 hétéroatomes choisis parmi S, O et N, ou ledit groupement aryle substitué par 1 à 3 substituants choisis indépendamment dans le groupe constitué des suivants : atome d'hydrogène, atome d'halogène, groupement alkyle ayant de 1 à 6 atomes de carbone, groupement halogénoalkyle ayant de 1 à 6 atomes de carbone, contenant de 1 à 3 substituants halogène, groupement alcanoyloxy de formule R₆COO-, dans laquelle R₆ représente un groupement alkyle ayant de 1 à 6 atomes de carbone, et groupement de formule -Y-R₅, dans laquelle Y représente O ou S et R₅ représente l'atome d'hydrogène ou un groupement alkyle ayant de 1 à 6 atomes de carbone ;
R₂ représente l'atome d'hydrogène, un groupement alkyle ayant de 1 à 6 atomes de carbone, un groupement halogénoalkyle ayant de 1 à 6 atomes de carbone, contenant de 1 à 3 substituants halogène, ou un groupement protecteur du carboxy ;
R₃ représente l'atome d'hydrogène ou NH₂ ;
X représente N, CH, COH, C-O-alkyle, CF, CCl, C-alkyle ou C-NH-alkyle où dans chaque cas, le groupement alkyle contient de 1 à 6 atomes de carbone ;
R₄ représente l'atome d'hydrogène, un groupement alkyle ayant de 1 à 6 atomes de carbone ou un groupement halogénoalkyle ayant de 1 à 6 atomes de carbone, contenant de 1 à 3 substituants halogène ;
et de ses sels pharmaceutiquement acceptables ;
en ajoutant un résidu dipeptidyle en tant que substituant de la fonction amino du médicament apparenté.

3. Procédé selon la revendication 1 ou 2, dans lequel ledit résidu peptidyle représente Gly-Phe, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Gly, Nval-Ala, Gly-Ala, Phe-Gly, D-Ala-L-Ala, Nval-Nval, Phe-Ala, Val-Ala, Val-Leu, Ala-Ala ou Ala-Phe.

4. Procédé selon la revendication 1 ou 2, dans lequel R₁ représente un groupement du type éthyle, t-butyle, 2-fluoroéthyle, cyclopropyle, 4-fluorophényle ou 2,4-difluorophényle ;
R₂ représente l'atome d'hydrogène ;
X représente N, CH, CF, CCH₃ ou CCl ; et
ledit résidu dipeptidyle représente Gly-Phe, Phe-Gly, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Nval, D-Ala-L-Ala, Nval-Gly, Nval-Ala, Gly-Ala, Phe-Ala, Val-Ala, Val-Leu, Ala-Phe ou Ala-Ala.

5. Procédé selon la revendication 1 ou 2, dans lequel ledit procédé produit un composé choisi dans le groupe constitué des suivants :
acide 7-(3-norvalylnorvalylamino-1-pyrrolidinyl)-1-(2,4-difluorophényl)-6-fluoro-1,4-dihydro-4-oxo-1,8-naphtyridine-3-carboxylique ;
acide 7-(3-glycylglycylamino-1-pyrrolidinyl)-1-(2,4-difluorophényl)-6-fluoro-1,4-dihydro-4-oxo-1,8-naphtyridine-3-carboxylique ;
acide 7-(3-alanylnorvalylamino-1-pyrrolidinyl)-1-(2,4-difluorophenyl)-6-fluoro-1,4-dihydro-4-oxo-1,8-naphtyridine-3-carboxylique ;
acide 7-(3-glycylnorvalylamino-1-pyrrolidinyl)-1-(2,4-difluorophényl)-6-fluoro-1,4-dihydro-4-oxo-1,8-naphtyridine-3-carboxylique ;
acide 7-(3-alanylalanylamino-1-pyrrolidinyl)-1-(2,4-difluorophényl)-6-fluoro-1,4-dihydro-4-oxo-1,8-naphtyridine-3-carboxylique ; et de leurs sels
pharmaceutiquement acceptables.

6. Utilisation d'un composé de formule : 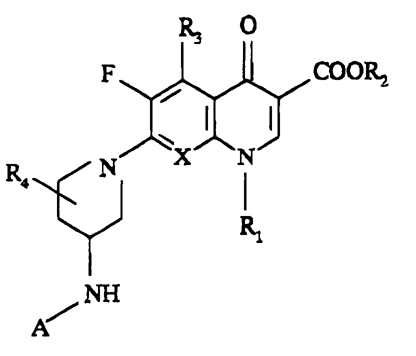 dans laquelle R₁ représente un groupement alkyle ayant de 1 à 6 atomes de carbone, un groupement halogénoalkyle ayant de 1 à 6 atomes de carbone, contenant de 1 à 3 substituants halogène, un groupement hydroxyalkyle ayant de 1 à 6 atomes de carbone, un groupement cycloalkyle ayant de 3 à 6 atomes de carbone, un groupement vinyle, un groupement aryle ayant 5 ou 6 atomes dans le système de cycle qui peut inclure de 1 à 3 hétéroatomes choisis parmi S, O et N, ou bien ledit groupement aryle substitué par 1 à 3 substituants choisis indépendamment dans le groupe constitué des suivants : atome d'hydrogène, atome d'halogène, groupement alkyle ayant de 1 à 6 atomes de carbone, groupement halogénoalkyle ayant de 1 à 6 atomes de carbone, contenant de 1 à 3 substituants halogène, groupement alcanoyloxy de formule R₆COO-, dans laquelle R₆ représente un groupement alkyle ayant de 1 à 6 atomes de carbone, et groupement de formule -Y-R₅, dans laquelle Y représente O ou S et R₅ représente l'atome d'hydrogène ou un groupement alkyle ayant de 1 à 6 atomes de carbone ;
R₂ représente l'atome d'hydrogène, un groupement alkyle ayant de 1 à 6 atomes de carbone, un groupement halogénoalkyle ayant de 1 à 6 atomes de carbone, contenant de 1 à 3 substituants halogène, ou un groupement protecteur du carboxy ;
R₃ représente l'atome d'hydrogène ou NH₂ ;
X représente N, CH, COH, C-O-alkyle, CF, CCl, C-alkyle ou C-NH-alkyle où, dans chaque cas, le groupement alkyle contient de 1 à 6 atomes de carbone ;
R₄ représente l'atome d'hydrogène, un groupement alkyle ayant de 1 à 6 atomes de carbone ou un groupement halogénoalkyle ayant de 1 à 6 atomes de carbone, contenant de 1 à 3 substituants halogène ;
A représente un groupement dipeptidyle ; et de ses sels pharmaceutiquement acceptables,
pour la préparation d'un médicament destiné au traitement d'une affection bactérienne chez un mammifère.

7. Utilisation selon la revendication 6, dans laquelle A représente Gly-Phe, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Gly, Nval-Ala, Gly-Ala, Phe-Gly, D-Ala-L-Ala, Nval-Nval, Phe-Ala, Val-Ala, Val-Leu, Ala-Ala ou Ala-Phe.

8. Utilisation selon la revendication 6, dans laquelle R₁ représente un groupement du type éthyle, t-butyle, 2-fluoroéthyle, cyclopropyle, 4-fluorophényle ou 2,4-difluorophényle ;
R₂ représente l'atome d'hydrogène ;
X représente N, CH, CF, CCH₃ ou CCl; et
A représente Gly-Phe, Phe-Gly, Gly-Nval, Ala-Nval, Ala-Met, Ala-Leu, Leu-Leu, Leu-Met, Leu-Ala, Leu-Nval, Gly-Gly, Met-Nval, Met-Met, Met-Leu, Met-Ala, Nval-Nval, D-Ala-L-Ala, Nval-Gly, Nval-Ala, Gly-Ala, Phe-Ala, Val-Ala, Val-Leu, Ala-Phe ou Ala-Ala.

9. Utilisation selon la revendication 6, dans laquelle ledit composé est choisi dans le groupe constitué des suivants :
acide 7-(3-norvalylnorvalylamino-1-pyrrolidinyl)-1-(2,4-difluorophenyl)-6-fluoro-1,4-dihydro-4-oxo-1,8-naphtyridine-3-carboxylique ;
acide 7-(3-glycylglycylamino-1-pyrrolidinyl)-1-(2,4-difluorophényl)-6-fluoro-1,4-dihydro-4-oxo-1,8-naphtyridine-3-carboxylique ;
acide 7-(3-alnylnorvalylamino-1-pyrrolidinyl)-1-(2,4-difluorophényl)-6-fluoro-1,4-dihydro-4-oxo-1,8-naphtyridine-3-carboxylique ;
acide 7-(3-glycylnorvalylamino-1-pyrrolidinyl)-1-(2,4-difluorophényl)-6-fluoro-1,4-dihydro-4-oxo-1,8-naphtyridine-3-carboxylique ;
acide 7-(3-alanylalanylamino-1-pyrrolidinyl)-1-(2,4-difluorophényl)-6-fluoro-1,4-dihydro-4-oxo-1,8-naphtyridine-3-carboxylique ; et de leurs sels
pharmaceutiquement acceptables.
